# EUROPÄISCHE PATENTSCHRIFT

(11) **EP 0 787 115 B1**
(45) Veröffentlichungstag und Bekanntmachung des Hinweises auf die Patenterteilung: **05.01.2000**
(21) Anmeldenummer: 95944807.7
(22) Anmeldetag: 23.10.1995
(51) Int. Cl.: C07B 53/00, C07C 217/58, C07D 491/00, C07D 491/06

(54) **VERFAHREN ZUM HERSTELLEN VON DERIVATEN DES 4a,5,9,10,11,12,-HEXAHYDRO-6H-BENZOFURO[3a,3,2-ef][2]BENZAZEPINS**
PROCESS FOR PRODUCING DERIVATIVES OF 4a,5,9,10,11,12,-HEXAHYDRO-6H-BENZOFURO[3a,3,2-ef][2]BENZAZEPINE
PROCEDE DE PREPARATION DE DERIVES DE 4a,5,9,10,11,12,-HEXAHYDRO-6H-BENZOFURO[3a,3,2-ef][2]BENZAZEPINE

(30) Priorität: 21.10.1994 AT 198094; 07.06.1995 US 487102
(43) Veröffentlichungstag der Anmeldung: 06.08.1997
(73) Patentinhaber: Sanochemia Pharmazeutika AG, 1090 Wien (AT)
(72) Erfinder: CZOLLNER, Laszlo, A-2491 Neufeld (AT); FRÖHLICH, Johannes, A-1050 Wien (AT); JORDIS, Ulrich, A-1190 Wien (AT); KÜENBURG, Bernhard, A-1190 Wien (AT)
(74) Vertreter: Hehenberger, Reinhard, Dipl.-Ing.
(86) Internationale Anmeldenummer: AT9500208
(87) Internationale Veröffentlichungsnummer: WO9612692

(56) Entgegenhaltungen:
- JOURNAL OF ORGANIC CHEMISTRY., Bd.59, Nr.18, 9. September 1994, EASTON US Seiten 5463 - 5465 WEN-CHUNG SHIEH ET AL 'Asymmetric transformation of either enantiomer of narwedine via total spontaneous resolution process, a concise solution to the synthesis of (-)-galanthamine'
- TETRAHEDRON, Bd.45, Nr.11, 1989, OXFORD, GB Seiten 3329 - 3345 R. VLAHOV ET AL 'Synthesis of galanthamine and related alkaloids-New approaches. I.'
- JOURNAL OF HETEROCYCLIC CHEMISTRY., Bd.25, Nr.6, 1988, PROVO US Seiten 1809 - 1811 J. SZEWCZYK ET AL 'An improved synthesis of galanthamine'
- JOURNAL OF THE CHEMICAL SOCIETY, SECTION C, Nr.18, 1969, LETCHWORTH GB Seiten 2602 - 2605 T. KAMETANI ET AL 'Studies on the synthesis of heterocyclic compounds. Part CCCXV. Modified total synthesis of (+-)-galanthamine through phenol oxidation'
- CHEMICAL ABSTRACTS, vol. 88, no. 21, 22. Mai 1978, Columbus, Ohio, US; abstract no. 152394m, T. KAMETANI ET AL 'Studies on the syntheses of heterocyclic compounds. DCXXXIV. Studies on the synthesis of analgesics. XLVIII. New synthetic method of tetrahydro2-benzazepine derivatives and syntheses of galanthamine analogs' Seite 588 ; & YAKUGAKU ZASSHI, Bd.97, Nr.12, 1977 Seiten 1353 - 1358
- JOURNAL OF HETEROCYCLIC CHEMISTRY., Bd.10, Nr.1, 1973, PROVO US Seiten 35 - 37 T. KAMETANI ET AL 'Studies on the syntheses of heterocyclic compounds. Part DVII. A synthesis of (+-)-norgalanthamine'
- JOURNAL OF HETEROCYCLIC CHEMISTRY., Bd.32, Nr.1, Januar 1995, PROVO US Seiten 195 - 199 J. SZEWCZYK ET AL 'Facile synthesis of (+-), (+), and (-)-galanthamine'

## Beschreibung

Die Erfindung betrifft Verfahren zum Herstellen von Derivaten des 4a,5,9,13,11,12,-Hexahydro-6H-benzofuro[3a,3,2-ef][2tenzazepins der allgemeinen Formel (I) 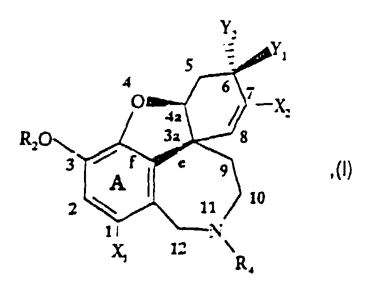 oder von Salzen derselben, worin R₂, R₄, X₁, X₂, Y₁ und Y₂ entweder gleich oder verschieden sind und Wasserstoff, Fluor, Chlor, Brom, Jod, eine Hydroxy- oder Alkoxygruppe, eine niedere, gegebenenfalls verzweigte und gegebenenfalls durch beispielsweise wenigstens ein Halogen substituierte Alkylgruppe, eine niedere, gegebenenfalls verzweigte Alkenylgruppe, eine niedere, gegebenenfalls verzweigte Alkinylgruppe, eine gegebenenfalls substituierte Aryl-, Aralkyl- oder Aryloxyalkylgruppe, dessen Alkylkette gegebenenfalls verzweigt und dessen aromatischer Kern gegebenenfalls substituiert ist, Formyl, aber auch unsubstituiertes sowie durch ein oder mehrere Halogene substituiertes, nicht verzweigtes oder verzweigtes Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl bedeutet oder Y₁ und Y₂ gemeinsam =O bedeuten und
worin A einen Benzolkem bedeutet, der gegebenenfalls ein- oder mehrfach durch wenigstens eine niedere, gegebenenfalls verzweigte Alkylgruppe, wenigstens eine niedere, gegebenenfalls verzweigte Alkengruppe, wenigstens eine niedere, gegebenenfalls verzweigte Alkingruppe, wenigstens eine niedere, gegebenenfalls verzweigte Alkoxygruope, durch Fluor, Chlor, Brom, Jod oder durch mehrere gleiche oder unterschiedliche Halogene, wenigstens eine durch ein Halogen oder durch mehrere gleichen oder unterschiedliche Halogene substituierte Alkylgruppe, wie Chlormethyl und Trifluormethyl, wenigstens eine gegebenenfalls substituierte Aralkylgruppe und/oder wenigstens eine Hydroxygruppe, primäre, sekundäre oder tertiäre Aminogruppe, Nitrogruppe, Nitrilgruppe, Alkylaminogruppe, Arylaminogruppe, Aldehydgruppe, Carbonsäuregruppe, sämtliche Derivate der Carbonsäuregruppe, wie Ester, Amide, Halogenide, substituiert ist.

Die Erfindung betrifft weiters Verfahren zum Herstellen von Derivaten des 4a,5,9,10,11,12,-Hexahydro-6H-benzofuro[3a,3,2-ef] [2]benzazepins der allgemeinen Formel (II) 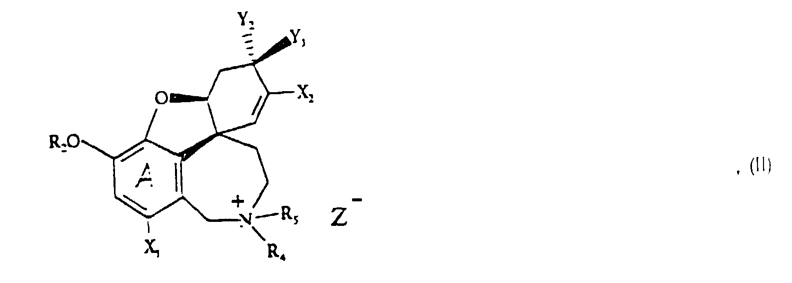 worin R₂, R₄, X₁, X₂, Y₁ und Y₂ sowie A die oben bei Formel (I) angegebenen Bedeutungen haben, Z⁻ ein organisches Anion einer pharmazeutisch verwendbaren Säure, wie Tartrat, Lactat, Citrat, Acetat, Maleinat oder ein anorganisches Anion, wie Fluorid, Chlorid, Bromid, Jodid, Sulfat, Phosphat, Chlorat, R₅ Wasserstoff, Formyl, unsubstituiertes oder durch wenigstens ein Halogen substituiertes, nicht verzweigtes oder verzweigtes Alkyl, Alkenyl, Aryl, Aralkyl, Alkylcarbonyl, Arylcarbonyl oder Aralkylcarbonyl, aber auch unsubstituiertes sowie durch ein oder mehrere Halogene substituiertes nicht verzweigtes oder verzweigtes Alkyloxycarbonyl, Aryloxycarbonyl, Aralkyloxycarbonyl, Alkylsulfonyl, Arylsulfonyl, Aralkylsulfonyl bedeutet.

Bevorzugte Bedeutungen der Substituenten R₁-R₆, X_{1,2}, Y_{1,2} sind:
R₁, R₂, R₃, R₆:Wasserstoff, unsubstituiertes sowie durch ein oder mehrere Halogene substituiertes, nicht verzweigtes oder verzweigtes Alkyl, Alkenyl, Aryl, Aralkyl, Alkylcarbonyl, Arylcarbonyl, Aralkyl-carbonyl, oder jede Kombination dieser Reste
X₁, X₂:H, F, Cl, Br, J-, t-Butyl sowie jede Kombination,
Y₁, Y₂:H, O-R₆, sowie Y₁ und Y₂=O,
R₄, R₅: die für R₁, R₂, R₃, R₆ genannten, bevorzugten Bedeutungen und unsubstituiertes sowie durch ein oder mehrere Halogene substituiertes, nicht verzweigtes oder verzweigtes Alkyloxycarbonyl, Aryloxycarbonyl, Aralkyloxycarbonyl, Alkylsulfonyl, Arylsulfonyl, Aralkylsulfonyl.

Galanthamin ist ein vorwiegend in Pflanzen vom Typus Amaryllidaceae vorkommendes, Alkaloid mit hoher pharmakologischer Aktivität. Hervorzuheben ist insbesondere seine Wirkung als selektiver Acetylcholinesterase Inhibitor und die damit im Zusammenhang stehende Anwendung bei Alzheimer Erkrankungen. Bisher wird Galanthamin in Mengen von wenigen kg jährlich zu einem Preis von über 30.000 US$/kg aus dem kaukasischen Schneeglöckcllen Galanthus Woronoyi isoliert. Seit Ende der sechziger Jahre sind Galanthaminsynthesen im Prinzip bekannt, wobei allerdings lange, unwirtschaftliche Reaktionswege mit schlechten Gesamtausbeuten verwendet wurden.

Die Synthese einiger Verbindungen der oben wiedergegebenen allgemeinen Formeln (I) und (II) ist an sich bekannt und in der Literatur beschrieben. So wurde N-(3-Hydroxy-4-methoxyphenyl)-N-methyl-4-hydroxy-phenylethylamin mit Hilfe von verschiedenen Oxidationsmitteln zu Narwedinderivaten (Narwedin ist der Vorläufer zum Galanthamin, besitzt aber bereits die dem Galanthamin eigene Ringstruktur) oxidativ cydisiert [Lit. 1-2], wobei die Ausbeuten in der Regel unter 1% der Theorie lagen. Damit konnte zwar die Struktur bewiesen, jedoch Galanthamin nicht in pharmazeutisch interessanten kg-Mengen hergestellt werden.

Optimierte Verfahren (vor allem Kametani, Lit. 3-7,22) beschreiben diesen Ringschluß an N-Methyl-benzamid- bzw. Phenylacetamid-Derivaten in Ausbeuten bis 40%, wobei jedoch die schlechten Gesamtausbeuten eine industrielle Nutzung unmöglich machen. Weiters finden sich in der Literatur die Cydisierung von N,N-disubstituierten Phenylethylamin-Derivaten (Lit. 8) sowie elektrochemische (Lit. 9-12), mikrobiologische, enzymatische (Lit. 8) sowie biomimetische Methoden (Lit. 14-15). In Lit. 23 wird die Herstellung von Narwedin aus Isovanillin in 44% Gesamtausbeute beschrieben, wobei jedoch die Verwendung von äquimolaren Mengen Palladium- sowie Talliumtrifiuoroacetat diese Synthese unwirtschaftlich machen. Auf diesem Weg (Lit. 23) gewonnenes (+/-) Narwedin wird in Lit. 24 an gewünschtem (-) Narwedin angereichert und mit L-Selektride in guter Ausbeute in Galanthamin übergeführt.

In Lit. 8 wird eine Synthese vorgeschlagen, in der die oxidative Cydisierung mit 21% Ausbeute beschrieben ist, jedoch fehlt die Trennung der Enatiomeren. Bekannt ist auch die Reduktion von Bromnarwedin mit LiAlH₄ in THF unter Bildung eines 53:31 Diastereomerengemisches von (+/-) Galanthamin und (+/-) Epigalanthamin.

Der Erfindung liegt die Aufgabe zugrunde, ein Syntheseverfahren zu entwickeln, durch welches größere Mengen der Titelsubstanzen auf reproduzierbare Art und Weise und in verbesserten Ausbeuten sowohl der Einzelschritte als auch der Gesamtausbeute hergestellt werden können.

Gelöst wird diese Aufgabe gemäß der Erfindung durch die Verfahren nach Anspruch 1 und 2, wobei die Unteransprüche bevorzugte und vorteilhafte Varianten und Ausführungsformen der Erfindung zum Gegenstand haben. Insbesondere haben sich die folgenden Maßnahmen der Erfindung als vorteilhaft erwiesen:

Ersatz halogenierter Lösungsmittel, z.B. Chloroform, durch Toluol. Halogenierte Lösungsmittel werden heutzutage aufgrund der Giftigkeit, der Schwierigkeiten bei der Entsorgung und der ökologischen Bedenklichkeit kaum mehr als technisches Lösungsmittel eingesetzL Toluol hingegen weist diese Nachteile nicht auf.

Die Aufarbeitung durch Extraktion benötigt organische Lösungsmittel. Mit der Erfindung können die Aufarbeitungen der meisten Stufen so optimiert werden, daß das Reaktionsprodukt zumeist in kristalliner Form aus der Lösung gewonnen werden kann. So können chromatographische Reinigungsstufen oder Extraktionen großteils vermieden werden.

Weiters können bei der Erfindung durch Verbesserung der Parameter die Ausbeuten in einem sehr engen Bereich reproduziert, sowie die Reinheit der Hauptprodukte und der Anteil an Nebenprodukten nach diesen Reaktionen definiert werden. Mit den Verfahren der Erfindung sind verbesserte und reproduzierbare Ausbeuten der Einzelstufen und der Gesamtausbeute möglich. Die Erfindung stellt unter anderem ein Verfahren zur Verfügung, bei dem Bromformylnarwedin mit Reduktionsmitteln reduziert wird. Als Reduktionsmittel kann L-Selektride verwendet werden, wobei die Reduktion diastereoselektiv zu N-Demethylbromgalanthamin in hoher Ausbeute (z.B. 85%) führt, welches durch N-Methylierung nach Eschweiler-Clark und Debromierung in (±) Galanthamin übergeführt werden kann. Bei diesem Verfahren konnte im Reaktionsprodukt (+/-) Epigalanthamin mit chromatographischen Methoden nicht nachgewiesen werden. Galanthamin und Galanthaminderivate können nach dem erfindungsgemäßen Verfahren in technischem Maßstab über in der Literatur nicht beschriebene Intermediate (siehe die in den Ansprüchen 64 bis 67 genannten Verbindungen) hergestellt werden.

Die Verfahren der vorliegenden Erfindung, die bezüglich Ausbeute und Reinheit der erhaltenen Produkte verglichen mit dem Stand der Technik wesentlich verbessert und im technischen Maßstab durchführbar sind, können beispielhaft wie folgt wiedergegeben werden:

Zur Synthese von Derivaten des 4a,5,9,10,11,12,-Hexahydro-6H-benzofuro[3a,3,2-ef] [2]benzazepines der allgemeinen Formel (I) 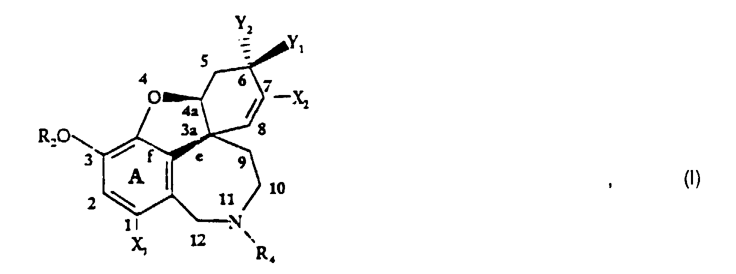 oder von Salzen derselben, worin R₂, R₄, X₁, X₂, Y₁ und Y₂ entweder gleich oder verschieden sind und Wasserstoff, Fluor, Chlor, Brom, Jod, eine Hydroxy- oder Alkoxygruppe, eine niedere, gegebenenfalls verzweigte und gegebenenfalls substituierte Alkylgruppe, eine niedere, gegebenenfalls verzweigte Alkengruppe, eine niedere, gegebenenfalls verzweigte Alkingruppe, eine gegebenenfalls substituierte Aryl-, Aralkyl- oder Aryloxyalkylgruppe, dessen Alkylkette gegebenenfalls verzweigt und dessen aromatischer Kern gegebenenfalls substituiert ist, eine Formylgruppe, aber auch unsubstituiertes sowie durch ein oder mehrere Halogene substituiertes, nicht verzweigtes oder verzweigtes Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl bedeuten, sowie Y₁,Y₂=O (Keton) bedeuten können,
worin A einen Benzolkem bedeutet, der gegebenenfalls ein- oder mehrfach durch wenigstens eine niedere, gegebenenfalls verzweigte Alkylgruppe, wenigstens eine niedere, gegebenenfalls verzweigte Alkengruppe, wenigstens eine niedere, gegebenenfalls verzweigte Alkingruppe, wenigstens eine niedere, gegebenenfalls verzweigte Alkoxygruppe, durch Fluor, Chlor, Brom, Jod oder durch mehrere gleiche oder unterschiedliche Halogene, wenigstens einfach substituierte Alkylgruppe, wie Chlormethyl und Trifluormethyl, wenigstens eine gegebenenfalls substituierte Aralkylgruppe, wenigstens eine Hydroxygruppe, primäre, sekundäre oder tertiäre Aminogruppe, Nitrogruppe, Nitrilgruppe, Alkylaminogruppe oder Arylaminogruppe, aldehydgruppe, Carbonsäuregruppe und sämtliche Derivate der Carbonsäuregruppe, wie Ester, Amide, Halogenide, substituiert ist, wird ein einen Kondensationsschritt mit anschließender Reduktion, eine N-Formylierung oder Einführen einer N-Schutzgruppe, eine Bromierung (die auch bereits auf der Stufe des Isovanillins gemäß Gesamtformelschema durchgeführt werden kann), eine oxidative Cyclisierung, eine Reduktion, je nach der Art des Reduktionsmittels auch noch eine N-Methylierung und Debromierung, sowie eine Trennung der optischen Isomeren enthaltendes Verfahren angewendet. Einzelne der genannten Verfahrensschritte können bei Bedarf auch entfallen.

Gegenstand der vorliegenden Erfindung ist ebenfalls die Herstellung von Salzen der Titelverbindungen.

Die Verbindungen der allgemeinen Formel (I) können in Salze mit organischen und anorganischen Säuren, z.B.:
von Mineralsäuren, wie Chlor- und Bromwasserstoffsäure, Schwefelsäure und Phosphorsäure, Perchlorsäure, oder pharmazeutisch unbedenklichen organischen Säuren, wie Milchsäure, substituierte und nichtsubstituierte Weinsäure, Essigsäure, Salicylsäure, Zitronensäure, Benzoesäure, B-Naphthoesäure, Adipinsäure usw., übergeführt werden.

Die Verfahren der Erfindung führen teilweise zu neuen Verbindungen. Zu den neuen Verbindungen gehören:
Bromgalanthamin der Formel 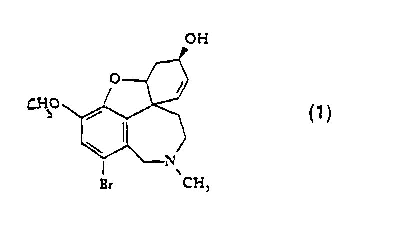 Epibromgalanthamin der Formel 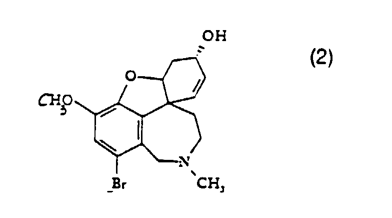 N-Demethylbromgalanthamin der Formel 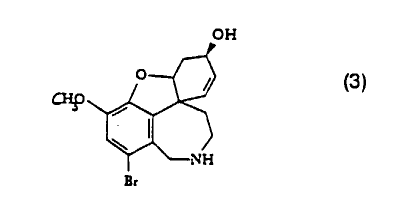 und
N-Demethyl-epibromgalanthamin der Formel

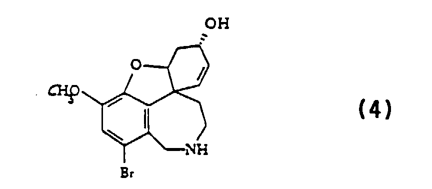

Die Herstellung von Salzen der substituierten Derivate des 4a,5,9,10,11,12-Hexahydro-6H-benzofuro[3a,3,2-ef][2]benzazepins der allgemeinen Formel (II) 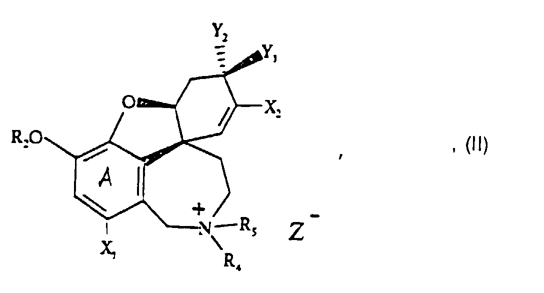 in der R₂, R₄, X₁, X₂, Y₁ und Y₂ sowie A die oben bei Formel (I) angegebenen Bedeutungen haben und Z⁻ ein organisches Anion einer pharmazeutisch verwendbaren Säure, wie Tartrat, Lactat, Citrat, Acetat, Maleinat usw., oder ein anorganisches Anion, wie ein Fluor-, Chlor- Brom oder Jodanion, ein Sulphat- oder Phosphonat- oder Chloratanion ist, R₅ ein Wasserstofratom, ein niederer, nicht verzweigter oder verzweigter Alkylrest, Aryl oder ein in der Alkylkette verzweigter oder nichtverzweigter Aralkylrest ist, nach dem oben beispielhaft beschriebenen Verfahren ist ebenfalls Gegenstand der Erfindung.

Die erfindungsgemäß erhältlichen Verbindungen und deren Salze weisen mindestens zwei asymmetri-sche Zentren auf und treten daher in mehreren stereoisomeren Formen auf. Die Erfindung beinhaltet auch die Trennung der entstehenden Diastereomeren bzw. Racemate in die optisch reinen Antipoden sowie deren Mischungen.

Die oben genannten Schritte können allgemein und beispielhaft wie folgt ausgeführt werden:

### 1. Kondensation und Reduktion.

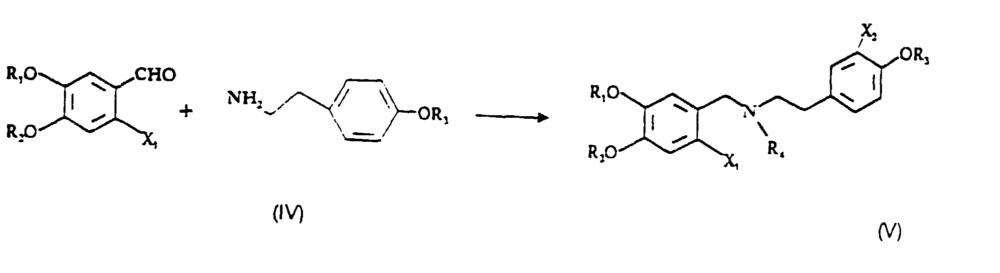

Zur Herstellung der Verbindungen der allgemeinen Formel (I) und (II) werden substituierte Derivate der allgemeinen Formel (V) mit R₄=H hergestellt, indem eine Verbindung der allgemeinen Formel (III), worin R₁ und R₂ Wasserstoff, ein niederes, nichtverzweigtes oder verzweigtes Alkyl oder ein in der Alkylkette verzweigtes oder nichtverzweigtes Aryl bzw. Aralkyl sowie Alkyl-Carbonyl, Arylcarbonyl und Aralkylcarbonyl, oder gemeinsame (R₁=R₂= -CH₂-) Alkylgruppe oder eine Kombination dieser Reste ist, X₁=H, Fluor, Chlor, Brom, Jod, t-Butyl ist, mit Tyramin oder substituiertem Tyramin (R₃=Wasserstoff, ein niederes nichtverzweigtes oder verzweigtes Alkyl, Aryl oder ein in der Alkylkette verzweigtes oder nichtverzweigtes Aralkyl sowie Alkyl-Carbonyl, Arylcarbonyl und Aralkyl-carbonyl) kondensiert wird. Dabei kann so vorgegangen werden:

Eine äquimolare Lösung von (III) und (IV) in Toluol, Xylol oder Benzol oder Mischungen dieser Lösungsmittel mit höheren Alkoholen, vorwiegend Toluol mit n-Butanol in Verhältnissen von 9:1 bis 1:9, vorwiegend 1:1, in Konzentrationen von 1-30% , wird bei Rückflußtemperatur umgesetzt und Wasser abgeschieden. Das Lösungsmittel wird anschließend durch Destillation abgetrennt und zu >95% rückgewonnen, der Rückstand in Alkohol, wie Methanol, Ethanol, n-Propanol, i-Propanol, Methylglykol, Ethylglykol, Wasser, Eisessig, oder Mischungen dieser Lösungsmittel, vorwiegend Methanol, in Konzentrationen von 1-30% gelöst und durch portionsweise Zugabe von 0,6 bis 5 Äquivalenten, vorzugsweise von 0,65 bis 0,7 Äquivalenten von Reduktionsmittel wie Natriumborhydrid, Kaliumbomydrid, Natriumcyanoborhydrid, LiAlH₄ sowie Mischungen aus diesen, vorwiegend aber Natriumborhydrid als Pulver oder Granulat, bei einer Temperatur von -30°C bis Rückflußtemperatur reduziert. Das Kondensationsprodukt (V) wird in Ausbeuten von 80 bis 85% als erste Fraktion durch Filtration aus der alkoholischen Lösung abfiltriert. Aufarbeiten der alkoholischen Lösung durch Destillation auf 15 bis 30% des Volumens und Filtration der 2. Fraktion erhöht die Ausbeute auf 90 bis 95% d.Th.. Alternativ kann die Reaktionslösung auf Wasser gegossen werden, wobei kristallines Produkt (V) ausfällt und nach Absaugen und Trocknen in Ausbeute bis zu 95% erhalten wird.

### 2. N-Formylierung bzw. N-Schutzgruppe:

Ausgangsverbindungen für die oxidative Cyclisierung der Formel (V) mit R₄ = Formyl, aber auch unsubstituiertes sowie durch ein oder mehrere Halogene substituiertes, nicht verzweigtes oder verzweigtes Aralkyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl werden durch Umsetzung der Verbindungen (V) mit R₄ = H mit den entsprechenden Säuren, Estem, Anhydriden, Halogeniden, Aziden, Carbonaten oder anderen reaktiven Derivaten dieser Schutzgruppen hergestellt.

Insbesondere kann eine Verbindung der allgemeinen Formel (V) mit R₄=H in Lösungsmitteln wie THF, Dioxan, DMF, Toluol, Xylol bzw. Mischungen dieser Lösungsmittel mit der äquimolaren bis 50-fach molaren Menge Ethylformiat und katalytischen Mengen Ameisensäure (0,001 bis 1 Äquivalente) bei einer Temperatur von 0°C bis Rücktlußtemperatur zu einer Verbindung der allgemeinen Formel (V) mit R₄=CHO umgesetzt werden. Die Lösungsmittel werden in diesem Verfahren durch Vakuumdestillation enffemt, der Destillationsrückstand durch portionsweise Zugabe von Wasser und Eis kristallisiert und das Produkt durch Filtration in Ausbeuten von >90% bei einem Gehalt von >95% gewonnen.

### 3. Bromierung:

Wenn in Verbindungen der allgemeinen Formel (V) mit R₁, R₂, R₃ = ein niedriges nicht verzweigtes oder verzweigtes Alkyl, Aryl, Aralkyl, Alkylcarbonyl, Arylcarbonyl, Aralkylcarbonyl, X₁, X₂ = H, R₄ = Formyl, aber auch unsubstituiertes sowie durch ein oder mehrere Halogene substituiertes, nicht verzweigtes oder verzweigtes Aralkyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl mit einem Gehalt von 90 bis 100% in Lösungsmittelmischungen halogenierter Kohlenwasserstoffe, wie Chloroform oder Methylenchlorid mit Alkoholen (Methanol, Ethanol, Methylglycol, Ethylglycol, Ethylenglycol, n-Propanol, i-Propanol) in Verhältnissen von 9:1 bis 1:9, vorzugsweise 3:2 bis 2:3, sowie aus reinen Alkoholen (Methanol, Ethanol, Methylglykol, Ethylglykol, Ethylenglykol, n-Propanol, i-Propanol) und deren Mischungen untereinander, vorzugsweise Ethanol/Methylglykol, in Verhältnissen von 9:1 bis 1:9, vorzugsweise 3:2 bis 2:3 mit Wasseranteilen von 0 bis 5%, vorzugsweise 0 bis 0,2%, bei einer Temperatur von -80 bis +60°C, vorzugsweise -40 bis 0°C, in einer Konzentration von 0,5 g bis 20g/100 ml Lösungsmittel mit 1,0 bis 3,0, vorzugsweise 1,4 bis 1,7 Äquivalenten eines Bromreagens, welches durch Zugabe von elementarem Brom in die angegebenen Lösungsmittel in einer Konzentration von 1 bis 90%, vorzugsweise 2 bis 10%, erhalten wird, mit Zugabezeiten des Bromreagens von 10 min bis 4 Stunden, vorzugsweise 15 bis 30 min umgesetzt wird, erhält man nach einer Reaktionszeit von 0,5 bis 24 Stunden, vorzugsweise 30 bis 60 min und nach Aufarbeiten (Einengen durch Destillation auf 10 bis 25% des Volumens und Gießen auf die 10- bis 50-fache Menge Eiswasser, Filtration und Trocknen) in Ausbeuten von 90 bis 96% d.Th. die Verbindung der Formel (V) mit X₁ = Br.

Herstellung des Intermediates (V) mit X₁=Br, R₄=CHO bzw. mehrfach bromierter Intermediate:
Weg 1 (s.S. 13, Gesamfformelschema): Wird eine Verbindung der Fcrmel (V) mit X₁,X₂=H und R₄=CHO entsprechend der angegebenen Arbeitsvorschriften bromiert, so werden beispielsweise 82% Produkt, 6% Edukt, 8% Nebenprodukt mit X₂=Br und 5% höherbromiene Produkte erhalten. (HPLC, Lichrosorb RP 18, 5µ, 300/4 mm, Eluens MeOH/H₂O 6:4 bei 280 nm). Wird die Bromierungsmethode verändert, ändern sich auch die Verhältnisse der angegebenen Produkte (zumeist bildet sich ein größerer Anteil an höherbromienen Produkten). Nach der oxidativen Cyclisierung konnte neben der gewünschten Verbindung der allgemeinen Formel (I) mit X₁=Br, R₄=CHO und Y₁=Y₂=O in Anteilen entsprechend des Anteils an Verbindung der allgemeinen Formel (V) mit X₁=X₂=Br, R₄=CHO, im Edukt nachgewiesen (HPLC, Lichrosorb Si 60, 10 µ, 300/4 mm, Eluens: CHCl₃/MeOH 95:5 bei 254 nm) und mittels präp. Chromatographie (Kieselgel 60, CHCl₃:MeOH 1-5%) isoliert werden. Nach der Reduktion mit L-Selektride oder mit anderen Reduktionsmitteln wird höherbromiertes Narwedin (X₁=X₂=Br) entweder ebenfalls zu Galanthamin reduziert oder durch präparative Chromatographie abgetrennt.
Weg 2: (s.S. 13, Gesamtformelschema). Ausgehend von Veratrumaldehyd über 6-Brom-isovanillin kann durch Kondensation und N-Formylierung die Verbindung der Formel (V) mit X₁=Br, R₄=CHO ohne höherbromierte Nebenprodukte dargestellt werden.
.

### 4. Oxidative Cyclisierung:

Zur oxidativen Cyclisierung von Verbindungen der allgemeinen Formel (V) mit R₂=Wasserstoff, ein niederes, verzweigtes oder nichtverzweigtes Alkyl, Aryl, oder ein in der Alkylkette verzweigtes oder nichtverzweigtes Aralkyl sowie Alkyl-Carbonyl, Arylcarbonal und Aralkyl-carbonyl oder eine Kombination dieser Reste, X₁=H, Fluor, Chlor, Brom, Jod, t-Butyl, R₄= Formyl, aber auch unsubstituiertes sowie durch ein oder mehrere Halogene substituiertes, nichtverzweigtes oder verzweigtes Aralkyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl, R₃=Wasserstoff, zu einer Verbindung der allgemeinen Formel (I) mit R₂, R₄, X₁= wie oben, Y₁, Y₂=O (Keton) und X₂=H, Br, wird in Lösungsmitteln, wie Chloroform, Methylenchlorid, Ethylacetat, THF, Dioxan, Eisessig, Wasser, deren Mischungen mit Alkoholen (Methanol, Ethanol, Methylglykol, Ethylglykol, Ethylenglykol, n-Propanol, i-Propanol) in Verhältnissen von 9:1 bis 1:9, sowie Toluol, Xylol, Benzol, vorwiegend Xylol und Toluol, in einer Konzentration von 0,05 g bis 10 g/100 ml Lösungsmittel mit Basen, wie Natriumhydrogencarbonat, Kaliumcarbonat, NaOH, KOH, Pyridin, vorzugsweise Kaliumcarbonat, in einer Konzentration von 0,1% bis gesättigte Lösung oder Suspension, vorwiegend 5 bis 20%, und Oxidationsmittteln, wie Pb(OAc)₄, KMnO₄, FeCl₃, Kälumferricyanid, H₂O₂, vorzugsweise Kaliumferricyanid, 4-10 Äquivalente, vorzugsweise 5,5-6 Äquivalente, allenfalls unter Zugabe von Phasentransferkatalysatoren wie Aliquat oder Kronenether sowie Ascorbinsäure, CuCI oder Trifluoressigsäure, bei einer Temperatur von -40°C bis Rückflußtemperatur, vorwiegend 50 bis 80°C, und durch rasche oder portionsweise Zugabe des Eduktes als Feststoff, als Lösung oder als Suspension in einem Lösungsmittel, vorzugsweise als Feststoff, bei einer Reaktionszeit von 10 min bis 72 Stunden, vorwiegend 15 bis 45 min, unter heftigem, mechanischem Rühren, vorzugsweise mit einem Rührwerk und einem Homogenisator, allenfalls unter Inertgas wie N₂, CO₂, Argon, vorwiegend Argon, umgesetzt. Aufarbeitung durch Filtration, Phasentrennung und Vakuumdestillation der Toluolphase ergibt das Rohprodukt in Ausbeuten von 5 bis 65%, aus welchen durch Reinigung der Zyklisierungsprodukte Ausbeuten von 5 bis 50% erhalten werden.

### 5. Reduktion:

Zur Reduktion von Verbindungen der allgemeinen Formel (I) in der R₂ ein niedriges nicht verzweigtes oder verzweigtes Alkyl, Aryl, Aralkyl, Alkylcarbonyl, Arylcarbonyl, Aralkylcarbonyl, X₁,X₂ Fluor, Chlor, Brom, Jod, t-Butyl, R₄ Formyl, aber auch unsubstituiertes sowie durch ein oder mehrere Halogene substituiertes, nicht verzweigtes oder verzweigtes Aralkyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl und Y₁, Y₂ = O bedeutet, (Brom-Narwedintyp) mit Hydridreagentien wie DiBAl-H, DiBAL-H/ZnCl₂, Al-isopropylat, Red-Al, K-Selektride, L-Selektride, KS-Selektride, LS-Selektride, Li-tri-t-Butoxy-AIH, Li-tri-ethoxy-AlH, 9-BBN, Superhydride, NaBH₄, Zn(BH₄)₂, AlH₃ AlCl₂H oder einer Kombination dieser Reduktionsmittel kann in der Weise gearbeitet werden, daß durch Zugabe des Reduktionsmittels in äquimolaren Mengen oder im Überschuß zum Ausgangsprodukt oder inverse Zugabe des Ausgangsproduktes zum Reduktionsmittel in einem inerten Lösungsmittel wie Diethylether, THF, Dioxan, Toluol, Xylol, Benzol bei Temperaturen von -50°C bis Rückflußtemperatur reduziert wird. Nach alkalischer (vorwiegend NH₄OH) bzw. saurer (vorwiegend 2n HCI) Aufarbeitung und anschließender Extraktion mit Lösungsmitteln wie Toluol, Xylol, Benzol, Ethylacetat, Ether, Chloforom oder Methylenchlorid wird das Rohprodukt über chromatographische Verfahren gereinigt und bei Bedarf die Diastereomeren isoliert oder die Rohprodukte direkt weiter umgesetzt.

Insbesondere wird durch Reduktion von Brom-N-Formylnarwedin (im Gegensatz zu Lit. 24, wo Narwedin verwendet wird) mit L-Selektride oder K-Selektride diastereoselektiv N-Demethylbromgalanthamin in Ausbeuten von 70-85% d.Th. nach Reinigung durch Säulenchromatographie erhalten. Es konnte mit chromatographischen Methoden kein Epi-N-demethylbromgalanthamin nachgewiesen werden.

N-Demethylbromgalanthamin wird durch N-Methylierung, beispielsweise durch 10-minütiges bis mehrstündiges Aufkochen in 5- bis 50-fachem Überschuß an Ameisensäure und wässeriger Formaldehydlösung in Ausbeuten von 80-90% d.Th. in Bromgalanthamin übergeführt.

Bromgalanthamin wird beispielsweise durch 1- bis 12-stündiges Erwärmen auf Rückflußtemperatur mit einem 5- bis 50-fachen molaren Überschuß an Ameisensäure und Triethylamin in Gegenwart von 0,1 bis 15% Palladium-Aktivkohle Katalysators unter Abspaltung von Brom in Galanthamin übergeführt Ausbeute: 70 bis 80% d.Th..

Die Reaktionsstufen können auch ohne Isolierung und Reinigung der Zwischenprodukte durchgeführt werden.

Durch Reduktion des Eduktes mit U-tri-t-Butoxy-AlH wird ein Gemisch von N-Demethylbromgalanthamin und Epi-N-demethylbromgalanthamin im Verhältnis ca. 1:1 erhalten.

Reduktion mit DiBAl-H ergibt 43% Bromgalanthamin und 41% Epibromgalanthamin.

Reduktion mit Li-AlH₄/wasserfreie H₂SO₄ ergibt ebenfalls Bromgalanthamin und Epibromgalanthamin im Verhältnis ca. 3:1.

Bei der Reduktion kann wie nachstehend beispielsweise angegeben gearbeitet werden:

Zur Reduktion einer Verbindung der allgemeinen Formel (I) mir R₂ = Alkyl, X₁ = Br, R₄ = CHO, X₂ = H, Y₁, Y₂ = O (Keton) wird das Edukt in einem Lösungsmittel wie THF, Dioxan oder anderen Ethem, vorwiegend THF, in Konzentrationen von 0,1 bis 20 9/100 ml durch Erwärmen gelöst. Dann werden bei einer Temperatur von -50°C bis Rückflußtemperatur, vorwiegend 0-20°C, 3 bis 5, vorwiegend 3,5 Äquivalente L-Selektride vorwiegend als 1 molare Lösung in THF zugegeben und zur Reaktion bei 0-20°C 20 min bis 48 Stunden, vorwiegend 1 Stunde gerührt. Der mit dem Reduktionsmittel gebildete Komplex wird durch Zugabe von Wasser und Ammoniumhydroxid zerstört und überschüssiges organisches Lösungsmittel im Vakuum unter Erwärmen auf maximal 30°C abgedampft. Extraktion mit Lösungsmitteln wie Ether (z.B. Diethylether), Ethylacetat, Butylacetat, Chloform, Methylenchlorid, Toluol, Benzol oder Xylol ergibt N-Demethylbromgalanthamin in Rohausbeuten von 90 bis 100% d.Th..

Zur Monomethylierung von N-Demethylbromgalanthamin wird eine Lösung von N-Demethyl-bromgalanthamin in einem 5- bis 30-fachen molaren Überschuß an Ameisensäure und wässeriger Formaldehydlösung (37%) mit oder ohne organisches Lösungsmittel 10 min bis 2 Stunden, vorwiegend 15 bis 20 min auf Rückflußtemperatur erwärmt.

Zur Debromierung von Bromgalanthamin oder Epibromgalanthamin wird Brom - bzw. Epibromgalanthamin in einem 5- bis 50-fachen molaren Überschuß von Ameisensäure und Triethylamin mit oder ohne organisches Lösungsmittel in Gegenwart von 0,1 bis 15% Palladium-Aktivkohle Katalysator 1 bis 12 Stunden, vorwiegend 2,5 Stunden auf Rückflußtemperatur erwärmt.

Zur Reduktion einer Verbindung der allgemeinen Formel (I) mit R₂ = Alkyl, X₁ = Br, R₄ = CHO, X₂ = H, Y₁, Y₂ = O (Keton), wird das Edukt in einem inerten organischen Lösungsmittel, wie Benzol, Toluol oder Xylol, vorwiegend Toluol in einer Konzentration von 0,1 bis 20 g/100 ml suspendiert und bei einer Temperatur von -50°C bis Rückflußtemperatur, vorwiegend 0 bis 20°C 3 bis 5, vorwiegend 3,5 Äquivalente DiBAL-H als vorwiegend 1,5 molare Lösung in Toluol zugetropft. Nun wird 20 min bis 12 Stunden, vorwiegend 30 min bis 1,5 Stunden bei dieser Teperatur gerührt, der gebildete Komplex mit Wasser und Ammoniumhydroxid zerstört, mit Toluol extrahiert und das Rohprodukt (90 bis 100% d.Th.) mittels Säulenchromatographie (Kieselgel, Aceton/Hexan 1:1) in 43% Bromgalanthamin und 41% Epibromgalanthamin getrennt.

### 6. Trennung der optischen Isomeren:

Um chirale 4a,5,9,10,1 1,12-Hexahydro-6H-benzofuro[3a,3,2-efI2]benzazepine der allgemeinen Formel (I), (Y₁=H, OH; Y₂=H, OH) in der A, R₂, R₄, X₁ und X₂ die oben angegebenen Bedeutungen haben, in die enantiomerenreinen Antipoden zu trennen, kann die Methode der fraktionierten Kristallisation von Salzen mit chiralen Säuren angewendet werden. Die Trennung der (+) und (-) Isomeren der Narwedintyp-Verbindungen (Verbindungen der allgemeinen Formel (I), in der Y₁ und Y₂ gemeinsam =O (Keton) bedeuten) durch fraktionierte Kristallisation erfolgt in der Weise, daß eine Lösung oder Suspension des optischen Isomerengemisches in der 5- bis 50-fachen Menge eines Lösungsmittel, wie Wasser, Methanol, Ethanol, Propanol, Isopropanol, Aceton oder Mischungen dieser Lösungsmittel, vorwiegend Methanol, mit der äquimolaren Menge oder einem Überschuß einer chiralen Säure (unsubstituierte, einfach oder mehrfach substituierte + oder - Weinsäure, Zitronensäure, Milchsäure, α-Methoxyphenylessigsäure, Kamphersulfonsäuren sowie deren Derivate, vorzugsweise Di-p-tolyl (+) Weinsäure), die in einem der oben genannten Lösungsmittel gelöst ist, vereinigt wird, daß die Lösung mit aus dem natürlichen (-) Galanthaminderivaten und chiralen organischen Säuren, wie Di-p-tolyl (+) Weinsäure, hergestellten Kristallen geimpft wird und bei -40 bis +20°C, vorzugsweise 0°C 2 bis 24 Stunden oder länger stehengelassen wird, daß die gebildeten Kristalle filtriert und getrocknet werden, anschließend im Überschuß mit NH₄OH versetzt und mit organischem Lösungsmittel, wie Chloroform, Methylenchlorid, Ethylacetat, Butylacetat, Diethylether, 1-Butylmethylether, Dibutylether, Petrolether, Xylol, Benzol, Toluol oder ähnlichen Lösungsmitteln extrahiert und durch Destillation des Lösungsmittels das entsprechende (-) Galanthamin-Derivat isoliert wird.

In diesem Verfahren ergibt Einengen der Mutterlauge, Aufnehmen im Überschuß NH₄OH, Extraktion mit einem organischen Lösungsmittel (wie oben angegeben) und Eindampfen weitere Frakionen von Galanthamin, aus dem in analoger Weise zu oben mit Hilfe chiraler organischer Säuren, wie z.B. Di-p-tolyl (-) Weinsäure die (+) Galanthamin-derivate gewonnen werden können.

Die nach der Erfindung erhaltenen Produkte können durch ein in der Chemie übliche Verfahren gereinigt werden, beispielsweise fraktionierte Destillation, Kristallisation oder Chromatographie.

W.C. Shieh und J.A. Carlson berichten in J. Org. Chem. 1994, 59, 5463-5465, daß (-)Galanthamin ein selektiver Acetylcholinesterase-Hemmer ist, der die cholinergische Funktion verstärkt und als Mittel zur Behandlung von Personen, die an Alzheimerkrankheit leiden, in Betracht gezogen wird.

Um enantiomerenreines (-)Galanthamin herzustellen, wird vorgeschlagen, (±)Narwedin in Lösung mit katalytischen Mengen von (-)Narwedin- oder (+)-Galanthamin-lmpf-Kristallen zu versetzen und kristallisieren zu lassen. Dabei kristallisiert aus der (±)Narwedin enthaltenden Lösung (-)Narwedin in Form weißer Kristalle aus. Um durch Reduktion (-)Narwedin in (-)Galanthamin überzuführen, wird eine diastereoselektive Reduktion von enantiomerenreinem Narwedin vorgeschlagen. Durch die diastereoselektive Kristallisation gewonnenes (-)Narwedin wird stereospezifisch durch L-Selektride bei -78°C in nahezu 99%-iger Ausbeute zu (-)Galanthamin reduziert. Für das zweistufige Verfahren (Kristallisieren und Reduktion) werden Gesamtausbeuten in der Umwandlung von racemischem Narwedin in (-)Galanthamin mit 90% angegeben. Bezüglich der Herstellung von (±)Narwedin wird auf Lit. 23 (Holton et.al.) verwiesen, eine Methode, bei welcher stöchiometrische Mengen Palladium und Thallium benötigt werden.

Nachteilig bei dem beschriebenen Verfahren ist es unter anderem, daß die Reduktion bei den beschriebenen Verfahrensbedingungen bei -78°C ausgeführt werden muß. Weiters wird nur ein Halbmikroansatz (285 mg Edukt) beschrieben, der in der ca. 200-fachen Menge an Lösungsmittel durchgeführt und chromatographisch unter Verwendung von CH₂Cl₂/methanol (6:1) aufgearbeitet wird.

Nachstehend werden Reaktionsschemata der erfindungsgemäßen Verfahren wiedergegeben.

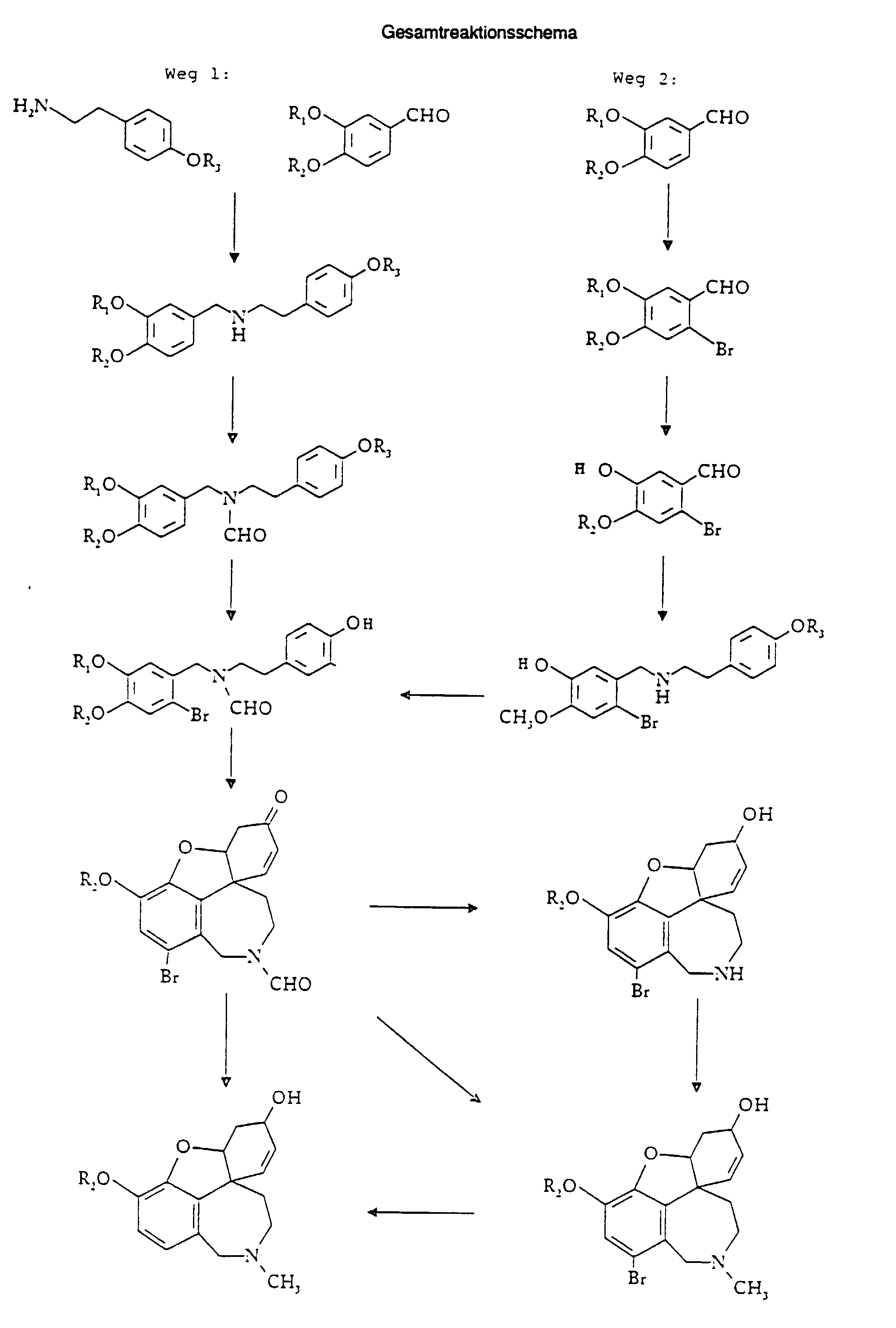

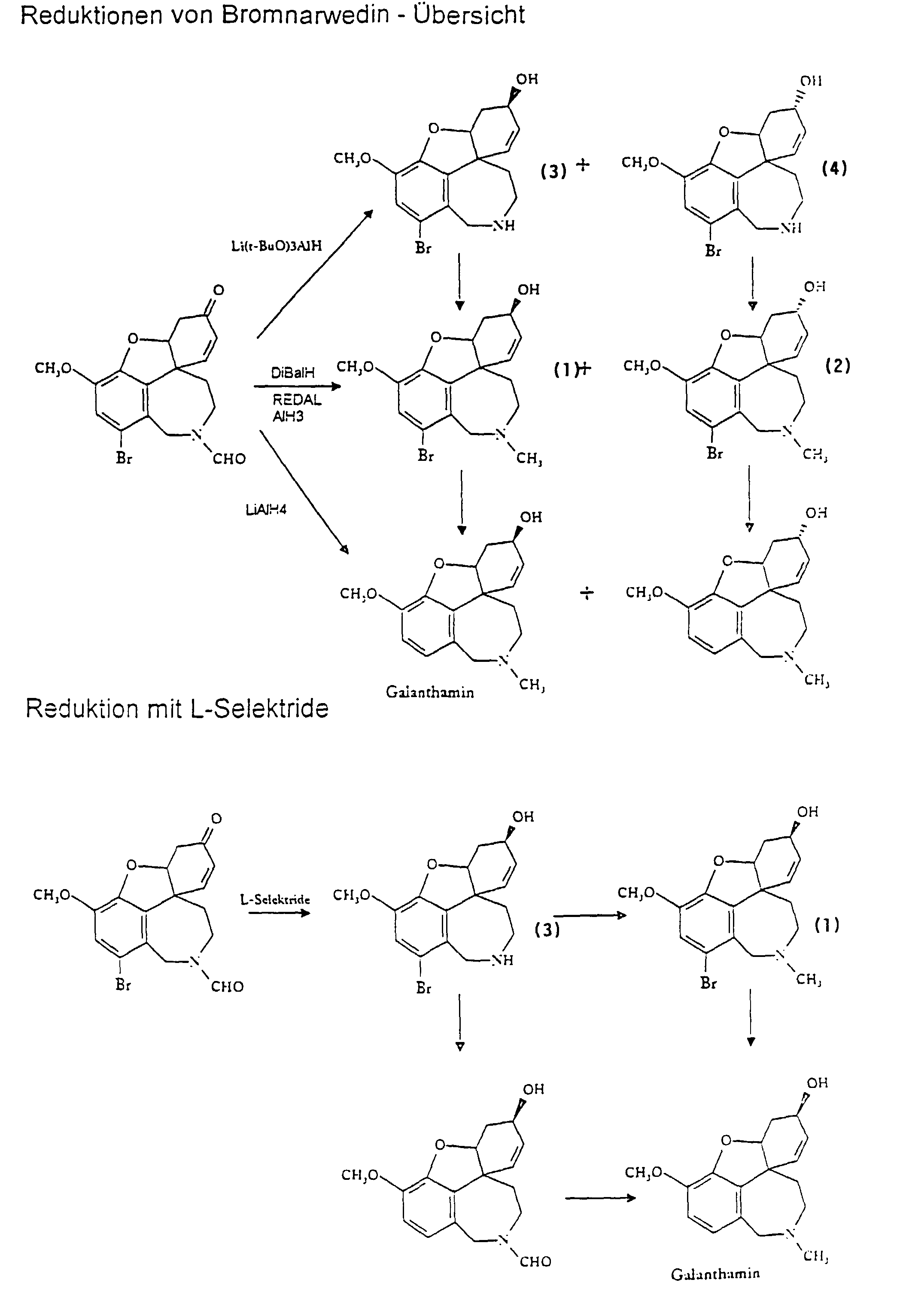

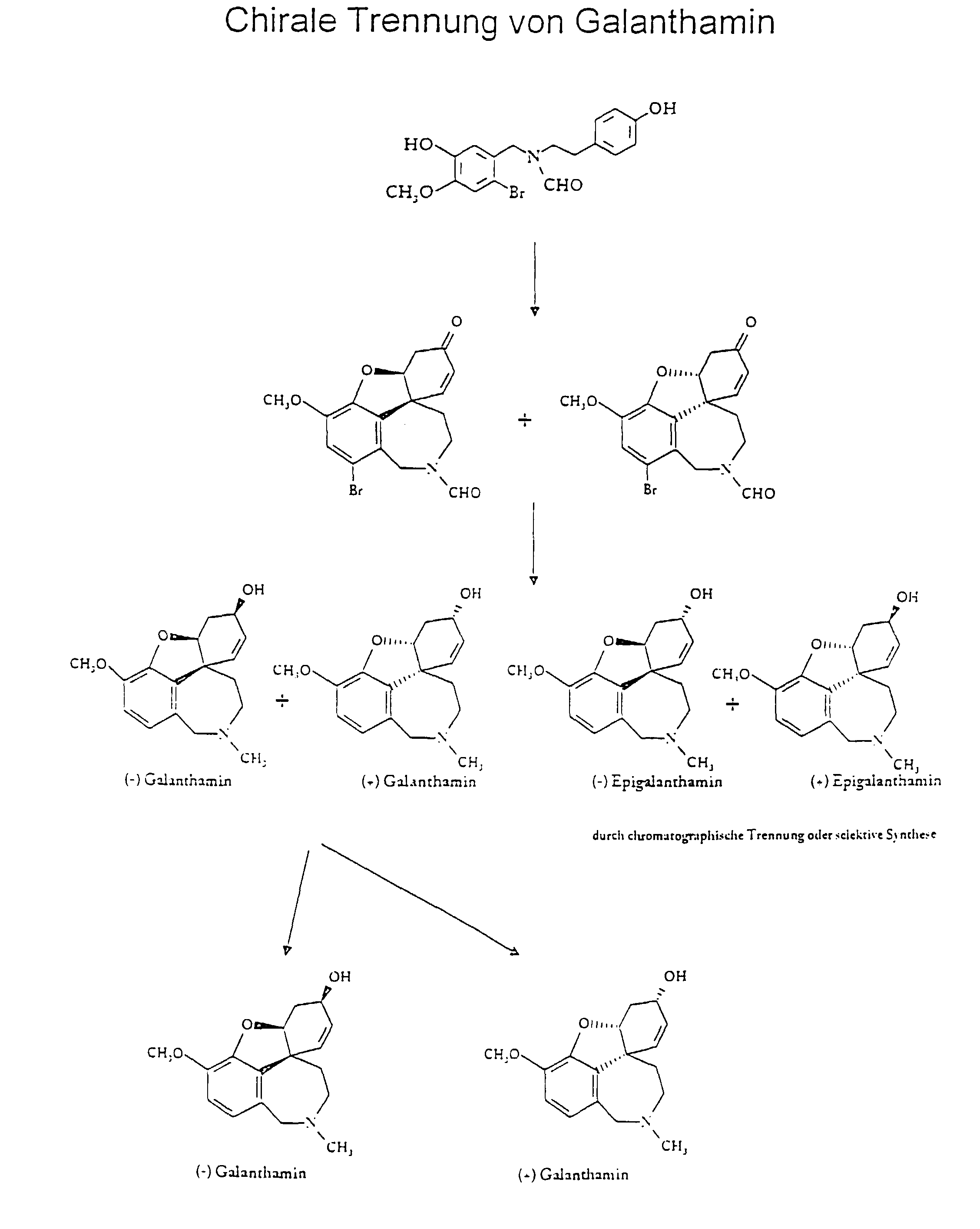

Gemäß einer Variante des Verfahrens der Erfindung kann ausgehend von der cyclisierten Verbindung der allgemeinen Formel (I) mit Y₁, Y₂ = O (Keton) über die Einführung eines cyclischen Ketals als Schutzgruppe (Y₁, Y₂ = substituiertes oder unsubstituiertes cyclisches Ketal oder Thioketal, z.B. Propylenglykol: O-CH₂-CH₂(CH₃)-O), anschließender Reduktion mit LiAIH₄ und Abspaltung der Ketal-Schutzgruppe Narwedin gewonnen werden. Racemisches Narwedin (oder eine Verbindung der allgemeinen Formel (I), in der Y₁, Y₂ =O (Keton) bedeuten) kann durch Zugabe katalytischer Mengen von (+)Galanthamin oder von (-)Narwedin angereichert werden und (-) Narwedin mit einer enantiomeren Reinheit von 〉98% gewonnen werden.

Der Vorteil dieser Variante der Erfindung ist, daß ungewünschtes Enantiomer in gewünschtes Enantiomer übergeführt werden kann.

Auf ebensolche Weise läßt sich durch Zugabe katalytischer Mengen von (-)Galanthamin oder (+)Narwedin racemisches Narwedin zu (+)Narwedin anreichern. Angereichertes Narwedin wird mit L-Selektride in guter Ausbeute in enantiomerenreines Galanthamin übergeführt, wobei durch entsprechende Aufarbeitung entweder die freie Base oder direkt das Hydrobromid gewonnen werden kann. Durch das Kristallisieren des Hydrobromides kann Galanthamin-Hydrobromid mit einem enantiomeren Gehalt von 〉99% gewonnen werden. Die Bestimmung des Gehaltes erfolgt durch Messung des Drehwertes und durch quantitative Bestimmung der Enantiomeren mittels Mikrokapillarelektrophorese in chiralem Elektrolyt.

Die oben genannten Schritte könnten allgemein und beispielhaft wie folgt ausgeführt werden:

### 7. Einführen der Schutzgruppe:

Zur Einführung einer Ketal-Schutzgruppe wird die Verbindung der allgemeinen Formel (I) mit Y₁, Y₂ =O (Keton), X₁ Br und R₁ CHO in Lösungsmitteln wie Benzol, Toluol, Xylol, vorwiegend aber Toluol, zusammen mit der 1 bis 30-fachen Menge an Diolen, wie Ethylenglykol, Propylenglykol, oder Dithiolen, wie 1,3-Dithiopropan, in Gegenwart katalytischer Mengen von p-Toluolsulfonsäure oder konzentrierte Schwefelsäure oder anderen Säuren mehrere Stunden am Wasserabscheider auf Rückfluß-temperatur erwärnt. Anschließend wird abgekühlt, die Diolphase (Dithiolphase) abgetrennt, mit Toluol extrahiert und durch Eindampfen der Toluolphasen das gewonnene Ketal (Thioketal) isoliert.

### 8. Reduktion, Abspalten der Schutzgruppe:

Gereinigtes oder rohes Ketal (Thioketal) der allgemeinen Formel (I) (vorwiegend mit X₁ Br und R₄ CHO) wird durch Reduktion mit LiAlH₄ und anschließende Abspaltung der Ketalgruppe in Narwedin übergeführt. Beispielsweise wird das Propylenglycolketal der Verbindung der allgemeinen Formel (I) in THF gelöst, mit der 3 bis 5-fachen stöchiometrischen Menge LiAlH₄ versetzt und 12 Stunden auf Rückflußtemperatur erwärmt. Dadurch werden auch gegebenenfalls X₁ Br in X₁ H und R₄ CHO in R₄ CH₃ übergeführt. Zersetzen des überschüssigen LiAlH₄ mit NH₄OH, Filtration und Extraktion mit EtOAc ergibt die ketalgeschützte Verbindung der allgemeinen Formel (1) vom Narwedintyp. Erwärmen des Rohproduktes in einer Säure, vorwiegend 2n Salzsäure, und Alkalischmachen mit NH₄OH ergibt die Verbindung der allgemeinen Formel (I) vom Narwedintyp in guter Ausbeute (ca. 80%). Wird mit LiAlH₄ bei -10° bis 0°C für 2 Stunden gerührt, anschließend mit NH₄OH hydrolisiert und mit EtOAc extrahiert, kann ketalgeschütztes N-Demethylbromnarwedin gewonnen werden. Vergleichbar mit der Reduktion mit L-Selektride bildet sich intermediär eine Verbindung der allgemeinen Formel (I) mit R₄ CH₂-OH, die bei der Hydrolyse zersetzt wird und die N-Demethyl-Verbindung ergibL Durch Behandlung in 2n HCI kann die Ketalgruppe abgespalten urd eine Verbindung vom Typ Demethyl-brom-narwedin erhalten werden. Alkylierung der O-geschützten oder ungeschützten Verbindungen vom Typ Demethyl-brom-narwedin oder Einführen einer N-Schutzgruppe und Abspalten der gegebenenfalls vorhandenen O-Schutzgruppe ergibt unterschiedlich substituierte Narwedine der allgemeinen Formel (I) mit Y₁, Y₂=O (Keton), mit R₄ substituiertes oder nicht substituiertes Alkyl, Alkenyl, Alkinyl, Aryl, Aralkyl bzw. jede Schutzgruppe bzw. quaternisierte Verbindungen der allgemeinen Formel (II). Debromierung, beispielsweise mit Zn/CaCl₂ ergibt N-substituierte Verbindungen vom Typ Narwedin.

Wird eine Verbindung der allgemeinen Formel (I) mit Y₁, Y₂=Ethylenglykolketal mit LiAIH₄ in THF 12 Stunden auf 45-50°C erwärmt, so entsteht entsprechend ketalgeschülztes Narwedin. Wird das Edukt 24 Stunden auf Rückflußtemperatur (65-68°C) erwärmt, so öffnet sich die cyclische Ketalstruktur und es entsteht ein Produkt mit Y₁ -CH₂-CH₂-OH und Y₂ H, das aber ebenfalls durch kurzes Erwärmen in einer Säure, vorwiegend 2n HCI in eine Verbindung vorn Narwedintyp übergeführt werden kann.

Interessant ist, daß die Reduktion mit LiAIH₄ bei 0°C zu Demethyl-Brom-Narwedin-Ketal, bei 45°C zu Narwedin-Ketal, bei 70°C und 48 Stunden zu Galanthamin-Hydroxyethylether und bei 45-70°C und anschließender HCI-Behandlung (spaltet auch das Ketal) zu Narwedin führt.

Reduktion einer ketalgeschützten Verbindung der allgemeinen Formel (I) mit X₁ Br, R₄ CHO mit Zn/CaCl₂ führt zur Reduktion des Broms, zur Abspaltung der Ketalgruppe aber zum Erhalt von R₄ CHO.

### 9. Anreicherung:

Eine racemische Verbindung der allgemeinen Formel (I) mit Y₁, Y₂=O (Keton), R₄ CH₃ wird in einem Lösungsmittel wie Wasser, Methanol, Ethanol, n-Propanol, Butanol, Methylenglykol, Ethylenglycol oder Mischungen dieser Lösungsmittel mit 1 bis 30% Triethylamin oder ähnlichen Basen auf Rückflußtemperatur erwärmt, mit optisch reinen Verbindungen beispielsweise (+) Galanthamin oder (-) Narwedin versetzt. Für (-)Narwedin werden beispielsweise entweder (+)Galanthamin oder (-)Narwedin verwendet, für (+)Narwedin werden beispielsweise (-)Galanthamin oder (+)Narwedin verwendet und langsam, stufenweise abgekühlt.

Bevorzugt wird 1 bis 14 Tage bei 40°C gerührt, anschließend auf 0-20°C abgekühlt, die ausgefallenen, optisch angereicherten Kristalle isoliert und mittels Mikrokapillarelektrophorese ein enantiomerer Gehalt von >98% bestimmt. Dabei werden z.B. für Narwedin Drehwerte von 405-407° (20°C, c=1/CHCl₃) erreicht, Bestimmung mittels Mikrokapillarelektrophorese in chiralem Elektrolyten ergibt einen enantiomeren Gehalt von >98%.

### 10. Reduktion:

Die enantiomerenreine Verbindung vom Narwedintyp (Y₁, Y₂ = O, Keton) kann mit L-Selektride analog der bereits angegebenen Vorschrift diastereoselektiv in enantiomerenreine Verbindung vom Galanthamintyp (Y₁ oder Y₂ OH) übergeführt werden. Aufarbeitung mit wässeriger HBr ergibt das entsprechende Galanthamin-Hydrobromid mit einem enantiomeren Gehalt von 〉99%, bei einer Ausbeute von 87-95% d.Th.

### 11. Abspaltung von Brom:

Eine Verbindung der allgemeinen Formel (I) mit X₁ Br wird in 5 bis 50-facher Menge eines Lösungsmittels wie Wasser, Methanol, Ethanol, n-Propanol, iso-Propanol, n-Butanol oder Mischungen daraus, vorwiegend 70% Ethanol, gelöst, die 1 bis 5-fache Menge Zinkpulver und die 1 bis 10-fache Menge CaCl₂ zugegeben und gerührt. Nach durchschnittlich ca. 1 bis 2 Stunden wurde der Feststoff abfiltriert, die Lösung eingedampft und chromatographiert (Kieselgel 60, Lösungsmittel z.B. Aceton) und ergibt in 80 bis 85% debromiertes Produkt.

Gegenüber der in Lit. 24 beschriebenen Methode konnte das Verfahren so verbessert werden, daß eine Durchführung im industriellen Maßstab möglich ist. Beispielsweise wird Edukt in Pulverform in eine vorzugsweise 1-molare Lösung von L-Selektride in THF bei Raumtemperatur zugegeben, eine Stunde gerührt, mit Methanol versetzt und eingedampft. Aufnehmen in Ethanol (beispielsweise 5-30-fache Menge) und Ansäuem mit wässriger HBr ergibt Galanthamin Hydrobromid in Ausbeuten von 90 bis 95% bei einem enantiomeren Gehalt von 〉99%.

Die beschriebene Verfahrensvariante führt teilweise zu neuen Verbindungen bzw. entstehen neue Verbindung als Zwischenprodukte. Die neuen Verbindungen sind:
Brom-N-formyl-narwedin-propylenglycolketal (**5**) 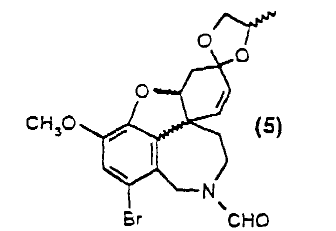 Narwedin-propylenglycolketal (**6**) 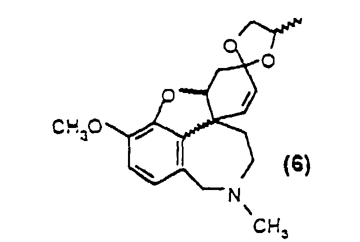 Brom-N-formyl-narwedin-ethylenglycolketal (**7**) 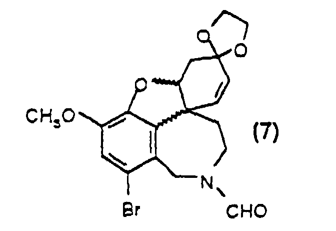 Narwedin-ethylenglycolketal (**8**) 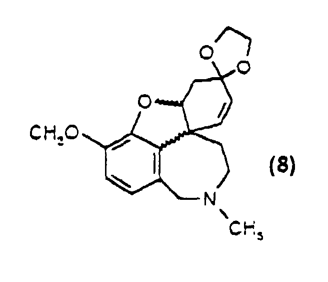 O-(2-Hydroxyethyl)-galanthamin (**9**) 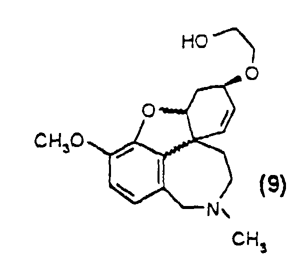 Brom-N-demethyl-narwedin-ethylenglycolketal (**10**) 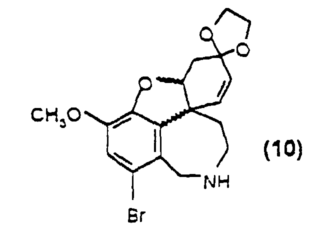 Brom-N-benzyl-narwedin-ethylenglycolketal (**11**) 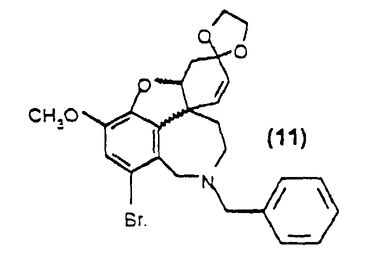 Brom-N-demethylnarwedin (**12**)

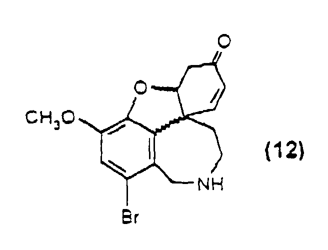

Die diesen Verbindungen zugeordneten Nummern werden auch in den nachstehend wiedergegebenen Reaktionsschemata verwendet.

Reaktionsschemata Narwedin über Ketal-geschütztes Bromformylnarwedin

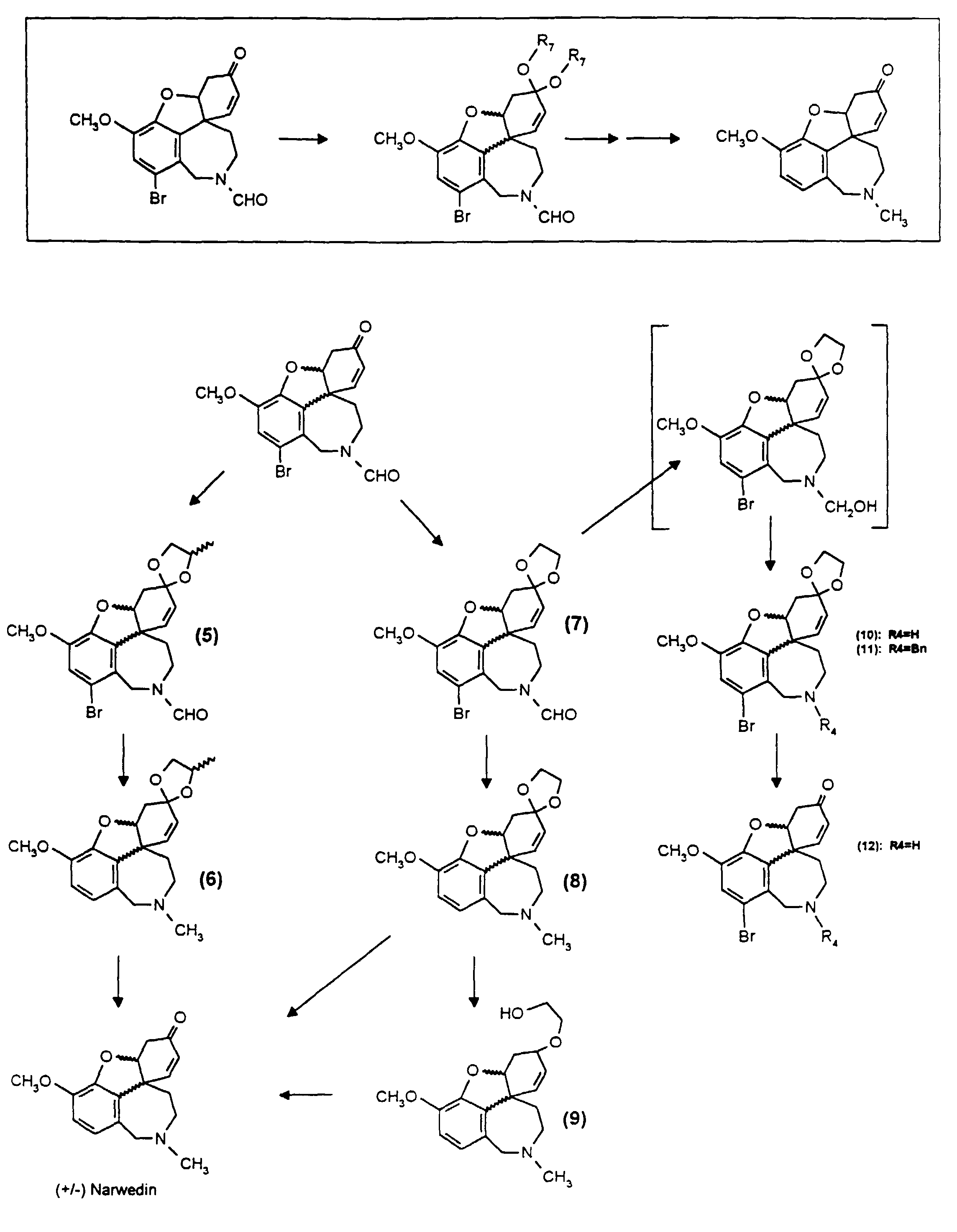

Gesamtübersicht über ein bevorzugtes Verfahren der Erfindung zur Synthese von (-)Galanthamin

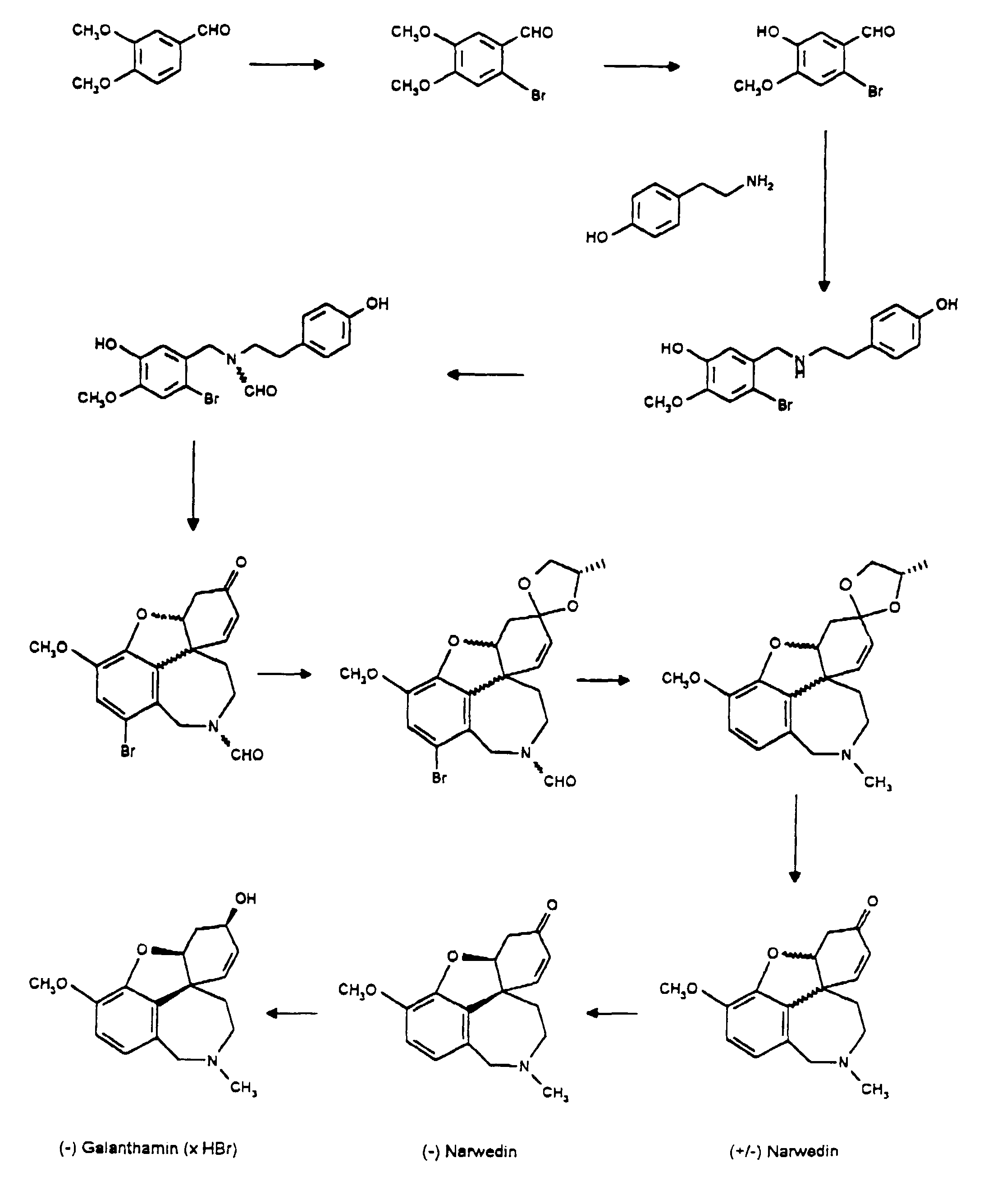

Nach einer weiteren Variante der Erfindung geht man zur Herstellung von racemischen Verbindungen vom Narwedintyp so vor, daß eine Verbindung der allgemeinen Formel (la) 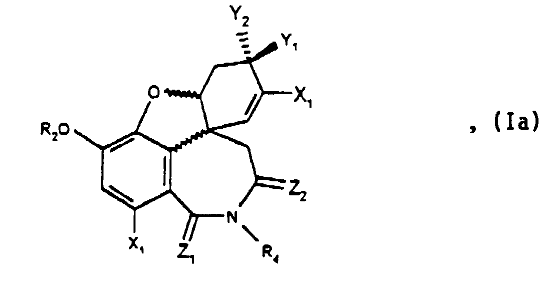 in der R₂, R₄, X₁, X₂ die bei der allgemeinen Formel (I) angegebenen Bedeutungen haben, Z₁ und Z₂ = O, S, N und Y₁, Y₂ =O (Keton) bedeuten durch oxidative Cydisierung einer Verbindung der allgemeinen Formel (Va) 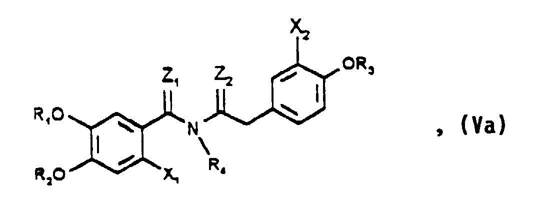 in der R₁, R₂, R₃, R₄, X₁, X₂ die bei der allgemeinen Formel (V) angegebenen Bedeutungen haben, und Z₁ und Z₂ =O, S, N bedeutet, hergestellt wird.

Anschließend wird beispielsweise analog der oben beschriebenen Stufe 7.) in ein Ketal oder Thioketal oder cyclisches Ketal oder cyclisches Thioketal übergeführt, mit einem Reduktionsmittel, wie LiAlH₄, analog der oben beschriebenen Stufe 8.) reduziert als Ketal oder Thioketal isoliert oder durch vorzugsweise saure Hydrolyse in die entsprechende Verbindung vom Narwedintyp übergeführt. Das Reaktionsschema ist in der Übersicht "Synthese von Narwedin über Benzazepinon-Typ" wiedergegeben (für Z₂ = H₂).

Als Nebenprodukt kann in unterschiedlichen Konzentrationen durch Alkoholyse eine Verbindung der Formel (VI) entstehen, 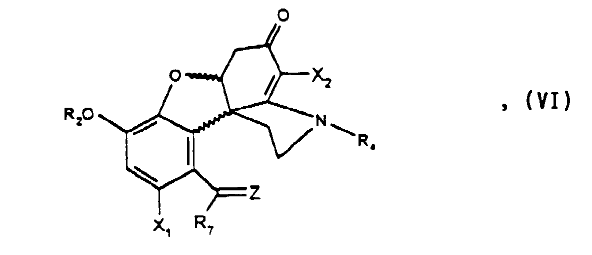 wobei R₂, R₄, X₁, X₂, Z₁ und Z₂ die bei der allgemeinen Formel (Ia) genannten Bedeutungen haben und R₇ dem für die Ketalherstellung verwendeten Alkohol oder Thiol, beispielsweise -0- CH₂CH(CH₃)-OH (Propylenglykolrest), entspricht.

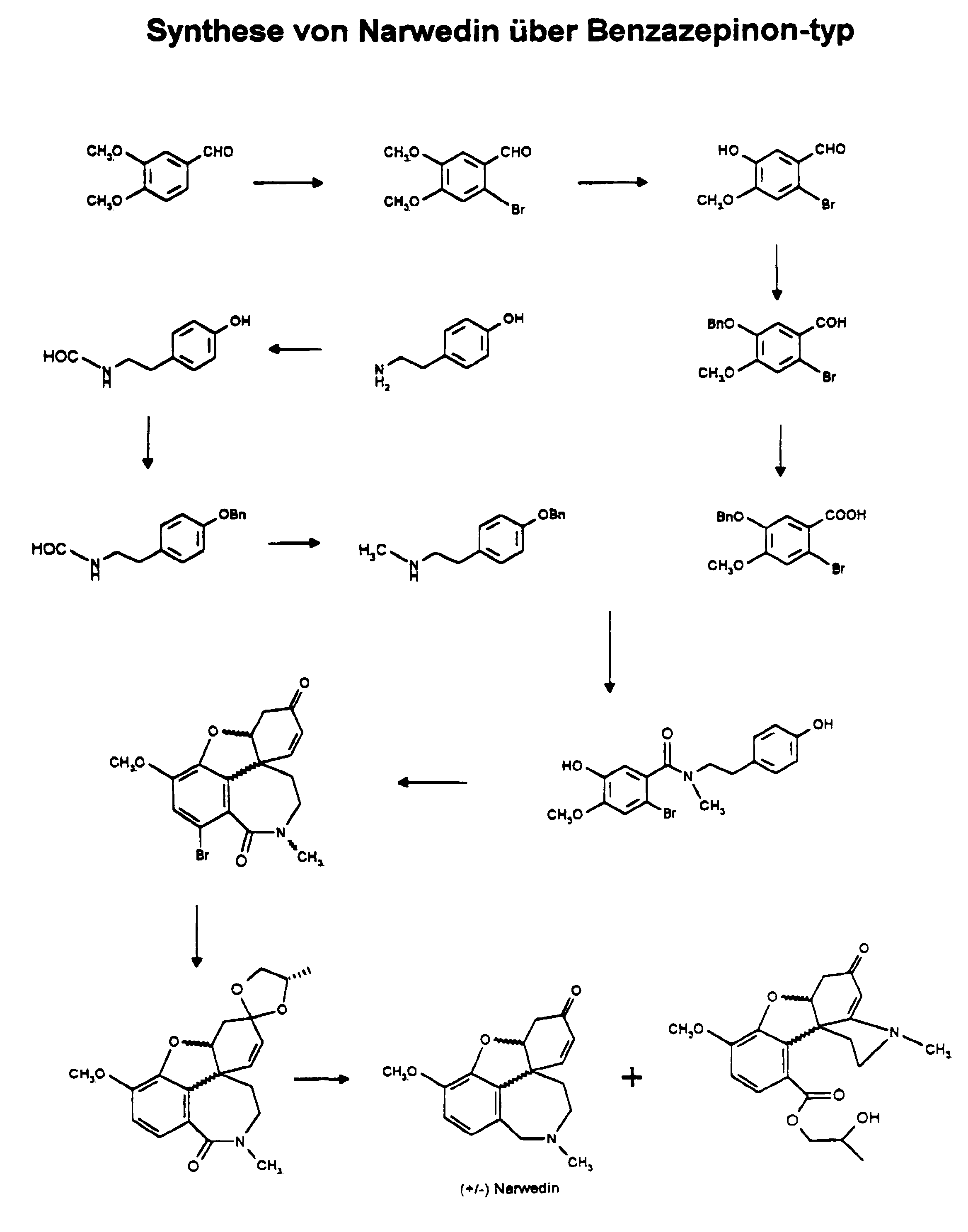

Die Reduktion der Verbindung der allgemeinen Formel (Ia), worin R₂, R₄, X₁, X₂, die bei der allgemeinen Formel (I) genannten Bedeutungen haben Z₁, Z₂ =O, S, N, und Y₁, Y₂ = O (Keton) bedeutet mit L-Selektride ergibt eine Verbindung der Formel (la) mit Y₁ = OH, Y₂ = H.

Die Reduktion einer Verbindung der Formel (la) mit Y₁, Y₂ = O mit LiAIH₄ ergibt eine Mischung vom Galanthamintyp (Y₁ = OH, Y₂ = H) und Epigalanthamintyp (Y₁ = H, Y₂ = OH) im Verhältnis von ca. 5:3, wobei X₁, X₂ = Br zu X₁, X₂ = H und Z = O zu Z = H₂ reduziert werden.

Die beschriebene Verfahrensvariante führt teilweise zu neuen Verbindungen:

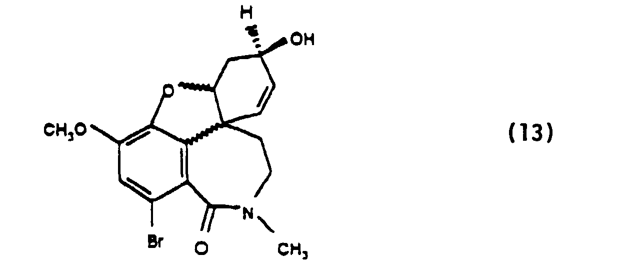

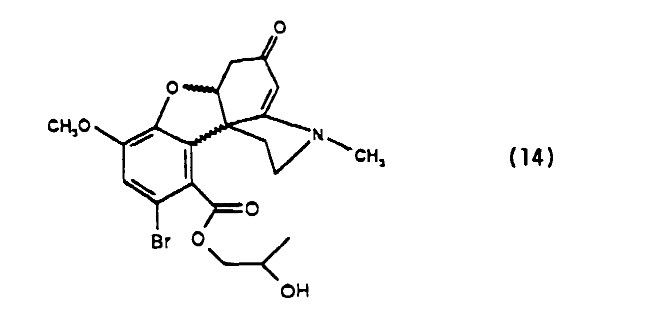

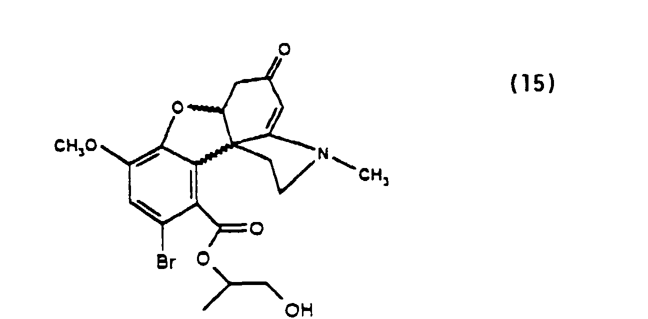

Nachstehend werden Beispiele für die Verfahren der Erfindung angegeben:

### Beispiel 1:

### N-(4-Hydroxyphenethyl)-(3-hydroxy-4-methoxy)benzylamin (allgemeine Formel (V) mit R₁=R₃=R₄=X₁=X₂=H, R₂=Me)

In einem gläsernen 5 l Doppelmantelgefäß werden 217,5 g (1,43 Mol) Isovanillin und 200 g (1,45 Mol) Tyramin in 2,5 l Toluol/n-Butanol (1:1) suspendiert und unter Wasserabscheidung auf Rückflußtemperatur erwärmt. Nach 4 Stunden wird das Lösungsmittel im Vakuum abdestilliert, der Rückstand in 2,5 1 Methanol aufgenommen und die klare Lösung mit 25 g NaBH₄ (0,66 Mol) versetzt. Das Reaktionsgemisch wird bei 0°C 4 Stunden gerührt, der ausgefallene Niederschlag filtriert, mit Methanol gewaschen und getrocknet.
Ausbeute: 332,3 g (85,1%)
Schmelzpunkt: 176-178°C
Molgewicht: C₁₆H₁₉NO₃ : 273,32

### Beispiel 2:

### N-(4-Hydroxyphenethyl)-(6-brom-3,4-dimethoxy)benzylamin (allgemeine Formel (V) mit R₃=R₄=X₂=H, R₁=R₂=Me, X₁ = Br)

In einen 100 ml Rundkolben werden 2,45 g (10 mMol) 6-Brom-3,4-dimethoxybenzaldehyd, 1,37 g (10 mMol) Tyramin in 50 ml Toluol/n-Butanol (1:1) suspendiert und unter Wasserabscheidung auf Rückflußtemperatur erwärmt. Nach 3 Stunden wird das Lösungsmittel im Vakuum abdestilliert, der Rückstand in 50 ml Methanol aufgenommen und die klare Lösung mit 0,8 g NaBH₄ versetzt. Das Reaktionsgemisch wird bei 0°C 4 Stunden gerührt, die Lösungsmittel im Vakuum abdestilliert, der Rückstand in 100 ml CH₂Cl₂ aufgenommen und die organische Phase mit zweimal 10 ml Wasser gewaschen. Die organische Phase wird über Na₂SO₄ getrocknet, filtriert und das Lösungsmittel im Vakuum entfernt. Der zurückgebliebene Rückstand wird auf 150 g Kieselgel mit Hexan:Ethylacetat=2:8 chromatographiert.
Ausbeute: 2,95 g (80,6%) viskoses Öl
Molgewicht: C₁₇H₂₀BrNO₃ : 366,23

### Beispiel 3:

### N-(4-Hydroxyphenethyl)-(4-methoxy-3-methoxymethoxy)benzylamin (allgemeine Formel (V) mit R₁=MeOCH₂O, R₂=Me, X₁=X₂=X₃=X₄=R₃=P₄=H)

In einem 100 ml Rundkolben werden 0,83 g (4,2 mMol) 4-Methoxy-3-methoxymethoxybenzaldehyd [Lit. 16-17] 0,55 g (4,0 mMol) Tyramin in 50 ml Toluol/n-Butanol (1:1) suspendiert und unter Wasserabscheidung auf Rückflußtemperatur erwärmt. Nach 4 Stunden wird das Lösungsmittel im Vakuum abdestilliert, der Rückstand in 50 ml Methanol aufgenommen und die klare Lösung mit 0,35 g NaBH₄ versetzt. Das Reaktionsgemisch wird bei 0°C 4 Stunden gerührt, die Lösungsmittel im Vakuum abdestilliert, der Rückstand in 100 ml CH₂Cl₂ aufgenommen und die organische Phase mit zweimal 10 ml Wasser gewaschen. Die organische Phase wird über Na₂SO₄ getrocknet, filtriert und das Lösungsmittel im Vakuum entfernt. Der zurückgebliebene Rückstand wird auf 65 g Kieselgel mit Ethylacetat:Methanol = 7:3 chromatographiert.
Ausbeute: 1,12 g (83,4%) viskoses Öl
Molgewicht: C₁₈H₂₃NO₄ : 317,37

### Beispiel 4:

### N-(4-Hydroxyphenethyl)-(6-brom-3-hydroxy-4-methoxy)benzylamin (allgemeine Formel (V) mit R₁=R₂=R₄=H, X₂=H, R₂=Me, X₁=Br)

### Methode 1:

In einen 50 ml Rundkolben werden 1,0 g (4,3 mMol) 6-Brom-4-methoxy-3-hydroxybenzaldehyd [Lit. 18] 0,6 g (4,3 mMol) Tyramin in 20 ml Toluol/n-Butanol (1:1) suspendiert und unter Wasserabscheidung auf Rückflußtemperatur erwärnt. Nach 90 min wird das Lösungsmittel im Vakuum abdestilliert, der Rückstand in 20 ml Methanol aufgenommen und die klare Lösung mit 0,33 g NaBH₄ versetzt. Das Reaktionsgemisch wird bei 0°C 4 Stunden gerührt, die Lösungsmittel im Vakuum abdestilliert, der Rückstand in 50 ml CH₂Cl₂ aufgenommen und die organische Phase mit zweimal 10 ml Wasser gewaschen. Die organische Phase wird über Na₂SO₄ getrocknet, filtriert und das Lösungsmittel im Vakuum entfernt. Der zurückgebliebene Rückstand wird auf 60 g Kieselgel mit Ethylacetat: Methanol=97:3→95:5 chromatographiert.
Ausbeute: 1,43 g (93,8%)

### Methode 2:

In einen 1 l Rundkolben werden 53,38 g (231 mMol) 6-Brom-4-methoxy-3-hydroxybenzaldehyd [Lit. 18], 31,7 g (231 mMol) Tyramin in 530 ml Toluol/n-Butanol (1:1) suspendiert und unter Wasserabscheidung auf Rückflußtemperatur erwärmt. Nach 90 min wird das Lösungsmittel im Vakuum abdestilliert, der Rückstand in 350 ml Methanol aufgenommen und die Suspension mit 12 g NaBH₄ versetzt. Das Reaktionsgemisch wird bei 0°C 1 Stunde gerührt und auf 3 l Eiswasser getropft. Nach 30-minütigem Rühren wird das ausgefallene Produkt filtriert, mit Wasser zweimal gewaschen und im Vakuumtrockenschrank bei 60°C getrocknet.
Ausbeute: 70,2 g (86,3%)
Schmelzpunkt: 122-125°C
Molgewicht: C₁₆H₁₈BrNO₃:352,21
IR /KBr): 655,76w; 800,45m, 824,97m; 1022,56m; 1165,88m; 1245,88s; 1409,83s; 1448,40s; 1510,79s; 1554,48s; 3200-3370br.
¹H-NMR(DMSO-d₆): 7,0-6,60 (m, 6 H); 6,73 (m, 2H); 3,77 (s, 3H); 2,75-2,58 (m, 4H); 2,88 (s, 2 OH). ¹³C-NMR(CDCl₃+DMSO-d₆): 155,46 s, 147,28 s, 145,95 s, 130,56 s, 129,68 s, 129,12 2d, 116,93 d, 115,61 d, 114,99 2d, 110,95 s, 55,85 q, 51,76 t, 50,16 t, 34,50 t.

### Beispiel 5:

### N-(4-Hydroxyphenethyl)-(4-methoxy-3-t-butylcarbonyloxy)benzylamin (allgemeine Formel (V) mit R₁=Me₃CCO, R₂=Me, X₁=X=R₃=R₄=H)

In einen 50 ml Rundkolben werden 3,63 g (16,5 mMol) (4-Methoxy-3-t-butylcarbonyloxy)benzaldehyd und 2,06 g (15 mMol) Tyramin in 32 ml Toluol/n-Butanol=1:1 suspendiert und unter Wasserabscheidung auf Rückflußtemperatur erwärnt. Nach 3 Stunden wird das Lösungsmittel im Vakuum abdestilliert, der Rückstand in 32 ml Methanol aufgenommen und die klare Lösung mit 1,32 g NaBH₄ versetz. Das Reaktionsgemisch wird bei 0°C 4 Stunden gerührt, die Lösungsmittel im Vakuum abdestilliert, der Rückstand in 50 ml CH₂Cl₂ aufgenommen und die organische Phase mit zweimal 10 ml Wasser gewaschen. Die organische Phase wird über Na₂SO₄ getrocknet, filtriert und das Lösungsmittel im Vakuum entfernt. Der zurückgebliebene Rückstand wird auf 140 g Kieselgel mit Ethylacetat:Methanol=9:1→8:2 chromatographiert.
Ausbeute: 1,7 g (28,8%) viskoses Öl
Molgewicht: C₂₁H₂₇NO₄ : 357,43

### Beispiel 6:

### N-Formyl-N-(4-hydroxyphenethyl)-(3-hydroxy-4-methoxy)benzylamin (allgemeine Formel (V) mit: R₁=R₃=X₁=X₂=H, R₂=Me, R₄=CHO)

In einen 10 1 3-Halskolben (Tropftrichter Rückflußkühler, Blasenzähler, Gaseinleitrohr) werden 370 g (1,35 Mol) Verbindung 5(R₁=R₃=R₄=X₁=X₂=H, R₂=Me), 5 1 technisches Dioxan und 370 ml technisches DMF vorgelegt. Der Tropftrichter wird mit einer Mischung von 1100 ml (13,66 Mol) HCOOEt und 10 ml HCOOH gefüllt, die Suspension unter Argon magnetisch gerührt und bis zum Sieden erhitzt. Die Innentemperatur steigt bis 100 bis 103°C wobei die Suspension homogen wird. Zu dieser Lösung wird die Lösung aus dem Tropftrichter in 20 bis 30 min zugegeben. Die Innentemperatur senkt sich dabei bis 87 bis 89°C. Die trüb gewordene Reaktionsmischung wird 4 Stunden bei Rückflußtemperatur gerührt. Das Lösungsmittel wird im Vakuum entfernt und der Rückstand portionsweise mit 8 l Eiswasser versetzt. Die ausgefallenen Kristalle werden filtriert, dreimal mit 2 1 Wasser gewaschen und im Vakuum 12 Stunden getrocknet.
Ausbeute: 360,5 g (88,6%)
Schmelzpunkt: 144 bis 148°C
Molgewicht: C₁₇H₁₉NO₄:301,33

### Beispiel 7:

### N-Formyl-N-(4-hydroxyphenethyl)-(6-brom-3,4-dimethoxy)benzylamin (allgemeine Formel (V) mit: R₃=X₂=H, X₁=Br, R₁=R₂=Me, R₄=CHO)

In einem 250 ml 3-Halskolben (Tropftrichter, Rückflußkühler, Blasenzähler, Gaseinleiterrohr) wird eine Mischung von 4,53 g (12,2 mMol) 5 (R₃=R₄=X₂=H, X₁=Br, R₁=R₂=Me), 100 ml technisches Dioxan 10,0 ml (122,0 mMol) HCOOEt und 0,1 ml HCOOH unter Rückfluß gekocht. Nach 68 Stunden wird das Lösungsmittel im Vakuum entfernt und der Rückstand aus 40 ml MeOH kristallisiert.
Ausbeute: 3,61 g (75%)
Schmelzpunkt: 160 bis 162°C
Molgewicht: C₁₈H₂₀BrNO₄ : 394,24

### Beispiel 8:

### N-Formyl-N-(4-hydroxyphenethyl)-(4-methoxy-3-t-butylcarbonyloxy)benzylamin (allgemeine Formel (V), mit R₁=Me₃CCO, R₂=Me, X₁=X₂=R₃=H, R₄=CHO)

In einem 500 ml 3-Halskolben wird eine Mischung von 1,7 g (4,7 mMol) der Verbindung der Formel (V) (R₁=Me₃CCO, R₂=Me, X₁=X₂=R₂=R₄=H), 7,5 ml technisches Dioxan, 7,5 ml HCOOEt und einem Tropfen HCOOH unter Rückfluß gekocht. Nach 15 Stunden wird das Lösungsmittel im Vakuum entfernt und der Rückstand auf 30 g SiO₂ mit AcOEt chromatographiert.
Ausbeute: 1,5 g (81,8%) Öl
Molgewicht C₂₂H₂₇NO₅:385,44
¹H-NMR (CDCl₃): 8,20 uns 7,80 (2s, 1 H); 7,16-6,80 (m, 7H); 4,30 (d, 2 H); 3,78 (s, 3 H); 3,35 (m, 2 H); 2,70 (m, 2 H); 1,38 (s, 9 H).
¹³C-NMR (CDCl₃): 176,69 s; 163,24 und 162,90 d; 155,36 und 154,99 s; 150,99 und 150,70 s; 140,35 und 140,18 s; 129,67 bis 112,30 m; 55,85 q; 50,94 und 48,46 t; 44,60 und 43,61 t; 38,94 s; 33,60 und 32,24 t; 27,05 3q.

### Beispiel 9:

### N-Formyl-N-(4-hydroxyphenethyl)-(6-brom-3-hydroxy-4-methoxy) benzylamin (allgemeine Formel (V) mit R₁=R₃=X₂=H, X₁=Br, R₂=Me, R₄=CHO)

### Methode 1:

In einem 500 ml 3-Halskolben (Tropftrichter, Rückflußkühler, Blasenzähler, Gaseinleiterrohr) wird eine Mischung von 27 g (76,6 mMol) die Verbindung (V) (R₁=R₃=R₄=X₂=H, X₁=Br, R₂=Me), 300 ml technisches Dioxan, 30,0 ml (37,2 mMol) HCOOEt und 0,1 ml HCOOH unter Rückfluß gekocht. Nach 72 Stunden wird das Lösungsmittel im Vakuum entfernt und der Rückstand aus 50 ml Chloroform kristallisiert.
Ausbeute: 23,95 g (82,3%)

### Methode 2:

300 g der Verbindung (V) (R₁=R₃=X₁=X₂=H, R₂=Me, R₄=CHO), wurde in 2000 ml wasserfreiem Ethanol und 2000 ml Methylglykol (H₂O<0,1%) durch Erwärmen auf 40°C gelöst, anschließend auf -20°C abgekühlt und 14 ml Brom in 1000 ml Ethanol/Methylglykol (1:1) während 15 min zugetropft, so daß die Temperatur -20°C nicht überstieg. Nun wurde 30 min bei -20°C gerührt, anschließend die Lösung auf ca. 1000 ml eingeengt und unter heftigem Rühren auf 30 l Eis/Wasser gegossen. Es wurde 4 Stunden bei 0°C gerührt, abgesaugt und die farblose, kristalline Substanz im Vakuum (60°C) getrocknet.
Ausbeute: 370,2 g (96% d.Th.); Gehalt (HPLC) 82%
Schmelzpunkt: 162 bis 164°C
Molgewicht: C₁₇H₁₈BrNO₄:380,22

### Beispiel 10:

### N-Formyl-N-(4-hydroxyphenethyl)-(4-methoxy-3-methoxymethoxy)benzylamin (allgemeine Formel (V) mit R₁=MeOCH₂O, R₂=Me, X₁=X₂=R₃=H, R₄=CHO)

In einem 50 ml 3-Halskolben (Tropftrichter, Rückflußkühler, Blasenzähler, Gaseinleiterrohr) wird eine Mischung von 4,9 g (15,4 mMol) der Verbindung (V) (R₁=MeOCH₂O, R₂=Me, X₁=X₂=R₃=R₄=H), 60 ml HCOOEt und ein Tropfen HCOOH unter Rückfluß gekocht. Nach 18 Stunden wird das Lösungsmittel im Vakuum entfernt und der Rückstand aus AcOEt/Hexan kristallisiert.
Ausbeute: 3,95 g (74%)
Schmelzpunkt: 102 bis 104°C
Molgewicht: C₁₉H₂₃NO₅:345,38
¹H-NMR (CDCl₃): 8,23 und 7,83 (2s, 1 H); 7,05 bis 6,70 (m, 7 H); 5,20 (s, 2 H); 4,46 und 4,28 (2s, 2 H); 3,87 (s, 3 H); 3,52 (s, 3 H); 3,38 (m, 2 H); 2,70 (m, 2 H).
¹³C-NMR (CDCl₃): 163.20 und 162,86 d; 155,41 und 155,05 s; 149,53 und 149,30 s; 146,53 und 146,33 s; 129,66 und 129,59 s; 129,52 d; 128,56 und 128,02 s; 122,40 d; 121,64 d; 116,71 d; 115,88 d; 115,60 und 115,33 d; 111,75 d; 95,39 t; 56,13 q; 55,79 q; 51,44 und 48,62 t; 45,10 und 43,71 t; 33,72 und 32,27 t.

### Beispiel 11:

### 4a,5,9,10,11,12-Hexahydro-1-brom-3-methoxy-11-formyl-6H-benzofuro [3a,3,2-ef] [2]benzazepin-6-on (allgemeine Formel (I) mit: R₂=Me, R₄=CHO, X₁=Br, X₂=H, Y₁,Y₂=O)

Zu einer Suspension von 16 l Toluol, 600 g K₃[Fe(CN)₆] und 2 l 10% K₂CO₃-Lösung wird bei 70°C 120 g (0,316 Mol) fein pulverisierte Verbindung (V) (R₁=R₃=X₂=H, X₁=Br, R₂=Me, R₄=CHO) auf einmal zugegeben. Anschließend wird die Reaktionsmischung bei der gleichen Temperatur 30 min unter Zuschalten eines Homogenisators intensiv gerührt, wobei ein unlösliches Polymer ausfällt Die Reaktionsmischung wird filtriert, die organische Phase über Na₂SO₄ getrocknet, filtriert und das Lösungsmittel im Vakuum entfernt.
Ausbeute: 59,6 g (49,9%).

Wird für die Cyclisierung Edukt, hergestellt nach Beispiel 9, Methode 2, eingesetzt, so konnte nach Trennung mittels Säulenchromatographie (Kieselgel 60, CHCl₃/MeOH (1-5%)) Nebenprodukt der allgemeinen Formel (I) mir R₂=CH₃, X₁=X₂=Br, R₄=CHO; Y₁, Y₂ = O in 6% Ausbeute gewonnen werden.
¹H-NMR(CDCl₃): 8,23 (d,1H), 7,30 (s, 1H), 6,98 (s, 1H), 5,85-3,95 (m, 3H), 4,70 (s, 1H), 3,80 (s, 3H), 3,35 (m, 2H), 2,95 (m, 1H), 2,15 (m, 2H).
¹³C-NMR(CDCl₃ + DMSO d₆): 185,31 und 185,25 s, 162,43 und 161,43 d; 147,12 und 146,84 s; 144,61 und 144,37 s; 142,33 und 141,97 d, 129,27 und 129,13 s, 126,62 und 126,40 s, 123,40 und 123,25 s, 116,67 und 116,46 d, 114,27 und 112,74 s, 87,00 und 86,86 d, 56,01 q, 52,38 und 51,55 s, 46,18 und 45,80 t, 40,58 t, 37,68 und 36,86 t, 34,26 t.

### Beispiel 12:

### (4α,6β)-4a,5,9,10,11,12-Hexahydro -3-methoxy-11-methyl-6H-benzofuro[3a,3,2-ef][2]benzazepin-6-ol (Galanthamin) (allgemeine Formel (I) mit: R₂=R₄=Me, X₁=X₂=Y₂=H, Y₁=OH)

### Methode 1:

In einen 1 l 3-Halskolben legt man 4,6 g (121,21 mMol) LiAIH₄ in 80 ml abs. THF vor und kühlt auf 0°C. Zu dieser Suspension werden unter herftigem Rühren 7,36 g (19,47 mMol) der Verbindung (V) (R₁=H, R₄=CHO, X₁=Br, X₂=H, Y₁Y₂=O) in 460 ml abs. THF in 5 min zugetropft, 1 Stunde bei 0°C gerührt und 21 Stunden unter Rückfluß gekocht. Die Reaktionsmischung wird anschließend in einen 1 l Einhalskolben umgefüllt, auf 0°C gekühlt, überschüssiges LiAIH₄ mit einigen Tropfen H₂O zersetzt und das Lösungsmittel im Vakuum entfernt. Der Rückstand wird mit H₂O versetzt und der pH-Wert mit 2N HCl-Lösung auf 1 gestellt. Die Reaktionslösung wird geschütttelt und aufgewärmt, bis der Niederschlag gelöst ist. Der pH-Wert wird anschließend mit 2N NaOH auf pH 9 eingestellt, die trübe Lösung mit Ethylacetat versetzt, gut geschüttelt und der ausgefallene Niederschlag abfiltriert. Die organische Phase wird abgetrennt und die wässerige Phase mit Ethylacetat extrahiert. Die vereinigten organischen Phasen werden über Na₂SO₄ getrocknet, filtriert und das Lösungsmittel im Vakuum entfernt. Chromatographische Reinigung des Rückstandes (300 g SiO₂, mit CHCl₃:MeOH=97:3→95:5) ergibt farblose Kristalle.
Ausbeute: 2,23 g (40,03%)

### Methode 2:

Zu einer Suspension von 240 mg (6,3 mMol) LiAIH₄ in 4 ml abs. THF wird eine Lösung von 365 mg (1,0 mMol) der Verbindung (I) (R₂=R₄=Me, X₁=Br, X₂=Y₂=H, Y₁=OH) in 4 ml wasserfreiem THF bei 0°C zugetropft, eine Stunde bei Raumtemperatur und anschließend 23 Stunden bei Rückflußtemperatur gerührt. Nun wird die Reaktionsmischung auf 0°C gekühlt, überschüssiges Reduktionsmittel mit H₂O zersetzt, mit 50 ml Ethylacetat und 50 ml cc. NH₄OH verdünnt. Nach dem Schütteln wird der ausgefallene Niederschlag filtriert, die organische Phase getrennt und die wässerige Phase mit Ethylacetat gewaschen. Die vereinigten organischen Phasen werden über Na₂SO₄ getrocknet, filtriert und das Lösungsmittel im Vakuum entfernt. Chromatographische Reinigung des Rückstandes (25 g SiO₂, CHCl₃:MeOH=99:1→96:4) ergibt 140 mg (49%) der Verbindung (I) (R₂=R₄=Me, X₁=X₂= -Y₂=H, Y₁=OH).

### Methode 3:

Zu einer Suspension von 100 mg (0,27 mMol) der Verbindung (I) (R₂=R₄=Me, X₁=Br, X₂=H=Y₂=H, Y₁=OH) und 10 mg 10% Pd/C in 3 ml Et₃N wird 1,0 ml HCOOH tropfenweise zugegeben. Nach 2,5-stündigem Rühren bei Rückflußtemperatur wird das Pd/C durch Celite abfiltriet, das Lösungsmittel im Vakuum entfernt und der Rückstand in CH₂Cl₂ aufgenommen. Die organische Lösung wird zweimal mit gesättigter NH₄Cl-Lösung, einmal mit H₂O gewaschen, mit Na₂SO₄ getrocknet und das Lösungsmittel im Vakuum eingedampft. Der Rückstand wird mittels Säulenchromatographie getrennt (9 g SiO₂, CHCl₃:MeOH=95:5).
Ausbeute: 62 mg (79%) Verbindung (I) (R₂=R₄=Me, X₁=X₂=Y₂=H, Y₁=OH)
Schmelzpunkt: 119 bis 121°C
Molgewicht C₁₇H₂₁NO₃:287,34

### Beispiel 13:

(4α6β)-4a,5,9,10,1 1,12-Hexahydro-1-brom-3-methoxy-11-methyl-6H-benzofuro[3a,3,2-ef] [2]benzazepin-6-ol (Bromgalanthamin)
(allgemeine Formel (I) mit: R₂=R₄=Me, X₁=Br, X₂=Y₂=H, Y₁=OH) und
(4α6α)-4a,5,9,10,1 1,12-Hexahydro-1-brom-3-methoxy-11-methyl-6H-benzofuro[3a,3,2-ef] [2]benzazepin-6 ol (Epibromgalanthamin)
(allgemeine Formel (I) mit: R₂=R₄=Me, X₁=Br, X₂=Y₁=H, Y₂=OH)

Zu einer Suspension von 8,0 g (21 mMol) der Verbindung (V) (R₂=Me, R₄=CHO, X₁=Br, X₂=H, Y₁,Y₂=O) in 150 ml Toluol wird 10 ml (36 mMol) 1,5M DIBAl-H-Lösung in Toluol bei 0°C zugetropft. Die Reaktion wird 1 Stunde bei Raumtemperatur gerührt, das restliche Reduktionsmittel mit H₂O zersetzt und anschließend 12 ml konzentriertes NH₄OH zugegeben. Nach 20-minütigem Rühren bei Raumtemperatur wird das ausgefallene Material abfiltriert, die organische Phase abgetrennt und die wässerige Phase mit 50 ml Toluol gewaschen. Die vereinigten organischen Phasen werden über Na₂SO₄ getrocknet, filtriert und das Lösungsmittel im Vakuum entfernt. Der Rückstand (7,7 g) wird mittels Säulenchromatographie getrennt.
Ausbeute: 3,2 g (45,1%) Verbindung (I) mit (R₂=R₄=Me, X₁=Br, X₂=Y₂=H, Y₁=OH) und 0,8 g (20,7%) Verbindung (I)

(R₂=R₄=Me, X₁=Br, X₂=Y₁=H, Y₂=OH)

Daten von Bromgalanthamin (Verbindung (I) mit R₂=R₄=Me, X₁=Br, X₂=Y₂=H, Y₁=OH):
Molgewicht: C₁₇H₁₉BrNO₃: 365,23
IR(KBr): 689,03m; 778,57m; 839,37m; 989,86m; 1050,66s; 1212,43s; 1279,87s; 1434,08s; 14,72s; 1613,99s; 2667,39m; 3370 bis 3778br
¹H-NMR (CDCl₃): 6,9 (s, 1 H); 6,06 (m, 2 H); 4,60 (d, 1 H); 4,15 (t, 1 H); 3,92 (d, 1 H); 3,82 (s, 3 H); 3,24 (m, 1 H); 2,98 (dt, 1 H); 2,68 (dd, 1 H); 2,42 (s, 3 H); 2,05 (m, 2 H); 1,60 (dt, 1 H).
¹³C-NMR (CDCl₃): 145,32 s; 144,00 s, 133,96 s; 127,95 d; 127,68 s; 126,51 d; 115,61 d; 114.22 s; 88,56 d; 61,58 d; 58,56 t; 55,95 q; 53,26 t; 48,56 s; 42,06 q; 33,47 t; 29,69 t.

Daten von Epibromgalanthamin (Verbindung (I) mit R₂=R₄=Me, X₁=Br, X₂=Y₁=H, Y₂=OH):
Molgewicht: C₁₇H₁₉BrNO₃: 365,23
IR(KBr): 667,95w; 752m; 836,68m; 1040,31s; 1208,39s; 12,82m; 1435,25m; 1485,72m; 1512,94w; 1558,27w; 1615,19m; 1667,14w; 2943,24w; 3360 bis 3575br.
¹H-NMR (CDCl₃): 6,85 (s, 1 H); 5,96 (AB, 2H); 4,69 (m, 2 H); 4,28 (d, 1 H); 3,90 (d, 1 H); 3,83 (s, 1H); 3,25 (m, 1 H), 2,95 (m, 1 H); 2,85 (dt, 1 H); 2,36 (s 3 H); 2,15 (td, 1 H); 1,69 (m, 2 H).
¹³C-NMR (CDCl₃ + DMSO-d₆): 145,84 s; 143,49 s; 133,89 s; 133,14 d; 126,12 s; 124,35 d; 115,04 s; 113,01 s; 88,26 d; 61,10 d; 57,44 t; 55,58 q; 52,84 t; 47,86 s; 41,20 q; 33,35 t; 31,43 t.

### Beispiel 14:

### (4α6α)-4a,5,9,10,1 1,12-hexahydro-3 -methoxy-11-methyl6H-benzofuro[3a,3,2-ef][2]benzazepin-6-ol (Epigalanthamin) (allgemeine Formel (I) mit: R₂=R₄=Me, X₁=X₂=Y₁=H, Y₂=OH)

Zu einer Suspension von 240 mg (6,3 mMol) LiAlH₄ in 4 ml wasserfreiem THF wird eine Lösung von 365 mg (1,0 mMol) Verbindung (I) (R₂=R₄=Me, X₁=Br, X₂=Y₁=H, Y₂=OH) in 4 ml abs. THF bei 0°C zugetropft, eine Stunde bei Raumtemperatur und anschließend 23 Stunden bei Rückflußtemperatur gerührt. Nun wird die Reaktionsmischung auf 0°C gekühlt, überschüssiges Reduktionsmittel mit H₂O zersetzt, 50 ml Ethylacetat und mit 50 ml konzentrierter NH₄OH verdünnt. Nach dem Schütteln wird der ausgefallene Niederschlag filtriert, die organische Phase abgetrennt und die wässerige Phase mit Ethylacetat gewaschen. Die vereinigten organischen Phasen werden über Na₂SO₄ getrocknet, filtriert und das Lösungsmittel im Vakuum entfernt. Der Rückstand wird mittels Säulenchromatographie getrennt (25 g SiO₂), CHCl₃:MeOH=99:1→96:4).
Ausbeute: 140 mg (49%) 1 (R₂=R₄=Me, X₁=X₂=Y₁=H, Y₂=OH)
Schmelzpunkt: 199 bis 201°C
Molgewicht: C₁₇H₂₁NO₃:287,34

### Beispiel 15:

### (4α6β)-4a,5,9,10,11,12-hexahydro-1-brom-3-methoxy-6H-benzofuro[3a,3,2-ef] [2]benzazepin-6-ol (N-Demethyl-bromgalanthamin) (allgemeine Formel (I) mit: R₂=Me, X₁=Br, R₄=X₂=Y₂=H, Y₁=OH)

Zu einer Suspension von 10 g (26,4 mMol) (I) (R₂=Me, R₄=CHO, X₁=Br, X₂=H, Y₁=Y₂=O) in 200 ml THF wird 100 ml (100 mMol) 1 M Lösung von L-Selektride bei 0°C in 30 min zugetropft. Nach 60-minütigem Rühren bei 0°C wird der mit dem Reagenz gebildete Komplex mit H₂O zersetzt und die Reaktionsmischung mit 100 ml 25%iger NH₄OH-Lösung versetzt. Nach 30-minütigem Rühren bei Raumtemperatur wird das Lösungsmittel im Vakuum auf die Hälfte konzentriert, in einen Schütteltrichter überführt, mit 100 ml 25%iger NH₄OH-Lösung versetzt und mit 3 x 200 ml CH₂Cl₂ extrahiert. Die vereinigten organischen Phasen werden mit Na₂SO₄ getrocknet, filtriert und das Lösungsmittel im Vakuum abgedampft. Chromatographische Reinigung des Rückstandes (650 g SiO₂ Kieselgel CJCl₃:MeOH=95:5→9:1) ergibt farblosen Schaum.
Ausbeute: 7,3 g (75,8%)
C₁₆H₁₈BrNO₃: 352,21
IR(KBr): 748,19 m; 793,11 m; 828,59m; 927,62w; 991,65w; 1058,8s; 1214,79s; 1281,61s; 14,29s; 1488,49s; 1571,11w; 1616,51s; 2912,36s; 3280 bis 3420br.
UV(MeOH): λₘₐₓ: 225,0 und 297,5 nm.
¹H-NMR (CDCl₃): 6,85 (s, 1H); 6,02 (AB, 2 H); 4,53 (s, 1H); 4,81 und 4,48 (AB, 2H); 4,10 (m, 1H); 3,78 (s, 3H); 3,22 (m, 2H); 2,63 (dd, 1H); 2,29 (s, br, 2H); 2,00 (m 1H); 1,78 (m, 2H).
¹³C-NMR (CDCl₃): 145,79s; 143,96s; 134,06s; 131,64s; 127,87d; 126,83d; 115,46d; 113,02s; 88,44d; 61,67d; 56,04q; 52,65t; 49,23s; 46,59t; 39,81tt; 29,71t.

### Beispiel 16:

### (4α6β)-4a,5,9,10,11,12-hexahydro-1-brom-3-methoxy-6H-benzofuro[3a,3,2-ef] [2]benzazepin-6-ol (N-Demethyl-bromgalanthamin) (allgemeine Formel (I) mit: R₂=Me, X₁=Br, R₄=X₂=Y₂=H, Y₁=OH) und (4α6β)-4a,5,9,10,11,12-hexahydro-1-brom-3-methoxy-6H-benzofuro[3a,3,2-ef] [2]benzazepin-6-ol (N-Demethyl-epibromgalanthamin) (allgemeine Formel (I) mit: R₂=Me, X₁=Br, R₄=X₂=Y1=H, Y2=OH)

Zu einer Suspension von 1,0 g (2,6 mMol) (I) (R₂=Me, R₄=CHO, X₁=Br, X₂=H, Y_{**1**}=Y_{**2**}=O) in 5 ml THF wird 3,0 g (11,8 mMol) LiAIHt-B₄O)₃ in 15 ml THF 0°C in 30 min zugetropft. Nach 30-minütigem Rühren bei 0°C wird die Reaktionsmischung unter Rückfluß gekocht. Nach 22-stündigem Kochen wird der mit dem Reagenz gebildete Komplex mit H₂O zersetzt und die Reaktionsmischung mit 10 ml 25%iger NH₄OH-Lösung versetzt. Nach 30-minütigem Rühren bei Raumtemperatur wird das Lösungsmittel im Vakuum auf die Hälfte konzentriert, in einen Schütteitrichter überführtt mit 10 ml 25%iger NH₄OH-Lösung versetzt und mit 3 x 20 ml CH₂Cl₂ extrahiert. Die vereinigten organischen Phasen werden mit Na₂SO₄ getrocknet, filtriert und das Lösungsmittel in Vakuum eingedampft. Chromatographische Reinigung des Rückstandes (60 g SiO₂ Kieselgel CHCl₃:MeOH=95:5→9:1→8:2) ergibt zwei Produkte.
300,0 mg (32,2% N-Demethyl-bromgalanthamin (allgemeine Formel (I) mit R₂=Me, X₁=Br, R₄=X₂= -Y₂=H, Y₁=OH) als farblosen Schaum und 270 mg (29,0% N-Demethyl-bromgalanthamin (allgemeine Formel (I) mit R₂=Me, X₁=Br, R₄=X₂=Y₁=H, Y₂=OH) als farblosen Schaum.

Daten von N-Demethyl-epibrom-galanthamin:
Molgewicht: C₁₆H₁₈BrNO₃: 352,21
IR(KBr): 781,60w; 834,28w; 976,63w; 1050,28m; 1179,73m; 1211,87m; 1280,07m; 1435,24m; 1486,10m; 1616,37m; 2923,54w; 3700-2900mbr.
¹H-NMR (CDCl₃): 6,86 (s, 1H); 5,92 (AB, 2H); 4,56 (m, 2H); 4,50 und 3,82 (AB, 2H); 3,80 (s, 3H); 3,28 (m, 2H); 2,52 (m, 1H); 2,20-1,70 (m, 3H).
¹³C-NMR (CDCl₃): 146,73s; 143,91s; 134,10s; 132,17s; 132,17d; 131,48d; 126,34d; 115,34d; 112,44s; 88,51d; 62,81d; 56,10q; 52,34t; 49,25s; 46,82t; 40,52t; 32,07t.

### Beispiel 17:

### (4α6β)-4a,5,9,10,11,12-hexahydro-1-brom-3-methoxy-11-methyl-6H-benzofuro[3a,3,2-ef] [2]benzazepin-6-ol (N-Demethyl-bromgalanthamin) (allgemeine Formel (I) mit: R₂=R₄=Me, X₁=Br, X₂=Y₂=H, Y₁=OH)

### Methode 1:

Zu einer Lösung von 2,0 g (5,6 mMol) (I) (R₂=Me, X₁=Br, R₄=X₂=Y₂=H, Y₁=OH) in 20 ml H₂O werden 5 ml 89%iger HCOOH, 5 ml 37%iger CH₂O gegeben und unter Rüclkfluß gekocht. Nach 15-minütigem Kochen wird die Reaktionsmischung mit H₂O verdünnt, der pH-Wert mit 25%iger NH₄OH auf 9 eingestellt und mit 3 x 20 ml CH₂Cl₂ extrahiert. Die vereinigten organischen Phasen werden mit Na₂SO₄ getrocknet, filtriert und das Lösungsmittel im Vakuum eingedampft. Chromatographische Reinigung des Rückstandes (150 g SiO₂ Kieselgel CHCl₃:MeOH=g7:→45:5) ergibt farblosen Schaum.
Ausbeute: 2,0 g (96,4%)

### Methode 2:

Zu einer Suspension von 10 g (26,4 mMol) (I) (R₂=Me, R₄=CHO, X₁=Br, X₂=H, Y₁=Y₂=O) in 200 ml THF wird 100 ml (100 mMol) 1 M Lösung von L-Selektride bei 0°C in 30 min zugetropft. Nach 60-minütigem Rühren bei O°C wird der Reagenz mit H₂O zersetzt und die Reaktionsmischung mit 100 ml 25%iger NH₄OH-Lösung versetzt. Nach 30-minütigem Rühren bei Raumtemperatur wird das Lösungsmittel im Vakuum auf die Hälfte konzentriert, in einen Schütteltrichter überführt, mit 100 ml 25%iger NH₄OH versetzt und mit 3 x 200 ml CH₂Cl₂ extrahiert. Die vereinigten organischen Phasen werden mit Na₂SO₄ getrocknet, filtriert und das Lösungsmittel im Vakuum eingedampft. Zum Rückstand werden 50 ml H₂O, 30 ml 98%ige HCOOH, 30 ml 37%ige CH₂O-Lösung gegeben und die Reaktionsmischung unter Rückfluß gekocht. Nach 15-minütigem Kochen wird die Reaktion mit NH₄OH neutralisiert und mit 3 x 200 ml CH₂Cl₂ extrahiert. Die vereinigten organischen Phasen werden über Na₂SO₄ getrocknet, filtriert und das Lösungsmittel im Vakuum eingedampft. Chromatographische Reinigung des Rückstandes (600 g SiO₂ Kieselgel CHCl₃:MeOH=9:1→8:2) ergibt farblosen Schaum.
Ausbeute: 6,4 g (66,2%).

### Beispiel 18:

### Optische Trennung von (±)Galanthamin

Eine Lösung jvon 500 mg (±)Galanthamin (1,74 mMol) Verbindung (I) (R₂=R₄=Me, X₁=X₂=Y₂=H, Y₁=OH) in 1,0 ml MeOH wird mit einer Lösung von 672,2 mg (1,74 mMol) (+)Di-p-Toluyl-D-weinsäure in 4 ml MeOH bei Raumtemperatur versetzt. Nach 24-stündigem Stehenlassen im Kühlschrank wird die ausgefallene kristalline Substanz filtriert und mit MeOH gewaschen. Die Mutterlauge wird für das andere Isomer aufgehoben. Umkristallisieren aus EtOH ergibt 450 mg(-) Galanthamin-(+)Di-p-Toluoyl-tartrat (Verbindung (II) R₂=R₄=Me, R₅=X₁=X₂=Y₂=H, Y₁=OH, Z=(+)Di-p-Toluoyl-tartrat), Schmelzpunkt: 182 bis 184°C. Die freie Base wird mit CHCl₃/NH₄OH aus dem Salz freigesetzt [α]_{D}= -101,8°.

Die methanolische Mutterlauge wird eingedampft, die Base mit CHCl₃/NH₄OH freigesetzt, in 0,5 ml MeOH aufgelöst und mit einer Lösung von 215 mg (0,55 mMol) (-)Di-p-Toluyl-L-weinsäure versetzt. Nach 24-stündigem Stehenlassen im Kühlschrank wird das ausgefallene Material filtriert und mit MeOH gewaschen. Umkristallisieren aus EtOH ergibt 242 mg (+)Galanthamin-(-)Di-p-Toluoyl-tartrat (Verbindung (II) R₂=R₄=Me, R₅=X₁=X₂=Y₂=H, Y₁=OH, Z=(-)Di-p-Toluoyl-tartrat), Schmelzpunkt: 144 bis 148°C. Das Salz wird in die freie Base mit CHcl₃/NH₄OH konventiert. [α]_{D}= +98,9°.

### Beispiel 19:

### 4a,5,9,10,11,12-Hexahydro-1-brom-3-methoxy-11-formyl-6H-benzofuro[3a,3,2-2-ef][2]benzazepin-6-ethylenketal (allgemeine Formel (I) mit R₂=Me, R₄=CHO, X₁=Br, X₂=H, Y₁, Y₂=-O-(CH₂)₂-O-)

5,0 g Verbindung (I) mit R₂=Me, R₄=CHO, X₁=Br, X₂=H, Y₁, Y₂=O, 10,0 g Ethylenglykol und 0,05 g p-TsOH wurden in 100 ml Toluol (bei Raumtemperatur 2-phasig) unter heftigem mechanischem Rühren auf Rückflußtemperatur erwärmt (ab ca. 90°C homogen) und 2 Stunden unter Wasserabscheidung gekocht. Nach dem Abkühlen wurden die Phasen getrennt (wobei die Toluolphase die obere Phase ist), die Ethylenglykolphase 2 mal mit 20 ml Toluol extrahiert, die vereinten Toluolphasen mit 2 x 50 ml ges. NaHCO₃ Lösung geschüttelt, über Na₂SO₄ getrocknet und eingedampft.
Ausbeute: 5,40 g gelblicher Schaum (96,7% d.Th. roh), der über Nacht kristallisierte. Säulenchromatographie von 1,0 g (60 g Kieselgel 60, CHCl₃/ 1 bis 2% MeOH) ergab: 0,62 g farbloser Schaum, der aus EtOAc kristallisierte.
Schmelzpunkt: 212 bis 214°C
Molgewicht: C₁₉H₂₀BrNO₅: 422,28
¹H-NMR (CDCl₃): 8,12 (d, H), 6,87 (s, H), 6-06 (t, H), 5,72 (d,H), 5,64 (d, H/2), 5,11 (d,H/2), 4,54 (b, H), 4,48 (d, H/2), 4,31 (d, H/2), 3,50-4,10 (m, 6H), 3,82 (s, 3H), 2,65 (d, H), 2,27 (d, H), 1,74-2,10 (m, 2H).
¹³C-NMR (CDCl₃): 162,40, 161,65, 147,08, 144,81, 144,55, 132,14, 131,96, 127,68, 127,48, 115,68, 115,43, 126,71, 126,44, 113,12, 111,59, 102,04, 87,07, 86,90, 65,14, 64,23, 55,88, 51,43, 46,11,
48,41, 40,67, 39,27, 35,96, 32,94.

### Beispiel 20:

### 4a,5,9,10,11,12-Hexahydro-1-brom-3-methoxy-11-formyl-6H-benzofuro[3a,3,2-ef][2]-benzazepin-6-propylenketal (allgemeine Formel (I) mit R₂=Me, R₄=CHO, X₁=Br, X₂=H, Y₁, Y₂=-O-CH₂-CH(CH₃)-O-)

100 g Verbindung (I) mit R₂=Me, R₄=CHO, X₁=Br, X₂=H, Y₁, Y₂=O, 100 g Propylenglykol und 0,5 g H₂SO₄ wurden in 800 ml Toluol (bei Raumtemperatur 2-phasig) unter heftigem mechanischem Rühren auf Rüclkflußtemperatur erwärmt (ab ca 90°C homogen) und 14 Stunden unter Wasserabscheidung gekocht. Nach dem Abkühlen wurden die Phasen getrennt (wobei die Toluolphase die obere Phase ist), die Propylenglykolphase 2 mal mit 100 ml Toluol extrahiert, die vereinten Toluolphasen mit 2 x 200 ml ges. NaHCO₃-Lösung geschüttelt, über Na₂SO₄ getrocknet und eingedampft.
Ausbeute: 115,3 g gelblicher Schaum (100% d.Th. roh), der über Nacht kristallisierte. Säulenchromatographie von 1,0 g (60 g Kieselgel 60, CHCl₃/ 1 bis 2% MeOH) ergab: 0,80 g farbloser Schaum, der aus EtOAc kristallisierte.
Schmelzpunkt: 170 bis 171°C
Molgewicht C₂₀H₂₂BrNO₅: 436,28
¹H-NMR (CDCl₃): 8,12 (d, H), 6,88 (s, H), 5,96-6,17 (m, H), 5,75 (dd, H), 5,68 (d, H/2), 5,10 (d, H/2), 4,53 (b, H), 4,48 (d, H/2), 4,31 (d, H/2), 3,12-4,38 (m, 5H), 3,82 (s, 3H), 2,56-2,80 (m, H), 2,05-2,35 (dd, H), 1,83-2,05 (m, 2H), 1,22-1,47 (m, 3H).
¹³C-NMR(CDCl₃): 162,48, 161,72, 147,17, 144,89, 144,64, 132,16, 129,04, 128,51, 128,57, 127,82, 127,70, 127,61, 115,70, 115,48, 127,09, 126,77, 126,5, 113,20, 111,66, 102,38, 102,22, 87,25, 87,07, 73,38, 72,46, 71,67, 71,41, 71,23, 70,55, 70,28, 55,92, 51,52, 46,18, 48,43, 40,77, 39,29, 36,07, 35,97, 34,58, 33,68, 33,44, 33,13, 18,68, 17,59, 17,45.

Anmerkung NMR₁ Diastereomere: Aufgrund des zusätzlich eingeführten chiralen Zentrums mittels der (+/-)Propylengruppe kommt es zur Bildung von Diastereomeren, die eine zusätzliche Signalaufspaltung zu jener, von der Formylgruppe verursachten, bewirken.

### Beispiel 21:

### 4a,5,9,10,11,12-Hexahydro-3-methoxy-11-methyl-6H-benzofuro[3a,3,2-ef][2]benzazepin-6-ethylenketal (allgemeine Formel (I) mit R₂=Me, R₄=CH₃, X₁=X₂=H, Y_{1"} Y₂=-O-CH₂-CH₂-O-)

2,0 g Verbindung (I) mit R₂=Me, R₄=CHO, X₁-Br, X₂=H, Y₁, Y₂=O-(CH₂)₂-O- wurden in 50 ml wasserfreiem THF suspendiert, auf 0°C abgekühlt, und 20 ml LiAIH₄/Diethylether (1M) über 5 min zugetropft und auf Raumtemperatur erwärmt. Nun wurde 12 Stunden bei Rückflußtemperatur (45 bis 52°C) gerührt, abgekühlt, 3 ml THF/Wasser (2:1) zugetropft, mit 50 ml NH₄OH (25%) alkalisch gemacht und 4 mal mit 50 ml EtOAc extrahiert. Die organischen Phasen wurden über Na₂SO₄ getrocknet und eingedampft.
Ausbeute: 1,52 g gelbliches Öl (92,9% d.Th.).
Säulenchromatographie (80 g Kieselgel 60, EtOAc/MeOH 8:2) ergab: 0,82 g farblose Kristalle Schmelzpunkt: 109-110°C
Molgewicht: (C₁₉H₂₃NO₄): 329,40
¹H-NMR (CDCl₃): 1,65 (ddd, 1H), 2,10 (ddd, 1H), 2,15 (dd, 1H), 2,40 (s, 3H), 2,65 (dd, 1H), 3,05 (ddd, 1H), 3,20 (ddd, 1H), 3,60 (d, 1H), 3,80 (s, 3H), 3,90-4,05 (m, 4H), 4,10 (d, 1H), 4,55 (dd, 1H), 5,65 (d, 1H), 6,15 (d, 1H), 6,55, 6,60 (2x d, 2H)
¹³C-NMR(CDCl₃): 33,2 (t), 33,8 (t), 41,7 (q), 47,8 (t), 53,8 (s), 55,5 (q), 60,2 (t), 64,0, 65,0 (2x t), 87,1 (d), 102,5 (s), 110,9 (d), 121,1 (d), 125,9 (d), 128,7 (s), 128,9 (s), 131,8 (d), 143,8 (s), 146,5 (s).

### Beispiel 22:

### 4a,5,9,10,1 1,12-Hexahydro-3-methoxy-11-methyl-6H-benzofuro[3a,3,2-ef][2]benzazepin-6-(2-hydroxyethyl)ether (allgemeine Formel (I) mit R₂=Me, R₄=CH₃, X₁=X₂=H, Y₁, =-O-CH₂-CH₂-OH, Y₂=H)

1,0 g Verbindung (I) mit R₂=Me, R₄=CHO, X₁=Br, X₂=H, Y₁, Y₂=O-(CH₂)₂-O- wurden in 25 ml THF gelöst, auf 0°C abgekühlt, 9 ml LiAlH₄ /THF (1M) über 5 min zugetropft und 30 min bei 0°C gerührt. Nun wurde 48 Stunden auf Rückflußtemperatur erwärmt, abgekühlt, 25 ml NH₄OH (25%) tropfenweise zugegeben und 4 mal mit 20 ml EtOAc extrahiert. Die organischen Phasen wurden über Na₂SO₄ getrocknet und eingedampft:
Ausbeute: 0,76 g gelbliches Öl (92,9% d.Th.).
Säulenchromatographie (40 g Kieselgel 60, CHCl₃/ 2-7% MeOH) ergab: 0,62 g farblosen Schaum. Molgewicht: (C₁₉H₂₄NO₄): 330,40
¹H-NMR (CDCL₃): 1,52 (dd, H), 1,85 (td, H), 2,10 (dt, H), 2,35 (s, 3H), 2,82 (d, H), 3,02 (d, H), 3,20 (b, H, D₂O-tauschbar), 3,24 (d, H), 3,53-3,72 (m, 5H), 3,78 (s, 3H), 3,94 (t, H), 4,10 (d, H), 4,54 (b, H), 5,94 (d, H), 6,22 (d, H), 6,33 (d, H), 6,61 (d, H).
¹³C-NMR (CDCl₃): 26,50, 34,35, 41,57, 48,01, 53,57, 55,72, 60,17, 61,78, 68,42, 69,48, 86,85, 111,06, 121,22, 124,60, 128,95, 129,21, 131,86, 143,88, 146,15.

### Beispiel 23:

### 4a,5,9,10,11,12-Hexahydro-11-brom-3-methoxy-11-demethyl-6H-benzofuro[3a,3,2-ef][2]benzazepin-6-ethylenketal (allgemeine Formel (I) mit R₂=Me, R₄=H, X₁=Br, X₂=H, Y₁, Y₂= -O-CH₂-CH₂-O-)

0,11 g Verbindung (I) mit R₂=Me, R₄=CHO, X₁=Br, X₂=H, Y₁, Y₂=O-(CH₂)₂-O- wurden in 10 ml THF gelöst, auf 0°C abgekühlt, 0,3 ml LiAlH₄ /THF (1M) über 5 min zugetropft und 30 min bei O°C gerührt. Überschüssiges THF wurde abgedampft, mit 10 ml NH₄OH (25%) aufgenommen und 3 x mit 10 ml EtOAc extrahiert. Die organischen Phasen wurden über Na₂SO₄ getrocknet und eingedampft.
Ausbeute: 0,13 g öliges Rohprodukt.
Säulenchromatographie (5 g Kieselgel 60, CHCl₃/ 2-7% MeOH) ergab: 80 mg farblosen Schaum (77,9% d.Th.)
Molgewicht: (C₁₈H₂₀BrNO₄): 394,27
¹H-NMR(CDCl₃): 6,82 (s, H), 6,16 (d, H), 5,67 (d, H), 4,55 (b, H), 4,48 (d, H), 3,84-4,08 (m, 5H), 3,78 (s, 3H), 3,04-3,37 (m, 2H), 2,62 (bd, H), 2,15 (dd, H), 1,70-1,95 (m, 3H)
¹³C-NMR(CDCl₃): 146,69, 144,00, 133,07, 131,29, 129,00, l12,16, 126,30, 115,25, l02,37, 87,28, 65,11, 64,17, 55,78, 52,46, 49,02, 40,13, 33,06.

### Beispiel 24:

### 4a,5,9,10,1 1,12-Hexahydro-1-brom-3-methoxy-11-benzyl-6H-benzofuro[3a,3,2-ef][2]-benzazepin-6-ethylenketal (allgemeine Formel (I) mit R₂=Me, R₄=-CH₂-Ph, X₁=Br, X₂=H, Y₁, Y₂=-O-CH₂-CH₂-O-)

250 mg (0,63 mMol) Verbindung (I) mit R₂=Me, R₄=H, X₁=Br, X₂=H, Y₁, Y₂=O-(CH₂)₂-O- (N-Demethylbromnarwedinethylenketal) und 63 mg (0,63 mMol) Triethylamin wurden in 15 ml absolutem Tetrahydrofuran vorgelegt, 108 m (0,63 mMol) Benzylbromid bei Raumtemperatur zugegeben und anschließend 24 Stunden gerührt. Das Reaktionsgemisch wurde mit 50 ml Wasser versetzt und die wässerige Phase dreimal mit je 20 ml Essigsäureethylester extrahiert. Die vereinigten organischen Phasen wurden einmal mit gesättigter wässeriger Natriumchloridlösung gewaschen, getrocknet (über Na₂SO₄) und eingedampft.
Ausbeute: 260 mg (84,7% d.Th.) farblose Kristalle
Schmelzpunkt: 118-119°C
DC: EtOAc: MeOH = 9:1
Molgewicht: (C₂₅H₂₆BrNO₄), 484,39
¹H-NMR (CDCl₃; d(ppm)): 1,65 (ddd, 1H), 2,05-2,30 (m, 2H), 2,65 (dd, 1H), 3,00-3,30 (m, 2H), 3,70 (s, 2H), 3,80 (s, 3H), 3,90-4,20 (m, 5H), 4,35 (dd, 1H), 4,60 (ddd, 1H), 5,70 (d, 1H), 6,25 (d, 1H), 6,85 (s, 1H), 7,25-7.30 (m, 5H).
¹³C-NMR (CDCl₃, d(ppm)): 33,1 (d), 33,4 (t), 48,5 (s), 50,7 (t), 55,8 (q), 56,4 (t), 56,9 (t), 64,2, 65,1 (2*t), 87,4 (d), 102,3 (s), 113,6 (s), 115,6 (d), 126,6, 128,2, 128,9 (3*d), 127,1 (d), 3,1 (s), 137,9 (s), 144,2 (s), 146,3 (s).

### Beispiel 25:

### 4a,5,9,10,11,12-Hexahydro-1-brom-3-methoxy-11-demethyl-6H-benzofuro[3a,3,2-ef][2]benzazepin-6-on (allgemeine Formel (I) mit R₂=Me, R₄=H, X₁=Br, X₂=H, Y₁, Y₂=O)=N-Demethylbromnarwedin

250 mg (0,63 mMol) Verbindung (I) mit R₂Me, R₄=H, X₁=Br, X₂=H, Y₁, Y₂=O-(CH₂)₂-O(=N-Demethylbromnarwedinethylenketal) wurden in 20 ml 2 N Salzsäure gelöst und für 15 min auf 100°C erhitzt. Anschließend wurden 20 ml konz. wässeriger Ammoniak zugegeben, kurz erhitzt, das Reaktionsgemisch abgekühlt und ein Niederschlag erhalten, der abgesaugt und bei 50°C(20 mm Hg) getrocknet wurde.
Ausbeute: 130 mg (58,6% d.Th.) farblose Kristalle
Schmelzpunkt: 173-174°C
DC: EtOAc: MeOH = 8:2
Molgewicht: (C₁₆H₁₆Br NO₃), 350,21
¹H-NMR (DMSO-d6, d(ppm)): 1m90-2,15 (m, 2H), 2,75 (dd, 1H), 2,95 (dd, 1H), 3,10-3,35 (m, 2H), 3,75 (s, 3H), 3,90 (d, 1H), 4,40 (d, 1H), 4,55 (dd, 1H), 5,90 (d, 1H), 6,90 (s, 1H), 7,05 (d, 1H ¹³C-NMR(DMSO-d6, d(ppm)): 36,3 (d), 37,0 (t), 45,6 (s), 49,5 (t), 51,3 (1), 55,9 (q), 87,6 (d), 112,5 (s), 116,0 (d), 126,6 (d), 129,6 (s), 132,0 (s), 143,7 (s), 144,8 (d), 146,6 (s), 194,0 (s)

### Beispiel 26:

### 4a,5,9,10,11,12-Hexahydro-3-methoxy-11-Demethyl-6H-benzofuro[3a,3,2-ef][2]benzazepin-6-on (allgemeine Formel (I) mit R₂=Me, R₄=H, X₁=X₂=H, Y₁, Y₂=O)

### Beispiel 27:

### 4a,5,9,10,11,12-Hexahydro-3-methoxy-11-benzyl-6H-benzofuro[3a,3,2-ef][2]benzazepin-6-on (allgemeine Formel (I) mit R₂=Me, R₄=CH₂-Ph, X₁,X₂=H, Y₁, Y₂=O)

### Beispiel 28:

### 4a,5,9,10,11,12-Hexahydro-3-methoxy-11-methyl-6H-benzofuro[3a,3,2-ef][2]benzazepin-6-propylenketal (allgemeine Formel (I) mit R₂=Me, R₄=CH₂, X₁=X₂=H, Y₁, Y₂=O-CH₂-CH(CH₃)-O-)

37,5 g LiAlH₄ wurden unter Argon in einen vorgetrockneten 4-1-Mehrhalskolben gefüllt und 800 ml THF aus einem Tropftrichter zulaufen gelassen, wobei unter heftigem Schäumen die Temperatur auf ca. 45°C anstieg (hängt vom Wassergehalt des THF und des Reaktionskolbens ab). Nun wurde eine Suspension aus 114 g Verbindung (I) mit R₂=Me, R₄=CHO, X₁= Brom, X₂=H, Y₁, Y₂=O-CH₂-CH(CH₃)-O- (roh) in 400 ml THF über 15 min zugetropft, wobei die Temperatur auf Rückflußtemperatur (ca. 65-68°C) anstieg. Nun wurde unter mechanischem Rühren 10 Stunden auf Rückflußtemperatur erwärmt, abgekühlt und tropfenweise unter Kühlen 100 ml Wasser in 100 ml THF zugegeben.

### Entnahme von 10 ml, Alkalischmachen mit NH₄OH und Extraktion mit EtOAc (3 x 20 ml) ergab nach Eindampfen öliges Produkt

### Säulenchromatographie (5 g Kieselgel 60, CHCl₃/3-5% MeOH) von 0,17 g ergab: 0,1 g farblosen Schaum

Molgewicht: (C₂₀H₂₅NO₄): 343,42
¹H-NMR (CDCl₃): 6,60 (dd, 2H), 6,16 (dt, H), 5,68 (dd, H), 4,55 (m, H), 4,38-4,00 (m, 3H), 3,80 (s, 3H), 3,68-2,95 (m, 4H), 2,78-2,60 (m, H), 2,35 (s, 3H), 2,24-2,02 (m, 2H), 1,62 (bd, H), 1,28 (t, 3H)
¹³C-NMR (CDCl₃): 146,59, 143,92, 132,04, 131,90, 129,57, 129,16, 128,86, 128,76, 128,39, 127,44, 126,92, 126,12, 126,02, 121,16, 111,05, 110,90, 110,77, 102,87, 102,73, 87,23, 73,15, 72,24, 71,43, 71,12, 70,44, 70,17, 60,28, 55,59, 55,53, 55,45, 53,83, 47,87, 47,80, 47,75, 41,80, 41,70, 34,84, 33,95, 33,66, 33,37, 18,66, 17,62, 17,43

Anmerkung NMR, Diastereomere: Aufgrund des zusätzlich eingeführten chiralen Zentrums mittels der (+/-)Propylengruppe kommt es zur Bildung von Diastereomeren, die eine zusätzliche Signalaufspaltung zu jener, von der Formylgruppe verursachten, bewirken.

### Beispiel 29:

### 4a,5,9,10,11,12-Hexahydro-3-methoxy-11-methyl-6H-benzofuro[3a,3,2-ef][2]benzazepin-6-on, Narwedin. (Allgemeine Formel (I) mit R₂=Me, R₄=CH₂, X₁=X₂=H, Y₁, Y₂=O)

37,5 g LiAlH₄ wurden unter Argon in einen vorgetrockneten 4-I Mehrhalskolben gefüllt und 800 ml THF aus einem Tropftrichter zulaufen gelassen, wobei unter heftigem Schäumen die Temperatur auf ca. 45°C anstieg (hängt vom Wassergehalt des THF und des Reaktionskolbens ab). Nun wurde eine Suspension aus 114 g Verbindung (I) mit R₂=Me, R₄=CHO, X₁ = Brom, X₂=H, Y₁, Y₂=O-CH₂-CH(CH₃)-O- (roh) in 400 ml THF über 15 min zugetropft, wobei die Temperatur auf Rückflußtemperatur (ca. 65-68°C) anstieg. Nun wurde unter mechanischem Rühren 10 Stunden auf Rückflußtemperatur erwärmt, abgekühlt und tropfenweise unter Kühlen 100 ml Wasser in 100 ml THF zugegeben.

Nun wurde mit 1,25 lit 2n HCI und 60 ml konz. HCI auf pH 0 bis 1 gebracht und 30 min bei 60°C gerührt, anschließend wurde in einen 5 lit. Scheidetrichter überführt, mit 1 lit. EtOAc überschichtet und mit NH₄OH (ca 250 ml) auf pH 10 gebracht und extrahiert. Die wässerige Phase wurde ein weiteres Mal mit 11 EtOAc + 300 ml THF extrahiert. anschließend der Niederschlag über Celite abfiltriert und weitere 2 mal mit 500 ml EtOAc extrahiert. Die vereinten organischen Phasen wurden über Na₂SO₄ getrocknet und eingedampft.
Ausbeute: 64,8 g (86,9% d.Th. roh) gelbe Kristalle
Molgewicht 285,32 (C₁₇H₁₉NO₃)
DC: CHCl₃/MeOH (5%)
Schmelzpunkt: 189-192°C

### Beispiel 30:

### (-)Narwedin:

122,4 g (+/-)Narwedin wurden in 1.9 l EtOH (96%)-Triethylamin (9:1) auf Rückflußtemperatur erwärmt, bis eine homogene Lösung entstand. Nun wurde langsam abgekühlt und bei 68°C 4,0 g (-)Narwedin zugegeben und 7 Tage bei 40°C gerührt. Abkühlen auf Raumtemperatur, Absaugen und Trocknen des kristallinen Niederschlages ergaben (-)Narwedin (Fraktion I). Die Mutterlauge wurde zur Trockene eingedampft, mit 200 ml Ethanol (96%)/Triethylamin (9:1) auf Rückflußtemperatur erwärmt und in der oben angeführten Weise mit 0,4 g (-)Narwedin versetzt und 7 Tage bei 40°C gerührt. Abkühlen, Absaugen und Trocknen ergaben (-)Narwedin
(Fraktion II).
Ausbeute:
FrI: 98,6 g farblose Kristalle (80,5% d.Th.)
Fr II: 7,4 g (6,0% d.Th.)
Drehwert:Fr I: [α]¹⁸ = -407° (c=1,5/CHCl₃)
FrII:[α]¹⁸ = -401° (c=1,5/CHCl₃)
Molgewicht: C₁₇H₁₉NO₃ (285,32)
Schmelzpunkt: 189-192°C

### Beispiel 31:

### (-)Galanthamin

98,6 g (-)Narwedin wurden bei Raumtemperatur portionsweise zu 1 I L-Selektride in THF (1-molar) zugegeben und 1 Stunde gerührt. Nun wurden 100 ml MeOH langsam zugetropft, die trübe Lösung zur Trockene eingedampft und mit 3 l Ethanol (96%) aufgenommen. Mit einer Lösung von wässriger 60% HBr in EtOH (1:1) wurde tropfenweise auf pH 1 angesäuert und über Nacht bei 0°C stehengelassen. Die ausgefallenen Kristalle wurden abgesaugt und getrocknet.
Ausbeute: 120,1 g (94,5% d.Th.)
Drehwert: [α]¹⁸ = -88° (c = 1,5/H₂O)
Molgewicht C₁₇H₂₁NO₃ HBr (368,25)
Schmelzpunkt: 244-247°C (Zersetzung)

### Beispiel 32:

### 3-Benzoyloxy-N-4-(benzyloxyphenethyl)-6-brom-4-methoxy-N-methylbenzamid (allgemeine Formel (Va) mit R₁, R₃ = Benzyl, R₂ = R₄ = CH₃, X₁ = Br, X₂ = H, Z₁ = O, Z₂ = H₂).

20,0 g 3-Benyloxy-6-brom-4-methoxybenzoesäure wurden in 250 ml p.a Chloroform gelöst, anschließend wurden 21,6 ml Thionylchlorid (35,29 mg = 0,297 mol = 5 Äqu.) zugegeben und 3 Stunden unter Rückfluß gekocht, anschließend überschüssiges CHCl₃ + SOCl₂ abdestilliert. Das erhaltene Säurechlorid wurde in 150 ml CHCl₃ aufgenommen.

14,24 g O-Benzyl-N-methyltyramin wurden in 60 ml p.a. CHCl₃ gelöst, dann wurden 100 ml 2N NaOH zugegeben. Dem 2-phasigen Gemisch wurde unter kräftigem Rühren bei Raumtemperatur das gelöste Säurechlorid zugegeben und über Nacht gerührt. Anschließend wurden die Phasen getrennt. Die organische Phase wurde mit H₂O gewaschen, mit Na₂SO₄ getrocknet und eingedampft. Das erhaltene Öl wurde aus 250 ml Ethanol umkristallisiert.
Ausbeute: 27,76 g 83% d.Th. farblose Kristalle.
DC: Petrolether/EtOAc (25:75)
¹H-NMR(CDCl₃): Aufgrund des Amides treten zwei Konformere (Rotamere) auf.
2,69 + 3,12 (2s, je 1,5H); 2,95 + 3,21 (2t, je 1H); 3,75 (t, 1H); 3,9 (s, 3H) 4,96 - 5,14 (m, 4H), 7,1 - 7,48 (m, 16H).
¹³C-NMR(CDCl₃):
32, 26, 36,55, 32,48, 33,39, 48,75, 52,34, 56,14, 70,92, 71,10, 112,82, 113,05, 114,77, 115,69, 127,23, 128,47, 129,73, 129,78

### Beispiel 33:

### 6-Brom-3-hydroxy-N-(4-hydroxyphenethyl)-4-methoxy-N-methylbenzamid (allgemeine Formel (Va) mit R₁ = R₃ = H, R₂ = R₄ = CH₃, X₁ = Br, X₂ = H, Z₁ = O, Z₂ = H₂)

5,0 g 3-Benzyloxy-N-4-(benzyloxyphenethyl)-6-brom-4-methoxy-N-methylbenzamid (allgemeine Formel (Va) mit R₁, R₃ = Benzyl, R₂ = R₄ = CH₃, X₁ = Br, X₂ = H, Z₁ = O, Z₂ = H₂) wurden mit 50 ml Ethanol und 21,6 ml HBr auf 60°C erwärmt und 9 Stunden gerührt. Lösung wurde langsam auf 1 l Eiswasser gegossen und um auszukristallisieren, zwei Stunden gerührt. Der Niederschlag wurde abgesaugt, mit Wasser gewaschen und getrocknet.
Ausbeute: 3,23 g (95,22% d.Th.) farblose Kristalle.
DC: CHCl₃:MeOH = 9:1
Schmelzpunkt: 162 - 166,5°C
¹H-NMR (CDCl₃/DMSO): Aufgrund des Amides treten zwei Isomere auf.
2,49 + 2,81 (2s, je 1,5H); 3,08 + 3,42 (2t, je 1H), 3,65 (s, 3H); 6,43 - 6,6 (m, 4H); 6,72 (s, 1H); 6,88 (s, 1H), 8,31 - 8,59 (b, 2H)
¹³C-NMR (CDCl₃/DMSO):
32,08+33,52, 32,36+36,54, 48,91+52,49, 55,92, 113,97, 114,39, 115,43+115,28, 129,44+129,30, 168,61+168,97.

### Beispiel 34:

### 4a,5,9,10,11,12-Hexahydro-1-brom-3-methoxy-11-methyl-12-oxo-6H-benzofuro[3a,3,2-ef][2]benzazepin-6-on (allgemeine Formel (Ia) mit R₂ = R₄ = CH₃, X₁ = Br, X₂ = H, Y₁, Y₂ = O, Z₁ = O, Z₂ = H₂)

40,5 g Kaliumhexacyanoferrat (III) (123 mol) und 18 g K₂CO₂ (0,13 mol) wurden in 2,7 l Toluol und 180 ml Wasser gelöst und auf 60°C erwärmt. Dann wurden 9,0 g 6-Brom-3-hydroxy-N-(4-hydroxyphenethyl)-4-methoxy-N-methylbenzamid (allgemeine Formel (Va) mit R₁, R₃ = H, R₂ = R₄ = CH₃, X₁ = Br, X₂ = H, Z₁ = O, Z₂ = H₂) (0,024 mol) dazugegeben. Das Reaktionsgemisch wurde 35 min stark mechanisch gerührt. Das entstandene Polymer über Celite abfiltriert. Die wässerige Phase wurden abgetrennt, die Toluolphase mit gesättigtem NaCl gewaschen, über Na₂SO₄ getrocknet und eingedampft.
Rohausbeute: 5,39 g (60,22% d.Th.) gebliches Öl.

1,8 g wurden über 100 g Kieselgel chromatographiert (Laufmittel: CHCl₃ : MeOH = 98:2).
Ausbeute: 1,13 g (37,8% d.Th.) farblose Kristalle.
DC: CHCl₃ : MeOH = 95:5
Schmelzpunkt: 218-222°C
¹H-NMR (CDCl₃):
1,92+2,48 (dd, 2H); 2,75+3,1 (dd, 2H); 3,34+3,82 (dd, 2H); 3,91 (s, 3H); 4,83 (t, 1H); 5,9-6,0+6,3-6,39 (dd, 2H), 7,11 (s, 1H)
¹³C-NMR (CDCl₃/DMSO):
34,00, 36,44+36,58, 48,44, 48,55, 56,31, 89,15, 113,88, 118,55, 122,84, 125,84, 129,35, 145,60, 146,02, 146,61, 164,57, 192,93

### Beispiel 35:

### 4a,5,9,10,11,12-Hexahydro-1-brom-3-methoxy-11-methyl-12-oxo-6H-benzofuro[3a,3,2-ef][2]benzazepin-6,6-propylenglycolketal (allgemeine Formel (la) mit R₂ = R₄ = CH₃, X₁ = Br, X₂ = H, Y₁, Y₂ = O-CH₂CH(CH₃)O-, Z₁ = O, Z₂ = H₂)

1g Edukt (12) (0,0026 mol), 50 ml Toluol, 2 ml Propylenglycol und 0,1 g p-Toluolsulfonsäure wurden unter Rückfluß am Wasserabscheider 4 Stunden gekocht. Nach dem Abkühlen wurde Lösung mit NaHCO₃ und H₂O extrahiert, mit Na₂SO₄ getrocknet und einrotiert.
Ausbeute: 0,92 g 79,75% d.Th.

0,9 g Produkt wurden über 50 g Kieselgel chromatographiert. Laufmittel CH₂Cl₂:MeOH = 99:1
Fraktion 1: 0,34 g farbloser Schaum (30,1% d.Th.)
Fraktion 2: 0,19 g farbloser Schaum
Fraktion 3: 0,17 g farbloser Schaum
DC: CHCl₃:MeOH =95:5

### Fraktion 1:

### ¹H-NMR(CDCl₃): 6,95 (s, 1H), 5,38-5,60 (m, 2H), 4,64 (m, 1H), 4,15 (m, 1H), 3,80 (s, 3H), 3,35-4,10 (m, 2H), 3,10 (s, 3H), 3,00 (dd, H), 2,85 (dd, H), 2,15-2,35 (m, 2H), 1,70-1,95 (m, 2H), 1,12-1,25 (m, 3H).

### Fraktion 2:

### ¹H-NMR(CDCl₃): 0,96-1,1 (m, 3H), 1,18-1,32 (m, 3H), 1,40-1,71 (m, 2H), 1,85 (b, H), 1,90-2,20 (m, 2H), 2,35-2,66 (m, 2H), 2,70-2,82 (m, H), 3,10 (s, 3H), 3,20 (b, H), 3,42-3,81 (m, 6H), 3,85 (s, 3H), 4,02 (m, H), 4,20 (m, H), 4,50 (bd, H), 7,05 (s, H).

### Fraktion 3:

### ¹H-NMR(CDCl₃): 0,95-1,1 (m, 3H), 1,20-1,35 (m, 3H), 1,51-1,72 (m, H), 1,82 (b, H), 1,80-2,12 (m, 3H), 2,30-2,68 (m, 2H), 3,12 (s, 3H), 3,20-3,75 (m, 7H), 3,83 (s, 3H), 3,96-4,15 (m, H), 4,22 (m, H), 4,52 (bd, H), 7,07 (s, H)

### Beispiel 36:

### Narwedin (allgemeine Formel (la) mit R₂ = R₄ = CH₃, X₁ = X₂ = H, Y₁, Y₂ = O, Z₁ = Z₂ = H₂)

0,35g4a,5,9,10,11,12-Hexahydro-1-brom-3-methoxy-1-methyl-12-oxo-6H-benzofuro[3a,3,2-ef][2]benzazepin-6,6-propylenglycolketal (allgemeine Formel (la) mit R₂ = R₄ = CH₃, X₁ = Br, X₂ = H, Y₁, Y₂ = O-CH₂CH(CH₃)O-, Z₁ = O, Z₂ = H₂) wurden zu einer Lösung von 0,2 g LiAlH₄ in 20 ml wasserfreiem THF unter Kühlen zugegeben und über Nacht bei Raumtemperatur gerührt. Nun wurde mit 20 ml 2n HCI versetzt, 30 min bei 40°C gerührt, mit konz. NH₄OH alkalisch gemacht und mit Ethylacetat (4 x 30 ml) extrahiert. Trocknen über Na₂SO₄ und Eindampfen der organischen Phase ergaben 0,21 g gelbliches Öl, das über 15 g Kieselgel mit CHCl₃/MeOH (98:2) chromatographiert wurde: 0,14 g (61,2% d.Th.) Narwedin als farblose Kristalle.
DC: CHCl₃/MeOH (95:5)

### Beispiel 37:

Reduktion von Verbindung (12) mit L-Selektride zu einer Verbindung der allgemeinen Formel (la) mit R₂ = R₄ = CH₃, X₁ = Br, X₂ = H, Y₁ = OH, Y₂ = H, Z₁ = O, Z₂ = H₂.

1 g Edukt (12) (0,0026 Mol) wurde in 50 ml wasserfreiem THF gelöst, anschließend wurden 7,93 ml L-Slektride (0,0079 Mol = 3 Äq.) zugegeben und drei Stunden bei Raumtemperatur gerührt. Die Lösung wurde mit 10 ml 2N HCI angesäuert, mit 5 ml NH₄OH neutralisiert und 3 mal mit Ethylacetat extrahiert. mit Na₂SO₄ getrocknet und einrotiert.
Ausbeute: 1,07 g 106,47% d.Th.
Produkt wurde mit 50 g Kieselgel chromatographiert, CHCl₃:MeOH = 98:2
Ausbeute Fr. 34-49: 0,31 g
DC: CHCl₃ : MeOH = 95:5
Schmelzpunkt: 75,2-80°C
¹H-NMR (CDCl₃): 1,65-1,80 (m, H), 1,95-2,17 (m, H), 2,19-2,38 (dt, H), 2,65 (dm, H), 3,13-3,22 (m, H), 3,15 (s, 3H), 3,70-3,88 (m, H), 3,85 (s, 3H), 4,12 (m, H), 4,70 (b, H), 5,50 (d, H), 5,88 (dd, H), 7,08 (s, H).
¹³C-NMR (CDCl₃): 29,64, 33,91, 38,01, 48,13, 48,63, 56,12, 60,59, 89,68, 113,47, 117,78, 123,08, 126,20, 130,64, 131,90, 144,61, 146,02, 164,94.

Erläuterung der in der vorstehenden Beschreibung verwendeten Abkürzungen:
DiBAI-H: Diisobutylaluminiumhydrid
Red-Al^{R}: Natrium-bis-(2-methoxyethoxy)-aluminiumdihydrid
Superhydride^{R}: Lithium-triethylborhydrid
9-BBN: 9-Borabicydo(3.3.1)nonan
L-Selektride^{R}: Lithium-tri-sec.-butylborhydrid (Aldrich)
K-Selektride^{R}: Kalium-tri-sec.-butylborhydrid (Aldrich)
LS-Selektride^{R}: Lithium-trisiamylborhydrid (Aldrich)
KS-Selektride^{R}: Kalium-trisiamylborhydrid (Aldrich)
Aliquat^{R}: 3-Methyl-trioctylammoniumchlorid
LM: Lösungsmittel
ML: Mutterlauge
THF:Tetrahydrofuran
DMF:Dimethylformamid
EtOAc: Ethylacetat
TsOH: p-Toluolsulfonsäure
RT: Raumtemperatur

| | Literatur |
|---|---|
| [1] | D.H.R. Barton, G.W. Kirby, Proc. Chem. Soc. 392, 1960. |
| [2] | D.H.R. Barton, G.W. Kirby, J. Chem. Soc. 806, 1962. |
| [3] | T. Kametani, T. Yamaki, H. Yagi, K. Fukumoto, J. Chem. Soc. 2602, 1969. |
| [4] | T. Kametani, T. Yamaki, H. Yagi, K Fukumoto, J. Chem. Soc. Chem. Comm. 25, 1969. |
| [5] | T. Kametani, C. Seino, K Yamaki, S. Shibuya, K. Fukumoto, K Kigassawa, F. Satoh, M. Hiiragi, T. Hayasaka, J. Chem. Soc. (C), 1043, 1971. |
| [6] | T. Kametani, K Yamaki, T. Terui, J. Het. Chem. 10, 35, 1973. |
| [7] | T. Kametani, K Shishido, E. Hayashi, C. Seino, T. Kohno, S. Shibuya, K Fukumoto, J. Org. Chem. 36, 1295, 1971. |
| [8] | J. Szewczyk, A.H. Lewin, F.1. Carroll, J. Het. Chem. 25, 1809, 1988. |
| [91 | Edinen Zentar po Chimia Sophia, DE 2945 161 800604, CA 94, 15945b. |
| [10] | Edinen Zentar po Chimia Sophia, US 4290862 810922, CA. 95, 212006t. |
| [11] | R. Vlahov, D. Krikorian, V. Tarpanov, G. Spassov, G. Snatzke, H. Duddeck, H.J. Schäfer, K. Kieslich, Izv. Khim. 20, 59, 1987, CA 108, 150799e. |
| [12] | D. Krikorian, R. Vlahov, S. Parushev, M. Chinova, I. Vlahov, H. Schäfer, H. Duddeck, G. Schnatzke, Tetrahedron Lett. 25, 2969, 1984. |
| [13] | R. Vlahov, D. Krikorian, G. Spassov, M. Chinova, I. VIahov, S. Parushev, G. Snatzke, L Emst, K. Kieslich, W. Abraham, W. Sheldrick, Tetrahedron 45, 3329, 1989. |
| [14] | K Shimizu, K. Tomioka, S. Yamada, K. Koga, Heterocycles 8, 277, 1977. |
| [15] | K. Shimizu, K Tomioka, S. Yamada, K Koga, Chem. Pharm. Bull. 26, 3765, 1978. |
| [16] | J.P. Yardley, H. Fletcher, Synth. 244, 1976. |
| [17] | R.L Edwards, D.V. Wilson, J. Chem. Soc. 5003, 1961. |
| [18] | S.D. Saraf, Synt. Commun. 13, 7, 1983. |
| [19] | B. Davis, M. Joullie, WO 8808708 Al. |
| [20] | T. Kametani, M. Premila, K. Fukumotu, Heterocycles 4(6), 1111-14, 1976. |
| [21] | Synform 283-94, 1983. |
| [22] | T. Kametani, K. Yamaki, H. Yagi, K. Futumoto, J. Chem. Soc. C 2601-5, 1969 |
| [23] | R.A. Holton, M.P. Sibi, W.S. Murphy, J.Am.Chem.Soc. 110, 314 (1988) |
| [24] | W.C. Shieh, J.A. Carlson, J.Org.Chem. 59, 5463-5465 (1994) |
| [25] | AB. Smith, S.J. Branca, MA. Guaciaro, P.M. Wovkulich, A. Korn, Organic Synthesis Coll. Voll 7, 271. |
| [26] | A. Hagedorn, D. Farnum, J. Org. Chem. 42, 3765 (1977). |

## Patentansprüche

1. Verfahren zum Herstellen von Derivaten des 4a,5,9,10,-11,12-Hexahydro-6H-benzofuro[3a,3,2-ef][2]benzazepins mit der allgemeinen Formel, ausgewählt aus der Gruppe bestehend aus 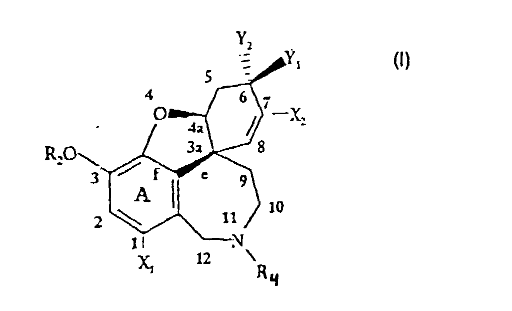 und 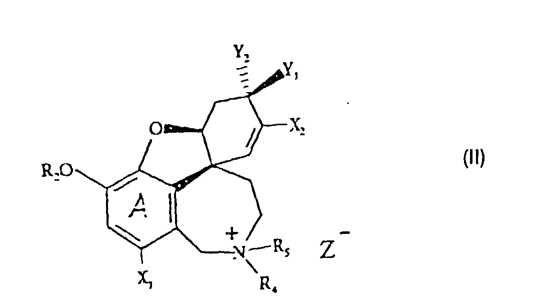 oder Salzen derselben, worin
R₂, R₄, X₁ und X₂ entweder gleich oder verschieden sind und ausgewählt sind aus der Gruppe bestehend aus Wasserstoff, Fluor, Chlor, Brom, Jod, Hydroxy, Alkoxy, niedriges Alkyl, das gegebenenfalls durch wenigstens ein Halogen substituiert ist, niedriges Alkenyl, niedriges Alkinyl, Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl und Arylsulfonyl;
Y₁ und Y₂ entweder gleich oder verschieden sind und ausgewählt sind aus der Gruppe bestehend aus Wasserstoff, Fluor, Chlor, Brom, Jod, Hydroxy, Alkoxy, niedriges Alkyl, das gegebenenfalls durch wenigstens ein Halogen substituiert ist, niedriges Alkenyl, niedriges Alkinyl, Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl oder gemeinsam =O (Keton) sind;
A ein Benzolkern ist, der gegebenenfalls wenigstens einfach durch niedriges Alkyl, niedriges Alken, niedriges Alkin, Alkoxy, Fluor, Chlor, Brom, Jod, Alkyl, das durch wenigstens ein Halogen substituiert ist, Aralkyl, Hydroxy, primäres Amino, sekundäres Amino, tertiäres Amino, Nitro, Nitril, Alkylamino, Arylamino, Aldehyd, Carbonsäure und Carbonsäurederivate substituiert ist;
Z⁻ ein Anion einer pharmazeutisch annehmbaren, organischen Säure oder ein anorganisches Anion ist; und
R₅ ausgewählt ist aus der Gruppe bestehend aus Wasserstoff, Formyl, Alkyl, Alkenyl, Aryl, Aralkyl, Alkylcarbonyl, Arylcarbonyl, Aralkylcarbonyl, Alkyloxycarbonyl, Aryloxycarbonyl, Aralkyloxycarbonyl, Alkylsulfonyl, Arylsulfonyl und Aralkylsulfonyl;
dadurch gekennzeichnet, daß man
(A) eine Verbindung der allgemeinen Formel (III) 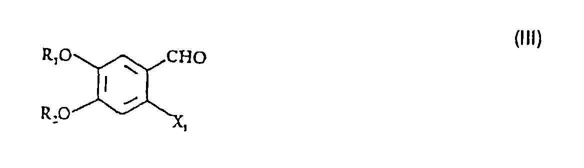 worin R₁ und R₂ ausgewählt sind aus der Gruppe bestehend aus Wasserstoff, Alkyl, das gegebenenfalls durch wenigstens ein Halogen substituiert ist, Alkenyl, Aryl, Arylcarbonyl, Aralkyl, Alkylcarbonyl und Aralkylcarbonyl; und worin X₁ ausgewählt ist aus der Gruppe bestehend aus Wasserstoff, Fluor, Chlor, Brom, Jod und tertiäres Butyl;
(B) mit einer Verbindung der allgemeinen Formel (IV) 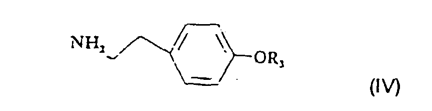 worin R₃ ausgewählt ist aus der Gruppe bestehend aus Wasserstoff, Alkyl, Alkenyl, Aryl, Aralkyl, Alkylcarbonyl, Arylcarbonyl und Aralkoxycarbonyl, wobei die Gruppe gegebenenfalls R₃ durch wenigstens ein Halogen substituiert ist, zu einer Verbindung der allgemeinen Formel (V) 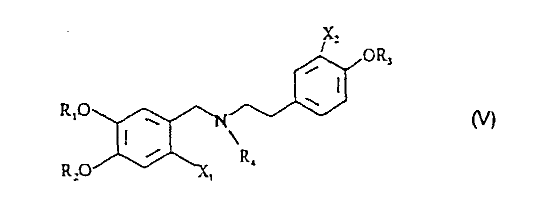 kondensiert, daß man das erhaltene Kondensationsprodukt (Schiff'sche Base) reduziert, wobei man, falls R₄ Wasserstoff ist, in die Verbindung der allgemeinen Formel (V) gegebenenfalls eine N-Schutzgruppe einführt;
(C) daß man die so erhaltene Verbindung der allgemeinen Formel (V) oxidativ cyclisiert, indem man mit einer Base und einem Oxidationsmittel umsetzt,
(D) daß man eine so erhaltene Verbindung der allgemeinen Formel (I), in der Y₁ und Y₂ gemeinsam =O (Keton) bedeuten, mit L-Selektride, K-Selektride, KS-Selektride oder LS-Selektride zur Verbindung der allgemeinen Formel (I), worin Y₁ Hydroxy bedeutet, reduziert und daß man eine racemische Verbindung der allgemeinen Formel (I), in der R₄ ausgewählt ist aus der Gruppe bestehend aus Wasserstoff, Alkyl, Alkenyl, Alkinyl, Aryl und Aralkyl, durch Kristallisation mit einer chiralen Säure in die entsprechenden Enantiomeren trennt.

2. Verfahren zum Herstellen von Derivaten des 4a,5,9,10,-11,12-Hexahydro-6H-benzofuro[3a,3,2-ef][2]benzazepins mit der allgemeinen Formel, ausgewählt aus der Gruppe bestehend aus 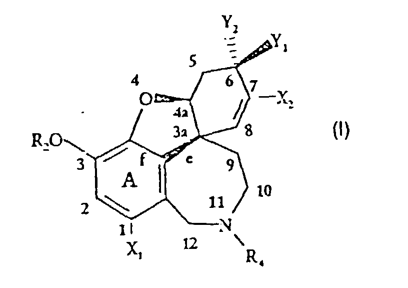 und 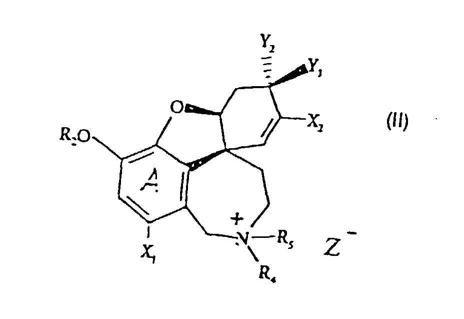 oder Salzen derselben, worin
R₂, R₄, X₁ und X₂ entweder gleich oder verschieden sind und ausgewählt sind aus der Gruppe bestehend aus Wasserstoff, Fluor, Chlor, Brom, Jod, Hydroxy, Alkoxy, niedriges Alkyl, das gegebenenfalls durch wenigstens ein Halogen substituiert ist, niedriges Alkenyl, niedriges Alkinyl, Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl und Arylsulfonyl;
Y₁ und Y₂ entweder gleich oder verschieden sind und ausgewählt sind aus der Gruppe bestehend aus Wasserstoff, Fluor, Chlor, Brom, Jod, Hydroxy, Alkoxy, niedriges Alkyl, das gegebenenfalls durch wenigstens ein Halogen substituiert ist, niedriges Alkenyl, niedriges Alkinyl, Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl oder gemeinsam =O (Keton) sind;
A ein Benzolkern ist, der gegebenenfalls wenigstens einfach durch niedriges Alkyl, niedriges Alken, niedriges Alkin, Alkoxy, Fluor, Chlor, Brom, Jod, Alkyl, das durch wenigstens ein Halogen substituiert ist, Aralkyl, Hydroxy, primäres Amino, sekundäres Amino, tertiäres Amino, Nitro, Nitril, Alkylamino, Arylamino, Aldehyd, Carbonsäure und Carbonsäurederivate substituiert ist;
Z⁻ ein Anion einer pharmazeutisch annehmbaren, organischen Säure oder ein anorganisches Anion ist; und
R₅ ausgewählt ist aus der Gruppe bestehend aus Wasserstoff, Formyl, Alkyl, Alkenyl, Aryl, Aralkyl, Alkylcarbonyl, Arylcarbonyl, Aralkylcarbonyl, Alkyloxycarbonyl, Aryloxycarbonyl, Aralkyloxycarbonyl, Alkylsulfonyl, Arylsulfonyl und Aralkylsulfonyl;
dadurch gekennzeichnet, daß man
(A) eine Verbindung der allgemeinen Formel (III) 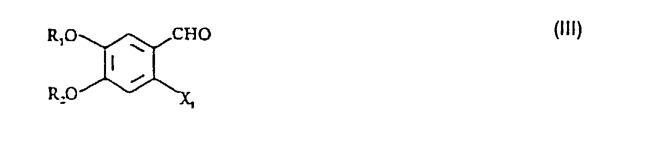 worin R₁ und R₂ ausgewählt sind aus der Gruppe bestehend aus Wasserstoff, Alkyl, das gegebenenfalls durch wenigstens ein Halogen substituiert ist, Alkenyl, Aryl, Arylcarbonyl, Aralkyl, Alkylcarbonyl und Aralkylcarbonyl; und
worin X₁ ausgewählt ist aus der Gruppe bestehend aus Wasserstoff, Fluor, Chlor, Brom, Jod und tertiäres Butyl;
(B) mit einer Verbindung der allgemeinen Formel (IV) 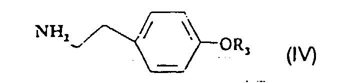 worin R₃ ausgewählt ist aus der Gruppe bestehend aus Wasserstoff, Alkyl, Alkenyl, Aryl, Aralkyl, Alkylcarbonyl, Arylcarbonyl und Aralkoxycarbonyl, wobei die Gruppe gegebenenfalls R₃ durch wenigstens ein Halogen substituiert ist, zur Verbindung der allgemeinen Formel (V) 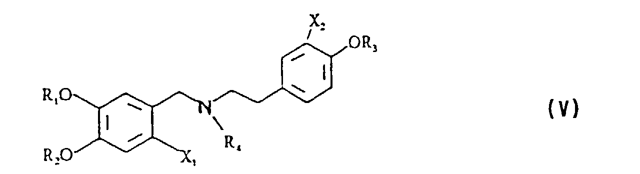 kondensiert, daß man das erhaltene Kondensationsprodukt (Schiff'sche Base) reduziert, wobei man, falls R₄ Wasserstoff ist, in die Verbindung der allgemeinen Formel (V) gegebenenfalls eine N-Schutzgruppe einführt;
(C) daß man die so erhaltene Verbindung der allgemeinen Formel (V) oxidativ cyclisiert, indem man mit einer Base und einem Oxidationsmittel umsetzt,
(D) daß man eine so erhaltene Verbindung der allgemeinen Formel (I), in der Y₁ und Y₂ gemeinsam =O (Keton) bedeuten, mit DiBAI, REDAI oder Superhydride zur Verbindung der allgemeinen Formel (I) reduziert, daß man die entstehenden Diastereomeren der Verbindung der allgemeinen Formel (I), in welchen entweder Y₁ oder Y₂ Hydroxy bedeutet, durch ein chromatografisches Verfahren trennt und daß man eine racemische Verbindung der allgemeinen Formel (I), in der R₄ ausgewählt ist aus der Gruppe bestehend aus Wasserstoff, Alkyl, Alkenyl, Alkinyl, Aryl und Aralkyl, durch Kristallisation mit einer chiralen Säure in die entsprechenden Enantiomeren trennt.

3. Verfahren zum Herstellen von Derivaten des 4a,5,9,10,-11,12-Hexahydro-6H-benzofuro[3a,3,2-ef][2]benzazepins mit der allgemeinen Formel, ausgewählt aus der Gruppe bestehend aus 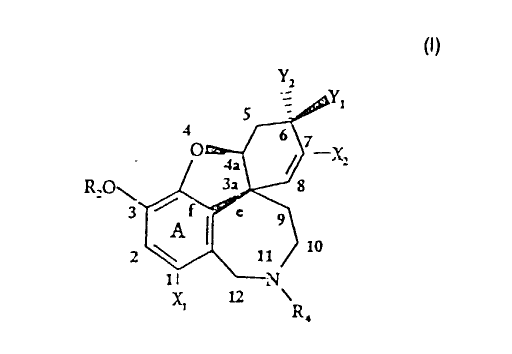 und 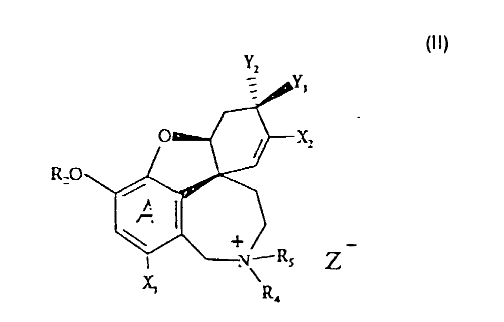 oder Salzen derselben, worin
R₂, R₄, X₁ und X₂ entweder gleich oder verschieden sind und ausgewählt sind aus der Gruppe bestehend aus Wasserstoff, Fluor, Chlor, Brom, Jod, Hydroxy, Alkoxy, niedriges Alkyl, das gegebenenfalls durch wenigstens ein Halogen substituiert ist, niedriges Alkenyl, niedriges Alkinyl, Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl und Arylsulfonyl;
Y₁ und Y₂ entweder gleich oder verschieden sind und ausgewählt sind aus der Gruppe bestehend aus Wasserstoff, Fluor, Chlor, Brom, Jod, Hydroxy, Alkoxy, niedriges Alkyl, das gegebenenfalls durch wenigstens ein Halogen substituiert ist, niedriges Alkenyl, niedriges Alkinyl, Aryl, Aralkyl, Araloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl oder gemeinsam =O (Keton) sind;
A ein Benzolkern ist, der gegebenenfalls wenigstens einfach durch niedriges Alkyl, niedriges Alken, niedriges Alkin, Alkoxy, Fluor, Chlor, Brom, Jod, Alkyl, das durch wenigstens ein Halogen substituiert ist, Aralkyl, Hydroxy, primäres Amino, sekundäres Amino, tertiäres Amino, Nitro, Nitril, Alkylamino, Arylamino, Aldehyd, Carbonsäure und Carbonsäurederivate substituiert ist;
Z⁻ ein Anion einer pharmazeutisch annehmbaren, organischen Säure oder ein anorganisches Anion ist; und
R₅ ausgewählt ist aus der Gruppe bestehend aus Wasserstoff, Formyl, Alkyl, Alkenyl, Aryl, Aralkyl, Alkylcarbonyl, Arylcarbonyl, Aralkylcarbonyl, Alkyloxycarbonyl, Aryloxycarbonyl, Aralkyloxycarbonyl, Alkylsulfonyl, Arylsulfonyl und Aralkylsulfonyl;
dadurch gekennzeichnet, daß man
(A) eine Verbindung der allgemeinen Formel (III) 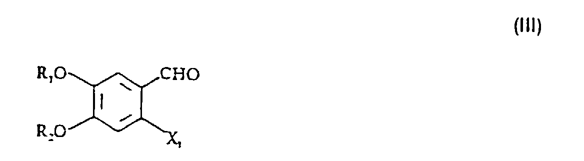 worin R₁ und R₂ ausgewählt sind aus der Gruppe bestehend aus Wasserstoff, Alkyl, das gegebenenfalls durch wenigstens ein Halogen substituiert ist, Alkenyl, Aryl, Arylcarbonyl, Aralkyl, Alkylcarbonyl und Aralkylcarbonyl; und
worin X₁ ausgewählt ist aus der Gruppe bestehend aus Wasserstoff, Fluor, Chlor, Brom, Jod und tertiäres Butyl;
(B) mit einer Verbindung der allgemeinen Formel (IV) 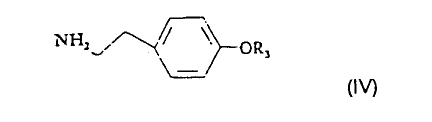 worin R₃ ausgewählt ist aus der Gruppe bestehend aus Wasserstoff, Alkyl, Alkenyl, Aryl, Aralkyl, Alkylcarbonyl, Arylcarbonyl und Aralkoxycarbonyl, wobei die Gruppe gegebenenfalls R₃ durch wenigstens ein Halogen substituiert ist, zur Verbindung der allgemeinen Formel (V) 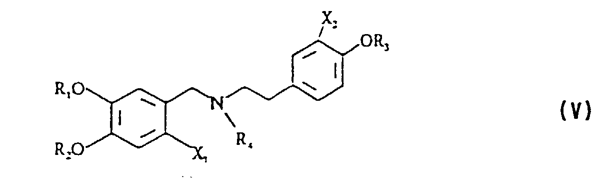 kondensiert, daß man das erhaltene Kondensationsprodukt (Schiff'sche Base) reduziert,
wobei man, falls R₄ Wasserstoff ist, in die Verbindung der allgemeinen Formel (V) gegebenenfalls eine N-Schutzgruppe einführt;
(C) daß man die so erhaltene Verbindung der allgemeinen Formel (V) oxidativ cyclisiert, indem man mit einer Base und einem Oxidationsmittel umsetzt,
(D) daß man eine so erhaltene Verbindung der allgemeinen Formel (I), in der Y₁ und Y₂ gemeinsam =O (Keton) bedeuten, in ein Ketal oder ein Thioketal überführt, daß man das so erhaltene Ketal oder Thioketal reduziert, daß man in der entstandenen Verbindung vom Typ Narwedinketal die Ketal oder Thioketalgruppe spaltet, daß man eine racemische Verbindung der allgemeinen Formel (I), in der R₄ ausgewählt ist aus der Gruppe bestehend aus Wasserstoff, Alkyl, Alkenyl, Alkinyl, Aryl und Aralkyl, durch chiral induzierte Kristallisation in das entsprechende Enantiomere überführt, und daß man das so erhaltene Enantiomere mit L-Selektride, K-Selektride, KS-Selektride oder LS-Selektride zur Verbindung der allgemeinen Formel (I) reduziert.

4. Verfahren zum Herstellen von Derivaten des 4a,5,9,10,-11,12-Hexahydro-6H-benzofuro[3a,3,2-ef][2]benzazepins mit der allgemeinen Formel, ausgewählt aus der Gruppe bestehend aus 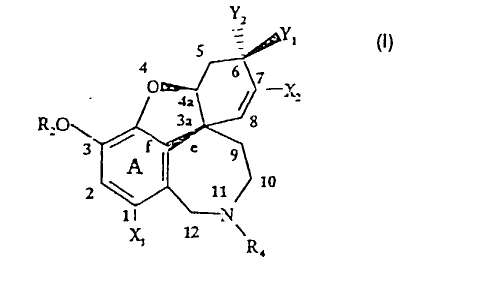 und 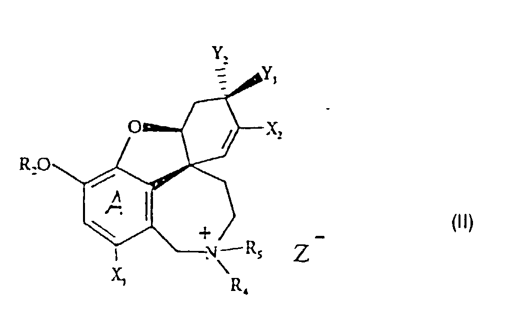 oder Salzen derselben, worin
R₂, R₄, X₁ und X₂ entweder gleich oder verschieden sind und ausgewählt sind aus der Gruppe bestehend aus Wasserstoff, Fluor, Chlor, Brom, Jod, Hydroxy, Alkoxy, niedriges Alkyl, das gegebenenfalls durch wenigstens ein Halogen substituiert ist, niedriges Alkenyl, niedriges Alkinyl, Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl und Arylsulfonyl;
Y₁ und Y₂ entweder gleich oder verschieden sind und ausgewählt sind aus der Gruppe bestehend aus Wasserstoff, Fluor, Chlor, Brom, Jod, Hydroxy, Alkoxy, niedriges Alkyl, das gegebenenfalls durch wenigstens ein Halogen substituiert ist, niedriges Alkenyl, niedriges Alkinyl, Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl oder gemeinsam =O (Keton) sind;
A ein Benzolkern ist, der gegebenenfalls wenigstens einfach durch niedriges Alkyl, niedriges Alken, niedriges Alkin, Alkoxy, Fluor, Chlor, Brom, Jod, Alkyl, das durch wenigstens ein Halogen substituiert ist, Aralkyl, Hydroxy, primäres Amino, sekundäres Amino, tertiäres Amino, Nitro, Nitril, Alkylamino, Arylamino, Aldehyd, Carbonsäure und Carbonsäurederivate substituiert ist;
Z⁻ ein Anion einer pharmazeutisch annehmbaren, organischen Säure oder ein anorganisches Anion ist; und
R₅ ausgewählt ist aus der Gruppe bestehend aus Wasserstoff, Formyl, Alkyl, Alkenyl, Aryl, Aralkyl, Alkylcarbonyl, Arylcarbonyl, Aralkylcarbonyl, Alkyloxycarbonyl, Aryloxycarbonyl, Aralkyloxycarbonyl, Alkylsulfonyl, Arylsulfonyl und Aralkylsulfonyl;
dadurch gekennzeichnet, daß man eine Verbindung der allgemeinen Formel (Va) 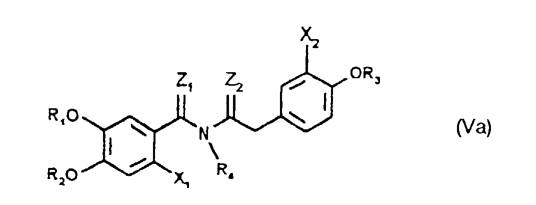 worin R₁, R₂, R₃, R₄, X₁, X₂ die in Anspruch 1 genannten Bedeutungen haben und worin Z₁ und Z₂ =O, S, N bedeuten, wobei man, falls R₄ Wasserstoff ist, in die Verbindung der allgemeinen Formel (Va) gegebenenfalls eine N-Schutzgruppe einführt, zu einer Verbindung der allgemeinen Formel (la) 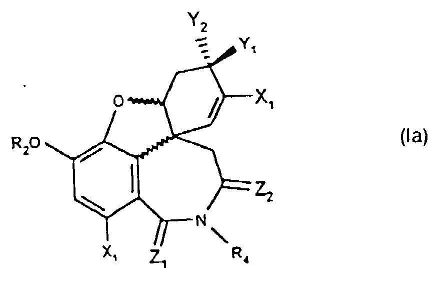 oxidativ cyclisiert, indem man mit einer Base und einem Oxidationsmittel umsetzt,
(D) daß man eine so erhaltene Verbindung der allgemeinen Formel (I), in der Y₁ und Y₂ gemeinsam =O (Keton) bedeuten, in ein Ketal oder ein Thioketal überführt, daß man das so erhaltene Ketal oder Thioketal reduziert, daß man in der entstandenen Verbindung vom Typ Narwedinketal die Ketal oder Thioketalgruppe spaltet, daß man eine racemische Verbindung der allgemeinen Formel (I), in der R₄ ausgewählt ist aus der Gruppe bestehend aus Wasserstoff, Alkyl, Alkenyl, Alkinyl, Aryl und Aralkyl, durch chiral induzierte Kristallisation in das entsprechende Enantiomere überführt, und daß man das so erhaltene Enantiomere mit L-Selektride, K-Selektride, KS-Selektride oder LS-Selektride zur Verbindung der allgemeinen Formel (I) reduziert.

5. Verfahren nach einem der Ansprüche 1 bis 4, dadurch gekennzeichnet, daß man die Kondensationsreaktion (Stufe B) in einem Lösungsmittel bei Rückflußtemperatur ausführt und allenfalls entstehendes Wasser abtrennt.

6. Verfahren nach einem der Ansprüche 1 bis 5, dadurch gekennzeichnet, daß man die Reduktion des in Stufe B erhaltenen Kondensationsproduktes (Schiff'sche Base) mit einem Reduktionsmittel ausführt, das ausgewählt ist aus der Gruppe bestehend aus Natriumborhydrid, Kaliumborhydrid, Natriumcyanoborhydrid, LiAlH₄ und Mischungen derselben.

7. Verfahren nach einem der Ansprüche 1 bis 6, dadurch gekennzeichnet, daß man in Stufe C die Verbindung der allgemeinen Formel (V) oder (Va) mit einer Base umsetzt, die ausgewählt ist aus der Gruppe bestehend aus Natriumhydrogencarbonat, Kaliumcarbonat, NaOH, KOH und Pyridin.

8. Verfahren nach einem der Ansprüche 1 bis 7, dadurch gekennzeichnet, daß man in Stufe C die Verbindung der allgemeinen Formel (V) oder (Va) mit einem Oxidationsmittel umsetzt, das ausgewählt ist aus der Gruppe bestehend aus Pb(OAc)₄, KMnO₄, Eisenchlorid, Kaliumferricyanid und H₂O₂.

9. Verfahren nach einem der Ansprüche 1 bis 8, dadurch gekennzeichnet, daß man die Umsetzung in Stufe C in einem Lösungsmittel ausgewählt aus der Gruppe bestehend aus Toluol und Xylol ausführt.

10. Verfahren nach einem der Ansprüche 3 bis 9, dadurch gekennzeichnet, daß in Stufe D die Verbindung der allgemeinen Formel (I) oder (Ia), in der Y₁ und Y₂ gemeinsame =0 (Keton) bedeuten, in ein Ketal oder Thioketal überführt, indem man die Verbindung der allgemeinen Formel (I) oder (Ia) mit einer Verbindung ausgewählt aus der Gruppe bestehend aus einem Alkohol R₆-OH und einem Thiol R₆-SH umsetzt, worin R₆ ausgewählt ist aus der Gruppe bestehend aus Alkyl, Alkenyl, Aryl, Aralkyl, Alkylcarbonyl, Arylcarbonyl und Aralkylcarbonyl, wobei R₆ gegebenenfalls durch wenigstens ein Halogen substituiert ist.

11. Verfahren nach einem der Ansprüche 3 bis 9, dadurch gekennzeichnet, daß in Stufe D die Verbindung der allgemeinen Formel (I) oder (Ia), in der Y₁ und Y₂ gemeinsame =0 (Keton) bedeuten, in ein Ketal oder Thioketal überführt, indem man die Verbindung der allgemeinen Formel (I) mit einem Diol R₆(OH)₂ oder einem Dithiol R₆(SH)₂, wobei R₆ die in Anspruch 10 genannten Bedeutungen hat, umsetzt.

12. Verfahren nach Anspruch 11, dadurch gekennzeichnet, daß man in Stufe D zur Bildung des Ketals mit Propylenglykol umsetzt.

13. Verfahren nach einem der Ansprüche 3 bis 12, dadurch gekennzeichnet, daß man das Ketal oder Thioketal mit einem Reduktionsmittel, ausgewählt aus der Gruppe bestehend aus Natriumborhydrid, Kaliumborhydrid, Natriumcyanoborhydrid, LiAlH₄, L-Selektride, DiBAI. REDAI, K-Selektride, KS-Selektride, LS-Selektride, Superhydride, 9-BBN, Zn/CacL₂ und Mischungen derselben reduziert.

14. Verfahren nach Anspruch 13, dadurch gekennzeichnet, daß das Reduktionsmittel LiAlH₄ ist.

15. Verfahren nach einerm der Ansprüche 1 bis 14, dadurch gekennzeichnet, daß die erhaltene Verbindung das quaternäre Ammoniumsalz der allgemeinen Formel (II) ist.

16. Verfahren nach Anspruch 15, dadurch gekennzeichnet, daß Z⁻ ausgewählt ist aus der Gruppe bestehend aus Tartrat, Lactat, Citrat, Acetat, Maleinat, Fluorid, Chlorid, Bromid, Jodid, Sulfat, Phosphat und Chlorat.

17. Verfahren nach Anspruch 16, dadurch gekennzeichnet, daß R₁, R₂ und R₃ ausgewählt sind aus der Gruppe bestehend aus Wasserstoff, Alkyl, Alkenyl, Aryl, Aralkyl, Alkylcarbonyl, Arylcarbonyl und Aralkylcarbonyl, wobei R₁, R₂ und R₃, gegebenenfalls durch wenigstens ein Halogen substituiert sind.

18. Verfahren nach einem der Ansprüche 1 bis 16, dadurch gekennzeichnet, daß R₄ und R₅ ausgewählt sind aus der Gruppe bestehend aus Wasserstoff, Formyl, Alkyl, Alkenyl, Aryl, Aralkyl, Alkylcarbonyl, Arylcarbonyl, Aralkylcarbonyl, Alkylsulfonyl, Arylsulfonyl und Aralkylsulfonyl, wobei R₄ und R₅ gegebenenfalls durch wenigstens ein Halogen substituiert sind.

19. Verfahren nach einem der Ansprüche 1 bis 18, dadurch gekennzeichnet, daß X₁ und X₂ ausgewählt sind aus der Gruppe bestehend aus Wasserstoff, Fluor, Chlor, Brom, Jod und t-Butyl.

20. Verfahren nach einem der Ansprüche 1 bis 19, dadurch gekennzeichnet, daß man den Stickstoff in der Verbindung der allgemeinen Formel (V) oder (Va) vor der Oxidation durch Einführen einer Verbindung ausgewählt aus der Gruppe bestehend aus Formyl, Aralkyl, Alkylcarbonyl, Arylcarbonyl, Aralkylcarbonyl, Aralkyloxycarbonyl, Alkylsulfonyl, Aralkylsulfonal und Arylsulfonyl schützt.

21. Verfahren nach Anspruch 20, dadurch gekennzeichnet, daß man eine Formylgruppe einführt, indem man die Verbindung der allgemeinen Formel (V) oder (Va) mit der 1- bis 50-fachen molaren Menge Ethylformat in Gegenwart katalytischer Mengen an Ameisensäure umsetzt.

22. Verfahren nach einem der Ansprüche 1 bis 21, dadurch gekennzeichnet, daß man eine Verbindung der allgemeinen Formel (V), worin
R₁, R₂ und R₃ ausgewählt sind aus der Gruppe bestehend aus Wasserstoff, Alkyl, Aryl, Aralkyl, Alkylcarbonyl, Arylcarbonyl und Aralkylcarbonyl;
X₁ Brom ist;
X₂ Wasserstoff ist; und
R₄ ausgewählt ist aus der Gruppe bestehend aus Wasserstoff, Formyl, Aralkyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl und Arylsulfonyl,
herstellt, indem man eine Verbindung der allgemeinen Formel (V) 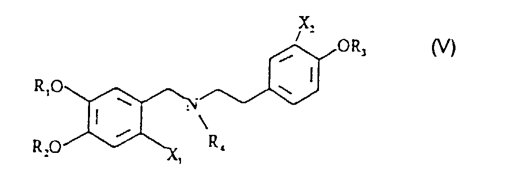 worin R₄ CHO und X₁ Wasserstoff bedeutet, mit einem Bromierungsreagenz umsetzt.

23. Verfahren nach einem der Ansprüche 1 bis 22, dadurch gekennzeichnet, daß man eine Verbindung der allgemeinen Formel (V) 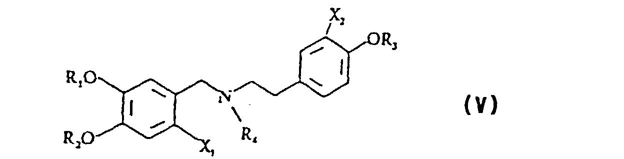 worin
R₂ ausgewählt ist aus der Gruppe bestehend aus Wasserstoff, Alkyl, Aryl, Arylcarbonyl, Aralkyl, Alkylcarbonyl und Arylcarbonyl;
X₁ ausgewählt ist aus der Gruppe bestehend aus Wasserstoff, Fluor, Chlor, Brom, Jod und t-Butyl;
R₄ ausgewählt ist aus der Gruppe bestehend aus Wasserstoff, Formyl, Aralkyl, Alkylcarbonyl, Arylcarbonyl, Aralkylcarbonyl, Alkoxycarbonyl, Aryloxycarbonyl, Aralkyloxycarbonyl, Alkylsulfonyl, Aralkylsulfonyl und Arylsulfonyl; und
R₃ Wasserstoff ist;
zu einer Verbindung der allgemeinen Formel (I), worin
R₂, R₄ und X₁ die oben genannten Bedeutungen haben;
X₂ Wasserstoff oder Brom ist; und
Y₁ und Y₂ gemeinsam =O (Keton) sind; cyclisiert, indem man mit einer Base und einem Oxidationsmittel umsetzt.

24. Verfahren nach Anspruch 23, dadurch gekennzeichnet, daß die oxidative Cyclisierung in Gegenwart von Tetraalkylammoniumchlorid, wie Aliquat, Kronenether, Ascorbinsäure, Kupferchlorid oder Trifluoressigsäure oder Mischungen derselben ausgeführt wird.

25. Verfahren zum Debromieren einer Verbindung der allgemeinen Formel (I), dadurch gekennzeichnet, daß man eine Verbindung der allgemeinen Formel (I), 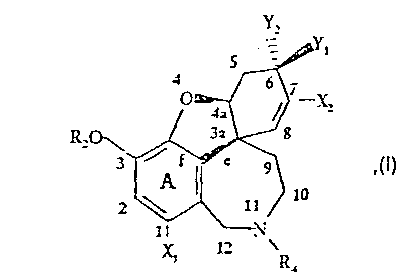 worin X₁ Brom ist;
R₂, R₄, und X₂ entweder gleich oder verschieden sind und ausgewählt sind aus der Gruppe bestehend aus Wasserstoff, Fluor, Chlor, Brom, Jod, Hydroxy, Alkoxy, niedriges Alkyl, das gegebenenfalls durch wenigstens ein Halogen substituiert ist, niedriges Alkenyl, niedriges Alkinyl, Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl und Arylsulfonyl;
Y₁ und Y₂ entweder gleich oder verschieden sind und ausgewählt sind aus der Gruppe bestehend aus Wasserstoff, Fluor, Chlor, Brom, Jod, Hydroxy, Alkoxy, niedriges Alkyl, das gegebenenfalls durch wenigstens ein Halogen substituiert ist, niedriges Alkenyl, niedriges Alkinyl, Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl oder gemeinsam =O (Keton) sind;
A ein Benzolkern ist, der gegebenenfalls wenigstens einfach durch niedriges Alkyl, niedriges Alken, niedriges Alkin, Alkoxy, Fluor, Chlor, Brom, Jod, Alkyl, das durch wenigstens ein Halogen substituiert ist, Aralkyl, Hydroxy, primäres Amino, sekundäres Amino, tertiäres Amino, Nitro, Nitril, Alkylamino, Arylamino, Aldehyd, Carbonsäure und Carbonsäurederivate substituiert ist;
zur Debromierung mit einer Mischung, bestehend aus
a) Ameisensäure, Triethylamin und Palladium/Aktivkohle oder
b) metallischem Zinkpulver und CaCl₂ in Alkohol
umsetzt.

26. Verfahren zum Herstellen von Verbindungen des Typs N-Demethylbromgalanthamin und N-Demethylepibromgalanthamin, dadurch gekennzeichnet, daß man eine Verbindung der allgemeinen Formel (I) 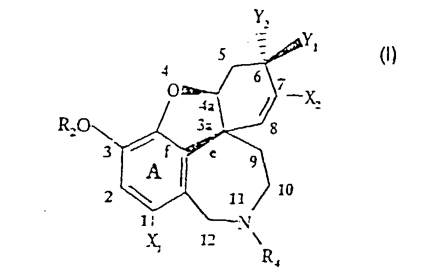 worin
R₂ Alkyl;
X₁ Brom;
R₄ CHO;
X₂ Wasserstoff;
Y₁ und Y₂ =O(Keton); sind und worin
A ein Benzolkern ist, der gegebenenfalls wenigstens einfach durch niedriges Alkyl, niedriges Alken, niedriges Alkin, Alkoxy, Fluor, Chlor, Brom, Jod, Alkyl, das durch wenigstens ein Halogen substituiert ist, Aralkyl, Hydroxy, primäres Amino, sekundäres Amino, tertiäres Amino, Nitro, Nitril, Alkylamino, Arylamino, Aldehyd, Carbonsäure und Carbonsäurederivate substituiert ist; reduziert.

27. Verfahren nach Anspruch 26,dadurch gekennzeichnet, daß man mit DIBAL-H, REDAI oder Superhydride, L-Selektride, K-Selektride, KS-Selektride oder LS-Selektride reduziert.

28. Verfahren nach einem der Ansprüche 1 bis 24,dadurch gekennzeichnet, daß die chirale Säure ausgewählt ist aus der Gruppe bestehend aus Dibenzoylweinsäure, di-p-Toluolylweinsäure, Weinsäure, Zitronensäure, Camphersäure, Camphansäure, Camphersulfonsäure oder Mandelsäure.

29. Verfahren nach Anspruch 28,dadurch gekennzeichnet, daß die chirale Säure ausgewählt ist aus der Gruppe bestehend aus (+)Di-p-Toluoyl-D-weinsäure und (-)Di-p-Toluoyi-L-weinsäure.

30. Verfahren nach einem der Ansprüche 3 bis 29,worin Y₁ und Y₂ ausgewählt sind aus der Gruppe bestehend aus OR₆ und OR₆O; X₁ Brom und R₄ CHO ist.

31. Neue Verbindung, ausgewählt aus der Gruppe bestehend aus
a) Bromgalanthamin der Formel 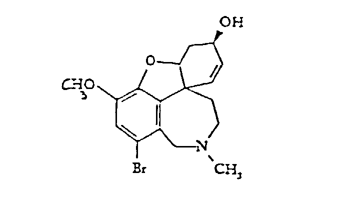
b) Epibromgalanthamin der Formel 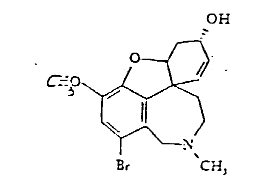
c) N-Demethylbromgalanthamin der Formel 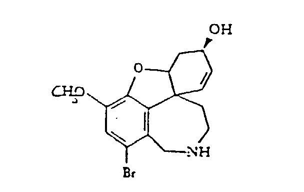
d) N-Demethyl-epibromgalanthamin der Formel 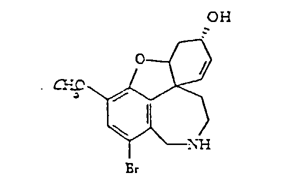
e) Brom-N-formyl-narwedin-propylenglykolketal der Formel 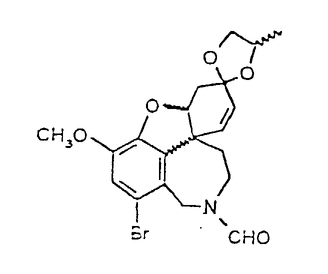
f) Narwedin-propylenglykolketal der Formel 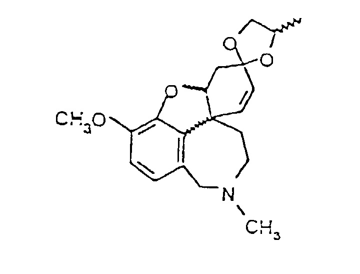
g) Brom-N-formyl-narwedin-ethylenglykolketal der Formel 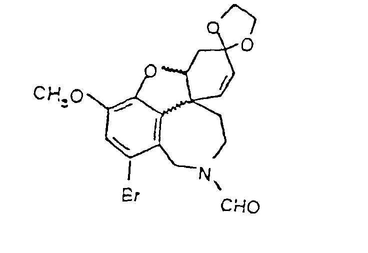
h) Narwedin-ethylenglykolketal der Formel 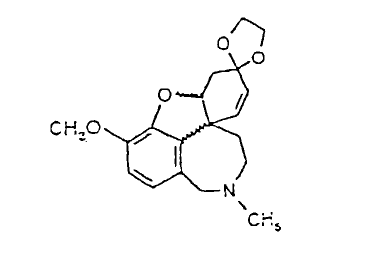
i) O-(2-Hydroxyethyl)-galanthamin der Formel 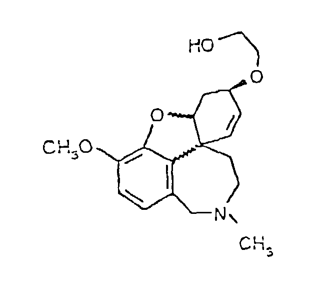
j) Brom-N-demethyl-narwedin-ethylenglykolketal der Formel 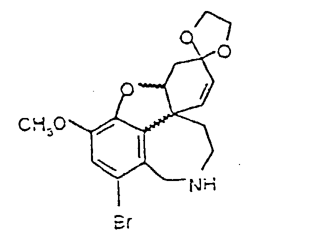
k) Brom-N-benzyl-narwedin-ethylenglykolketal der Formel 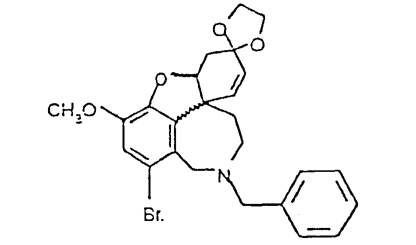
l) Brom-N-demethylnarwedin der Formel 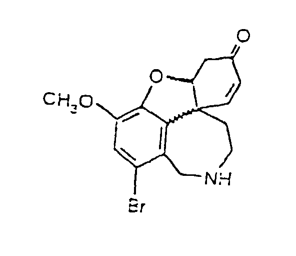
n) 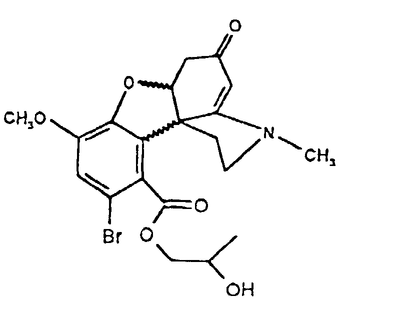
o)

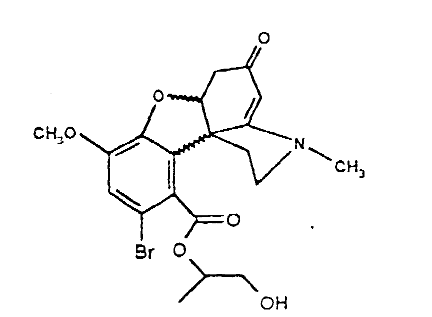

## Claims

1. Method for manufacturing derivatives of 4a, 5, 9, 10, -11, 12-hexahydro-6H-benzomro[3a, 3,2-ef][2] benzazepine with the general formula, selected from the group comprising 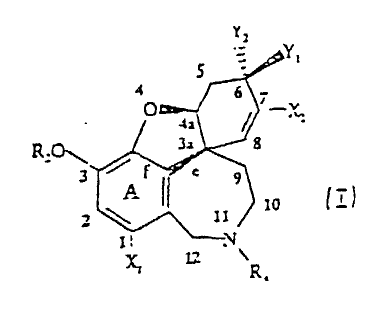 and 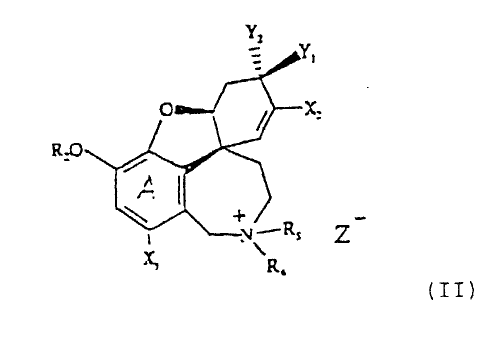 or salts of the same, in which
R₂, R₄, X₁, X₂ are either identical or different and are selected from the group comprising hydrogen, fluorine, chlorine, bromine, iodine, hydroxy, alkoxyl low alkyl, which if necessary is substituted by at least one halogen, low alkenyl, low alkinyl, aryl, aralkyl, aryloxyalkyl, formyl, alkylcarbonyl, arylcarbonyl, aralkylcarbonyl, alkyloxycarbonyl, aryloxycarbonyl, aralkyloxycarbonyl, alkylsulfonyl, aralkylsulfonyl and arylsulfonyl;
Y₁, Y₂ are either identical or different and are selected from the group comprising hydrogen, fluorine, chlorine, bromine, iodine, hydroxy, alkoxy, low alkyl, which if necessary is substituted by at least one halogen, low alkenyl, low alkinyl, aryl, aralkyl, aryloxyalkyl, formyl, alkylcarbonyl, arylcarbonyl, aralkylcarbonyl, alkyloxycarbonyl, aryloxycarbonyl, aralkyloxycarbonyl, alkylsulfonyl, aralkylsulfonyl and arylsulfonyl or are in common = O (ketone);
A is a benzole nucleus, which is if necessary singly substituted by low alkyl, low alkene, low alkine, alkoxyl fluorine, chlorine, bromine, iodine, alkyl, which is substituted by at least one halogen, aralkyl, hydroxy, primary amino, secondary amino, tertiary amino, nitro, nitrite, alkylamino, arylamino, aldehyde, carboxylic acid and carboxylic acid derivatives;
Z⁻ is an anion of a pharmaceutically acceptable, organic acid, or is an inorganic anion; and
R₅ is selected from the group comprising hydrogen, formyl, alkyl, alkenyl, aryl, aralkyl, alkylcarbonyl, arylcarbonyl, aralkylcarbonyl, alkyloxycarbonyl, aryloxycarbonyl, aralkyloxycarbonyl, alkylsulfonyl, arylsulfonyl and aralkylsulfonyl;
characterised in that
(A) a compound of the general formula (III) 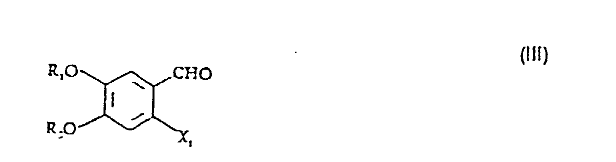 wherein
R₁ and R₂ are selected from the group comprising hydrogen, alkyl, which is if necessary substituted by at least one halogen, alkenyl, aryl, arylcarbonyl, aralkyl, alkylcarbonyl and aralkylcarbonyl; and
wherein X₁ is selected from the group comprising hydrogen, fluorine, chlorine, bromine, iodine and tertiary butyl;
(B) with a compound of the general formula (IV) 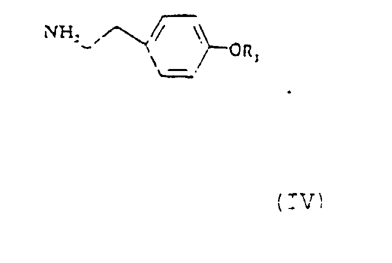 wherein R₃ is selected from the group comprising hydrogen, alkyl, alkenyl, aryl, aralkyl, alkylcarbonyl, arylcarbonyl and aralkoxycarbonyl, the group R3 being if necessary substituted by at least one halogen, is condensed to form the compound of the general formula (V) 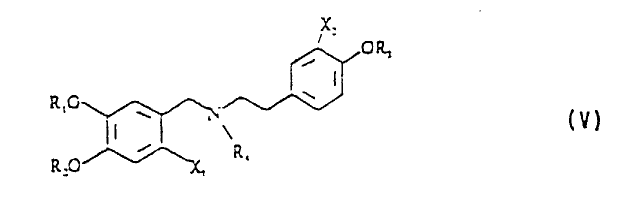 in that the obtained condensation product (Schiff's base) is reduced, an N- protective group being introduced if necessary into the compound of the general formula (V), if R₄ is hydrogen;
(C) in that the compound thus obtained of the general formula (V) is oxidatively cyclized by reacting with a base and an oxidation agent,
(D) in that a compound thus obtained of the general formula (1), in which Y₁ and Y₂ in common mean =O (ketone), is reduced with L-selectride, K-selectride, KS-selectride or LS-selectride to form the compound of the general formula (1) in which Y₁ means hydroxy and in that a racemic compound of the general formula (1) in which R4 is selected from the group comprising hydrogen, alkyl, alkenyl, alkinyl, aryl and aralkyl is separated by crystallisation with a chiral acid into the corresponding enantiomers.

2. Method for manufacturing derivatives of 4a, 5, 9, 10, -11, 12-hexahydro-6H-benzomro[3a, 3,2-ef][2] benzazepine with the general formula, selected from the group comprising 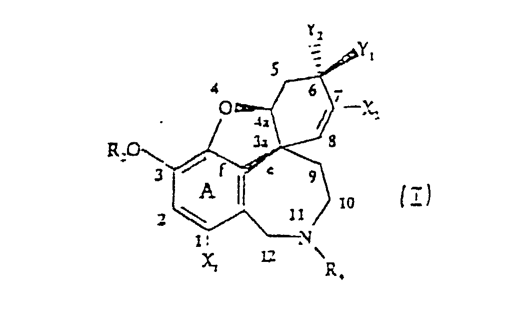 and 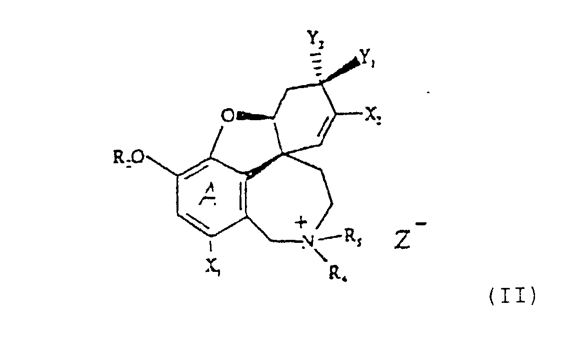 or salts of the same, in which
R₂, R₄, X₁, X₂ are either identical or different and are selected from the group comprising hydrogen, fluorine, chlorine, bromine, iodine, hydroxy, alkoxyl low alkyl, which if necessary is substituted by at least one halogen, low alkenyl, low alkinyl, aryl, aralkyl, aryloxyalkyl, formyl, alkylcarbonyl, arylcarbonyl, aralkylcarbonyl, alkyloxycarbonyl, aryloxycarbonyl, aralkyloxycarbonyl, alkylsulfonyl, aralkylsulfonyl and arylsulfonyl;
Y₁, Y₂ are either identical or different and are selected from the group comprising hydrogen, fluorine, chlorine, bromine, iodine, hydroxy, alkoxy, low alkyl, which if necessary is substituted by at least one halogen, low alkenyl, low alkinyl, aryl, aralkyl, aryloxyalkyl, formyl, alkylcarbonyl, arylcarbonyl, aralkylcarbonyl, alkyloxycarbonyl, aryloxycarbonyl, aralkyloxycarbonyl, alkylsulfonyl, aralkylsulfonyl and arylsulfonyl or are in common = 0 ketone);
A is a benzole nucleus, which is if necessary singly substituted by low alkyl, low alkene, low alkine, alkoxyl fluorine, chlorine, bromine, iodine, alkyl, which is substituted by at least one halogen, aralkyl, hydroxy, primary amino, secondary amino, tertiary amino, nitro, nitrile, alkylamino, arylamino, aldehyde, carboxylic acid and carboxylic acid derivatives;
Z⁻ is an anion of a pharmaceutically acceptable, organic acid, or is an inorganic anion; and
R₅ is selected from the group comprising hydrogen, formyl, alkyl, alkyloxycarbonyl, aryloxycarbonyl, aralkyloxycarbonyl, alkylsulfonyl, arylsulfonyl and aralkylsulfonyl;
characterised in that
(A) a compound of the general formula (III) 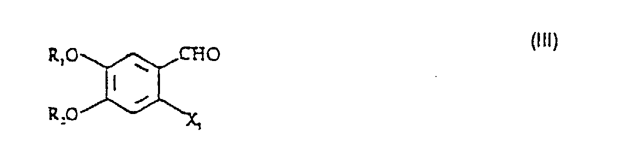 wherein R₁ and R₂ are selected from the group comprising hydrogen, alkyl, which is if necessary substituted by at least one halogen, alkenyl, aryl, arylcarbonyl, aralkyl, alkylcarbonyl and aralkylcarbonyl; and
wherein X₁ is selected from the group comprising hydrogen, fluorine, chlorine, bromine, iodine and tertiary butyl;
(B) with a compound of the general formula (IV) 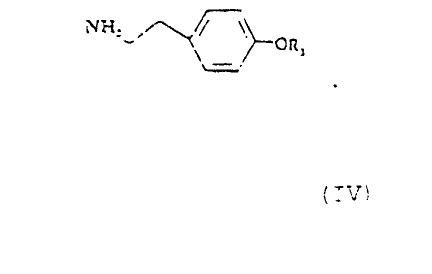 wherein R₃ is selected from the group comprising hydrogen, alkyl, alkenyl, aryl, aralkyl, alkylcarbonyl, arylcarbonyl and aralkoxycarbonyl, the group R₃ being if necessary substituted by at least one halogen, is condensed to form the compound of the general formula (V) 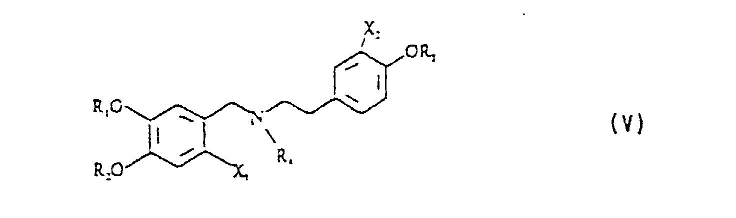 in that the obtained condensation product (Schiff's base) is reduced, an N- protective group being introduced if necessary into the compound of the general formula (V), if R₄ is hydrogen;
(C) in that the compound thus obtained of the general formula (V) is oxidatively cyclized by reacting with a base and an oxidation agent,
(D) in that a compound thus obtained of the general formula (1), in which Y₁ and Y₂ in common mean =0 (ketone) is reduced with DiBAI, REDAI or superhydride to form the compound of the general formula (1), in that the resulting diastereomers of the compound of the general formula (1), in which either Y₁ or Y₂ mean hydroxy, are separated by a chromatographic method and in that a racemic compound of the general formula (1) in which R₄ is selected from the group comprising hydrogen, alkyl, alkenyl, alkinyl, aryl and aralkyl is separated by crystallisation with a chiral acid into the corresponding enantiomers.

3. Method for manufacturing derivatives of 4a, 5, 9, 10, -11, 12-hexahydro-6H-benzofuro[3a, 3, 2-ef][2] benzazepine with the general formula, selected from the group comprising 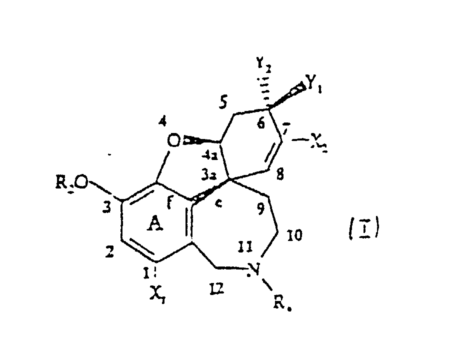 and 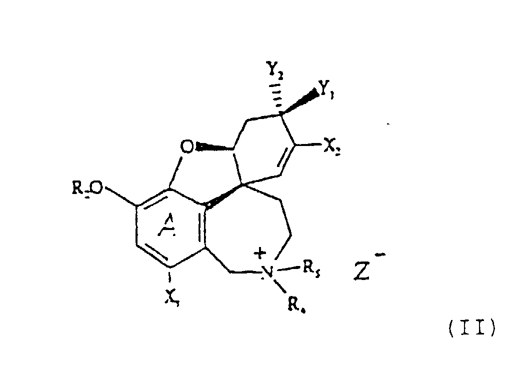 or salts of the same, in which
R₂, R₄, X₁, X₂ are either identical or different and are selected from the group comprising hydrogen, fluorine, chlorine, bromine, iodine, hydroxyl alkoxyl low alkyl, which if necessary is substituted by at least one halogen, low alkenyl, low alkinyl, aryl, aralkyl, aryloxyalkyl, formyl, alkylcarbonyl, arylcarbonyl, aralkylcarbonyl, alkyloxycarbonyl, aryloxycarbonyl, aralkyloxycarbonyl, alkylsulfonyl, aralkylsulfonyl and arylsulfonyl;
Y₁, Y₂ are either identical or different and are selected from the group comprising hydrogen, fluorine, chlorine, bromine, iodine, hydroxyl, alkoxyl low alkyl, which if necessary is substituted by at least one halogen, low alkenyl, low alkinyl, aryl, aralkyl, aryloxyalkyl, formyl, alkylcarbonyl, arylcarbonyl, aralkylcarbonyl, alkyloxycarbonyl, aryloxycarbonyl, aralkyloxycarbonyl, alkylsulfonyl, aralkylsulfonyl and arylsulfonyl or are in common = 0 ketone);
A is a benzole nucleus, which is if necessary singly substituted by low alkyl, low alkene, low alkine, alkoxyl fluorine, chlorine, bromine, iodine, alkyl, which is substituted by at least one halogen, aralkyl, hydroxy, primary amino, secondary amino, tertiary amino, nitro, nitrile, alkylamino, arylamino, aldehyde, carboxylic acid and carboxylic acid derivatives;
Z⁻ is an anion of a pharmaceutically acceptable, organic acid, or is an inorganic anion; and
R₅ is selected from the group comprising hydrogen, formyl, alkyl, alkenyl, aryl, aralkyl, alkylcarbonyl, arylcarbonyl, aralkylcarbonyl, alkyloxycarbonyl, aryloxycarbonyl, aralkyloxycarbonyl, alkylsulfonyl, arylsulfonyl and aralkylsulfonyl;
characterised in that
(A) a compound of the general formula (III) 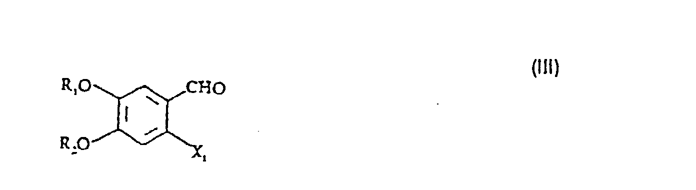 wherein R₁ and R₂ are selected from the group comprising hydrogen, alkyl, which is if necessary substituted by at least one halogen, alkenyl, aryl, arylcarbonyl, aralkyl, alkylcarbonyl and aralkylcarbonyl; and
wherein X₁ is selected from the group comprising hydrogen, fluorine, chlorine, bromine, iodine and tertiary butyl;
(B) with a compound of the general formula (IV) 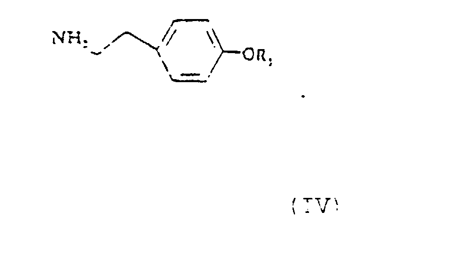 wherein R₃ is selected from the group comprising hydrogen, alkyl, alkenyl, aryl, aralkyl, alkylcarbonyl, arylcarbonyl and aralkoxycarbonyl, the group R₃ being if necessary substituted by at least one halogen, is condensed to form the compound of the general formula (V) 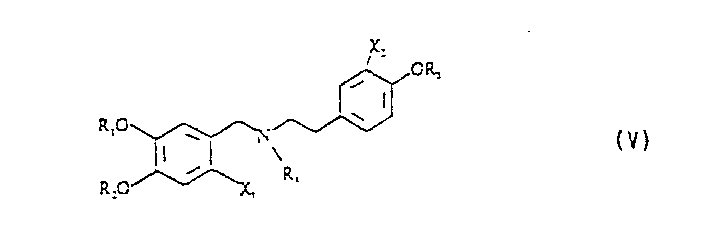 in that the obtained condensation product (Schiff's base) is reduced, an N- protective group being introduced if necessary into the compound of the general formula (V), if R₄ is hydrogen;
(C) in that the compound thus obtained of the general formula (V) is oxidatively cyclized by reacting with a base and an oxidation agent,
(D) in that a compound thus obtained of the general formula (1), in which Y₁ and Y₂ in common mean =O (ketone), is transformed into a ketal or a thioketal, in that the ketal or thioketal obtained thus is reduced, in that the ketal or thioketal group is split in the resulting compound from the type narwedine ketal, in that a racemic compound of the general formula (1) in which R₄ is selected from the group comprising hydrogen, alkyl, alkenyl, alkinyl, aryl and aralkyl is transformed by chirally-induced crystallisation into the corresponding enantiomers and in that the enantiomers obtained thus are reduced with L-selectrides, K-selectrides, KS-selectrides or LS-selectrides to form the compound of the general formula (1).

4. Method for manufacturing derivatives of 4a, 5, 9, 10, -11, 12-hexahydro-6H-benzofuro[3a, 3, 2-ef][2] benzazepine with the general formula, selected from the group comprising 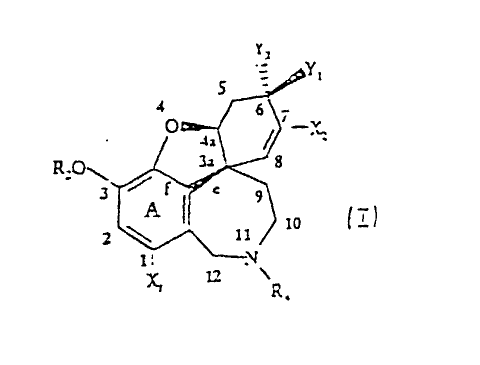 and 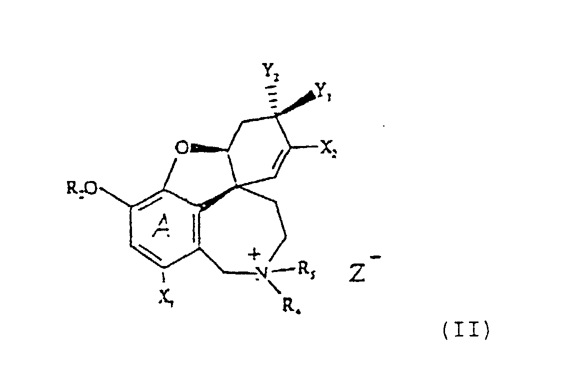 or salts of the same, in which
R₂, R₄, X₁, X₂ are either identical or different and are selected from the group comprising hydrogen, fluorine, chlorine, bromine, iodine, hydroxy, alkoxyl low alkyl, which if necessary is substituted by at least one halogen, low alkenyl, low alkinyl, aryl, aralkyl, aryloxyalkyl, formyl, alkylcarbonyl, arylcarbonyl, aralkylcarbonyl, alkyloxycarbonyl, aryloxycarbonyl, aralkyloxycarbonyl, alkylsulfonyl, aralkylsulfonyl and arylsulfonyl;
Y₁, Y₂ are either identical or different and are selected from the group comprising hydrogen, fluorine, chlorine, bromine, iodine, hydroxy, alkoxy, low alkyl, which if necessary is substituted by at least one halogen, low alkenyl, low alkinyl, aryl, aralkyl, aryloxyalkyl, formyl, alkylcarbonyl, arylcarbonyl, aralkylcarbonyl, alkyloxycarbonyl, aryloxycarbonyl, aralkyloxycarbonyl, alkylsulfonyl, aralkylsulfonyl and arylsulfonyl or are in common = 0 (ketone);
A is a benzole nucleus, which is if necessary singly substituted by low alkyl, low alkene, low alkine, alkoxyl fluorine, chlorine, bromine, iodine, alkyl, which is substituted by at least one halogen, aralkyl, hydroxy, primary amino, secondary amino, tertiary amino, nitro, nitrile, alkylamino, arylamino, aldehyde, carboxylic acid and carboxylic acid derivatives;
Z⁻ is an anion of a pharmaceutically acceptable, organic acid, or is an inorganic anion; and
R₅ is selected from the group comprising hydrogen, formyl, alkyl, alkenyl, aryl, aralkyl, alkylcarbonyl, arylcarbonyl, aralkylcarbonyl, alkyloxycarbonyl, aryloxycarbonyl, aralkyloxycarbonyl, alkylsulfonyl, arylsulfonyl and aralkylsulfonyl;
characterised in that a compound of the general formula (Va) 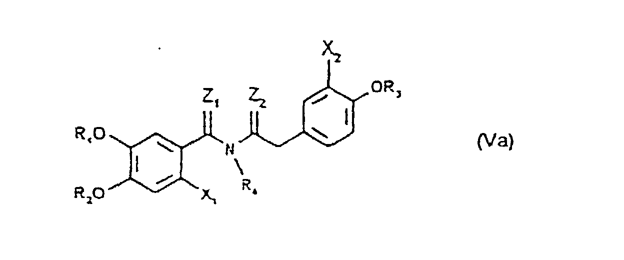 wherein R₁, R₂, R₃, R₄, X₁, X₂ have the meanings mentioned in Claim 1 and wherein Z₁ and Z₂ mean =O, S, N, an N protective group being introduced if necessary into the compound of the general formula (Va), if R₄ is hydrogen, is oxidatively cyclized by reacting with a base and an oxidation agent to form a compound of the general formula (1a), 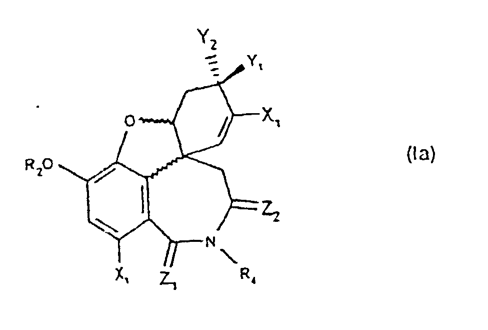 (D) in that a compound thus obtained of the general formula (1), in which Y₁ and Y₂ in common mean =O (ketone), is transformed into a ketal or a thioketal, in that the ketal or thioketal obtained thus is reduced, in that the ketal or thioketal group is split in the resulting compound from the type narwedine ketal, in that a racemic compound of the general formula (1) in which R₄ is selected from the group comprising hydrogen, alkyl, alkenyl, alkinyl, aryl and aralkyl is transformed by chirally-induced crystallisation into the corresponding enantiomers and in that the enantiomers obtained thus are reduced with L-selectrides, K-selectrides, KS-selectrides or LS-selectrides to form the compound of the general formula (1).

5. Method according to one of the Claims 1 to 4, characterised in that the condensation reaction (stage B) is performed in a solvent at reflux temperature and if necessary separates out any water arising.

6. Method according to one of the Claims 1 to 5, characterised in that the reduction of the condensation product (Schiff's base) obtained in stage B is performed with a reduction medium which is selected from the group comprising sodium borohydride, potassium borohydride, sodium cyanoborohydride, LiAIH₄ and mixtures of the same.

7. Method according to one of the Claims 1 to 6, characterised in that in stage C the compound of the general formula (V) or (Va) is converted with a base which is selected from the group comprising sodium hydrogen carbonate, potassium carbonate, NaOH, KOH and pyridine.

8. Method according to one of the Claims 1 to 7, characterised in that in stage C the compound of the general formula (V) or (Va) is converted with an oxidisation medium which is selected from the group comprising Pb(OAc)₄, KMnO₄, iron chloride, ferrous potassium cyanide and H₂O₂.

9. Method according to one of the Claims 1 to 8, characterised in that conversion at stage C is performed in a solvent selected from the group comprising toluene and xylene.

10. Method according to one of the Claims 3 to 9, characterised in that in stage D the compound of the general formula (1) or (1a), in which Y₁ and Y₂ in common mean = O (ketone), is converted into a ketal or thioketal, the compound of the general formula (1) or (1a) being converted with a compound selected from the group comprising alcohol R₆-OH and a thiol R₆-SH, wherein R₆ is selected from the group comprising alkyl, alkenyl, aryl, aralkyl, alkylcarbonyl, arylcarbonyl and aralkylcarbonyl, R₆ being substituted by at least one halogen if necessary.

11. Method according to one of the Claims 3 to 9, characterised in that in stage D the compound of the general formula (1) or (la), in which Y₁ and Y₂ in common mean =0 (ketone) are converted into a ketal or thioketal, the compound of the general formula (1) being converted with a diol R₆(OH)₂ or a dithiol R₆(SH)₂, R₆ having the meanings mentioned in Claim 10.

12. Method according to Claim 11, characterised in that in stage D conversion is performed with propylene glycol to form the ketone.

13. Method according to one of the Claims 3 to 12 characterised in that the ketal or thioketal is reduced with a reduction medium selected from the group comprising sodium borohydride, potassium borohydride, sodium cyanoborohydride, LiAIH₄, L-selectrides, DiBAI, REDAI, K-selectddes, KS-selectrides, LS-selectrides, superhydrides, 9-BBN, Zn/CacL₂ and mixtures of the same.

14. Method according to Claim 13, characterised in that the reduction means is LiAIH₄.

15. Method according to Claims 1 to 14, characterised in that the compound obtained is the quaternary ammonium salt of the general formula (II).

16. Method according to Claim 15, characterised in that Z- is selected from the group comprising tartrate, lactate, citrate, acetate, maleinate, fluoride, chloride, bromide, iodide, sulphate, phosphate and chlorate.

17. Method according to Claim 16, characterised in that R₁, R₂ and R₃ are selected from the group comprising hydrogen, alkyl, alkenyl, aryl, aralkyl, alkylcarbonyl, arylcarbonyl and aralkylcarbonyl, R₁, R₂ and R₃ being if necessary substituted by at least one halogen.

18. Method according to one of Claims 1 to 16, characterised in that R₄ and R₅ are selected from the group comprising hydrogen, formyl, alkyl, alkenyl, aryl, aralkyl, alkylcarbonyl, arylcarbonyl, aralkylcarbonyl, alkylsulfonyl, arylsulfonyl and aralkylsulfonyl, R₄ and R₅ being if necessary substituted by at least one halogen.

19. Method according to one of Claims 1 to 18, characterised in that X₁ and X₂ are selected from the group comprising hydrogen, fluorine, chlorine, bromine, iodine and t-butyl.

20. Method according to one of Claims 1 to 19, characterised in that the nitrogen in the compound of the general formula (V) or (Va) is protected before oxidation by introduction of a compound selected from the group comprising formyl, aralkyl, alkylcarbonyl, arylcarbonyl aralkylcarbonyl, aralkyloxycarbonyl, alkylsulfonyl, aralkylsulfonal and arylsulfonyl.

21. Method according to Claim 20, characterised in that a formyl group is introduced, the compound of the general formula (V) or (Va) being converted with 1 to 50 times the molar quantity of ethyl formate in the presence of catalytic quantities of formic acid.

22. Method according to one of Claims 1 to 21, characterised in that a compound of the general formula (V) is produced, wherein R₁, R₂ and R₃ are selected from the group comprising hydrogen, alkyl, aryl, aralkyl, alkylcarbonyl, arylcarbonyl and aralkylcarbonyl;
X₁ is bromine;
X₂ is hydrogen; and
R₄ is selected from the group comprising hydrogen, formyl, aralkyl, alkylcarbonyl,arylcarbonyl, aralkylcarbonyl, alkyloxycarbonyl, aryloxycarbonyl, aralkyloxycarbonyl, alkylsulfonyl, aralkylsulfonyl and arylsulfonyl,
a compound of the general formula (V) 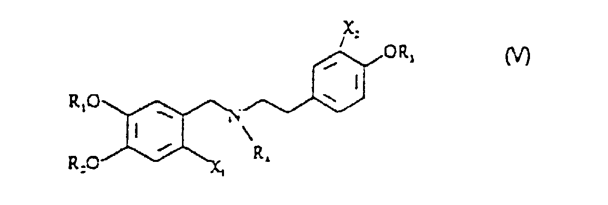 wherein R₄ means CHO and X₁ hydrogen, being converted with a bromination reagent.

23. Method according to one of Claims 1 to 22, characterised in that a compound of the general formula (V) 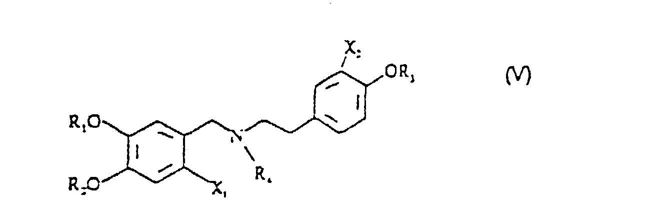 wherein
R₂ is selected from the group comprising hydrogen, alkyl, aryl, arylcarbonyl, aralkyl, alkylcarbonyl and arylcarbonyl;
X₁ is selected from the group comprising hydrogen, fluorine, chlorine, bromine, iodine and t-butyl;
R₄ is selected from the group comprising hydrogen, formyl, aralkyl, alkylcarbonyl, arylcarbonyl, aralkylcarbonyl, alkoxycarbonyl, aryloxycarbonyl, aralkyloxycarbonyl, alkylsulfonyl, aralkylsulfohyl and arylsulfonyl; and
R₃ is hydrogen;
is cyclised to form a compound of the general formula (I) wherein
R₂, R₄ and X₁ have the abovenamed meanings;
X₂ is hydrogen or bromine; and
Y₁ and Y₂ are in common =0 (ketone), conversion being carried out with a base and an oxidising agent.

24. Method according to Claim 23, characterised in that the oxidative cyclisation is carried out in the presence of tetraalkylammonium chloride, such as aliquat, crown ether, ascorbic acid, copper chloride trifluoroacetic acid or mixtures of the same.

25. Method of debrominating a compound of the general formula (1), characterised in that a compound of the general formula (1) is converted 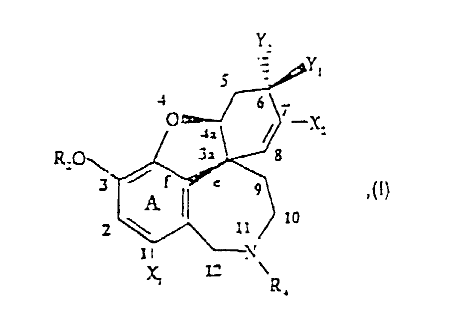 wherein
X₁ is bromine;
R₂, R₄ and X₂ are either identical or different and are selected from the group comprising hydrogen, fluorine, chlorine, bromine, iodine, hydroxy, alkoxy, low alkyl, which if necessary is substituted by at least one halogen, low alkenyl, low alkynil, aryl, aralkyl, aryloxyalkyl, formyl, alkylcarbonyl, arylcarbonyl, aralkylcarbonyl, alkyloxycarbonyl, aryloxycarbonyl, aralkyloxycarbonyl, alkylsulfonyl, aralkylsulfonyl and arylsulfonyl;
Y₁ and Y₂ are either identical or different and are selected from the group comprising hydrogen, fluorine, chlorine, bromine, iodine, hydroxy, alkoxy, low alkyl, which if necessary is substituted by at least one halogen, low alkenyl, low alkinyl, aryl, aralkyl, aryloxyalkyl, formyl, alkylcarbonyl, arylcarbonyl, aralkylcarbonyl, alkyloxycarbonyl, aryloxycarbonyl, aralkyloxycarbonyl, alkylsulfonyl, aralkylsulfonyl and arylsulfonyl or are in common = 0 (ketone);
A is a benzole nucleus, which is if necessary singly substituted by low alkyl, low alkene, low alkine, alkoxyl fluorine, chlorine, bromine, iodine, alkyl, which is substituted by at least one halogen, aralkyl, hydroxy, primary amino, secondary amino, tertiary amino, nitro, nitrile, alkylamino, arylamino, aldehyde, carboxylic acid and carboxylic acid derivatives;
for debromination with a mixture comprising
a) formic acid and triethylamine and palladium/activated carbon or
b) metallic zinc powder and CaCl₂ in alcohol.

26. Method of producing a compounds of the type N-demethylbromine I galanthamine and N-demethyl-epibromine galanthamine, characterised in that a compound of the general formula (I) 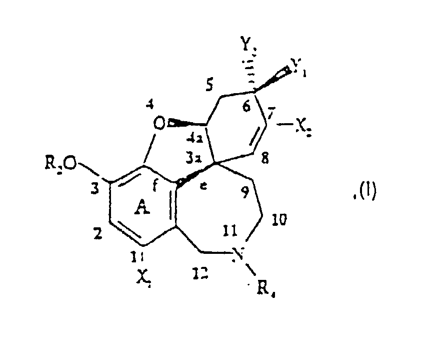 wherein
R₂ is alkyl;
X₁ bromine;
R₄ CHO;
X₂ hydrogen;
Y₁ and Y₂ are in common =0 (ketone) and; wherein
A is a benzole nucleus, which is if necessary singly substituted by low alkyl, low alkene, low alkine, alkoxy, fluorine, chlorine, bromine, iodine, alkyl, which is substituted by at least one halogen, aralkyl, hydroxy, primary amino, secondary amino, tertiary amino, nitro, nitrile, alkylamino, arylamino, aldehyde, carboxylic acid and carboxylic acid derivatives, is reduced.

27. Method according to Claim 26, characterised in that reduction is carried out with DIBAL-H, REDAI or superhydrides, L-selectrides, K-selectrides, KS-selectrides or LS-selectrides.

28. Method according to Claim 1 to 24, characterised in that the chiral acid is selected from the group comprising dibenzoyl tartaric acid, di-p-toluolyl tartaric acid, tartaric acid, citric acid, camphoric acid, camphanic acid, camphoric sulfonic acid or mandelic acid.

29. Method according to Claim 28, characterised in that the chiral acid is selected from the group comprising (+)Di-p-toluoyl-D-tartaric acid and (-)Di-p-toluoyl-L-tartaric acid.

30. Method according to one of Claims 3 to 29, wherein Y₁ and Y₂ are selected from the group comprising OR₆ and OR₆O; X₁ is bromine and R₄ is CHO.

31. New compound selected from the group comprising
a) bromine galanthamine of the formula 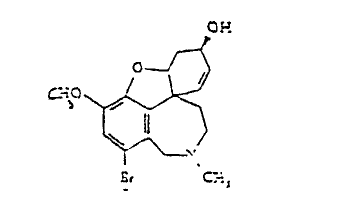
b) epibromine galanthamine of the formula 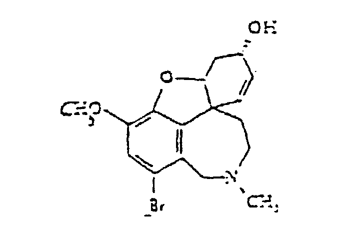
c) N-demethylbromine galanthamine of the formula 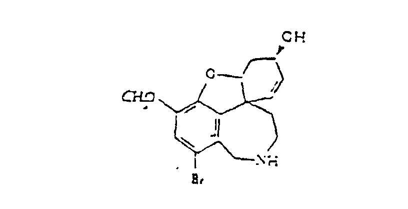
d) N-demethyl-epibronine galanthamine of the formula 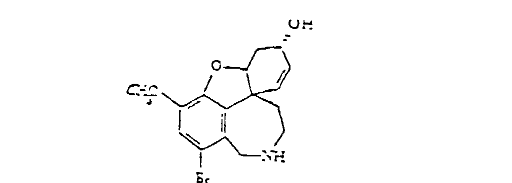
e) bromine-N-formyl-narwedine-propylene glycol ketal of the formula 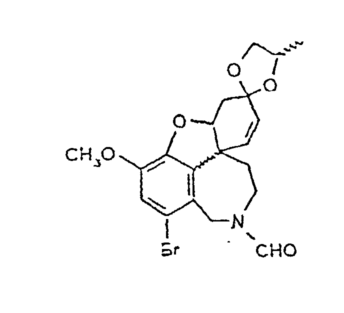
f) narwedine-propylene glycol ketal of the formula 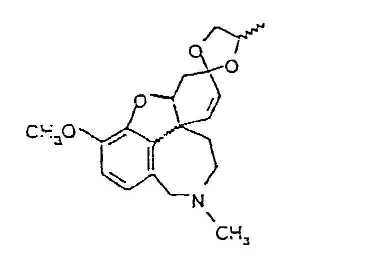
g) bromine-N-formyl-narwedine-ethylene glycol ketal of the formula 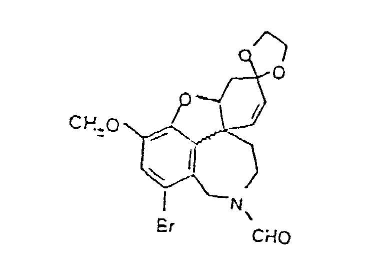
h) narwedine-ethylene glycol ketal of the formula 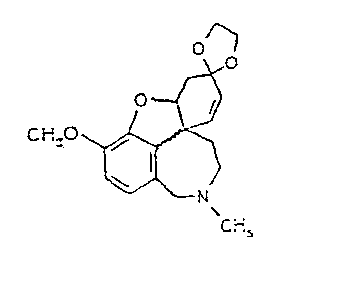
i) O-(2-hydroxyethyl)-galanthamine of the formula 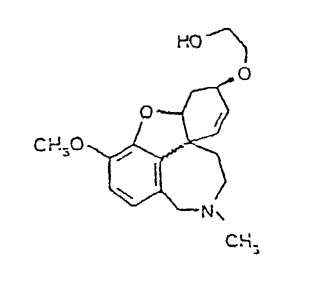
j) bromine-N-demethyl-narwedine-ethylene glycol ketal of the formula 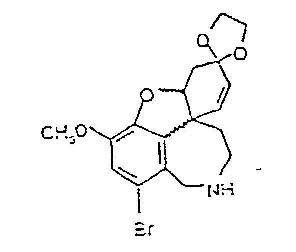
k) bromine-N-benzyl-narwedine-ethylene glycol ketal of the formula 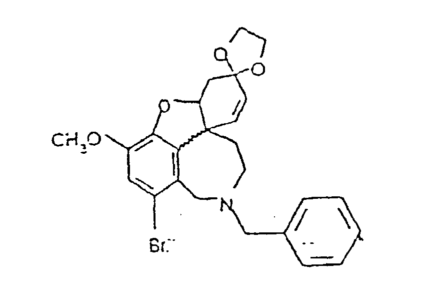
l) bromine-N-demethylnarwedine of the formula 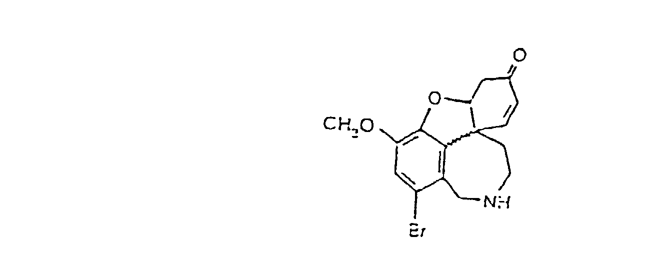
n) 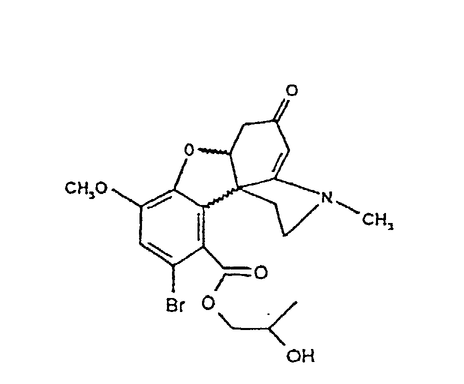
o)

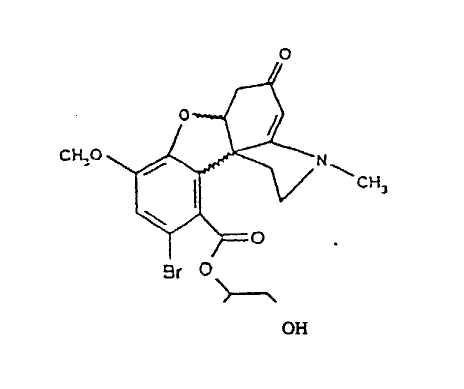

## Revendications

1. Procédé de préparation de dérivés de 4a,5,9,10,-11,12-hexahydro-6H-benzofuro[a,3,2-ef][2]benzazépine avec la formule générale choisie parmi le groupe constitué de 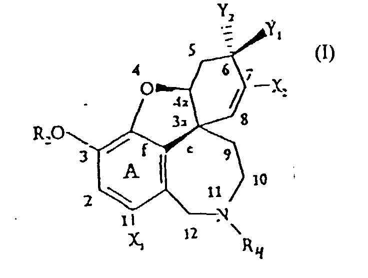 et 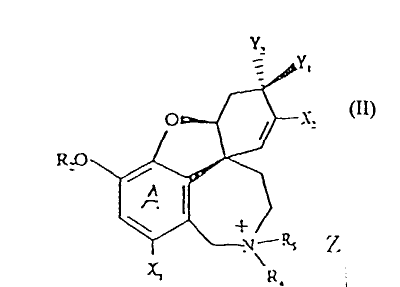 ou parmi des sels de ce groupe, où
R₂, R₄, X₁ et X₂ sont soit identiques soit différents et choisis parmi le groupe constitué d'hydrogène, de fluore, de chlore, de brome, d'iode, d'hydroxy, d'alkoxy, d'un alkyle de faible indice substitué, le cas échéant, par un halogène au moins, d'un alkényle de faible indice, d'alkinyle de faible indice, d'aryle, d'aralkyle de faible indice, d'araloxyalkyle, de formyle, d'alkylcarbonyle, d'arylcarbonyle, d'aralkylcarbonyle, d'alkyloxycarbonyle, d'aryloxycarbonyle, d'aralkylecarbonyle, d'alkylsulfonyle, d'aralkylsulfonyle et d'arylsulfonyle ;
où Y₁ et Y₂ sont soit identiques, soit différents et choisis parmi le groupe constitué de d'hydrogène, de fluore, de chlore, de brome, d'iode, d'hydroxy, d'alkoxy, d'un faible alkyle substitué, le cas échéant par un halogène au moins, d'un faible alcènyle, d'un faible alkinyle, d'aryle, d'aralkyle, d'aryloxyalkyle, de formyle, d'alkylecarbonyle, d'arylcarbonyle, d'aralkylcarbonyle, d'alkyloxycarbonyle, d'aryloxycarbonyle, d'aralkyloxycarbonyle, d'alkylsulfonyle, d'aralkylesulfonyle, d'arylsulfonyle ou qui sont ensemble = O (cétone) ;
où A est un noyau benzénique qui est substitué, le cas échéant, au moins une fois par de l'alkyle de faible indice, de l'alcène de faible indice, de l'alkine de faible indice, de l'alkoxy, du fluore, du chlore, du brome, de l'iode, de l'alkyle qui est substitué par au moins un halogène, par de l'aralkyle, de l'hydroxy, de l'amino primaire, de l'amino secondaire, de l'amino tertiaire, du nitro, du nitrile, de l'alkylamino, de l'aldéhyde, de l'acide de carbone et de dérivés d'acide de carbone ;
où Z est un anion d'un acide organique acceptable pharmaceutiquement ou un anion anorganique ; et
où R₅ est choisi parmi le groupe constitué d'hydrogène, de formyle, d'alkyle, d'alkényle, d'aryle, d'aralkyle, d'alkylecarbonyle, d'arylcarbonyle, d'aralkylecarbonyle, d'alkyloxycarbonyle, d'aryloxycarbonyle, d'aralkyloxycarbonyle, d'alkylsulfonyle, d'arylsulfonyle et d'aralkylsulfonyle;
caractérisé en ce que l'on condense
(A)= un produit de la formule générale (III) 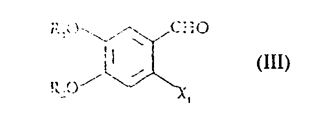
dans laquelle R₁ et R₂ sont choisis parmi le groupe constitué d'hydrogène, d'alkyle substitué, le cas échéant, par un halogène au moins, d'alkényle, d'aryle, d'arylcarbonyle, d'aralkyle, d'alkylecarbonyle et d'aralkylcarbonyle ; et
dans laquelle X₁ est choisi parmi le groupe constitué d'hydrogène, de fluore, de chlore, de brome, d'iode et de butyle tertiaire ;
(B) avec un produit de la formule générale (IV) 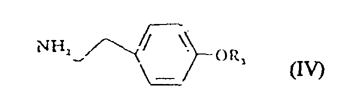
où R₃ est choisi parmi le groupe constitué d'hydrogène, d'alkyle, d'alkényle, d'aryle, d'aralkyle, d'alkylcarbonyle, d'arylcarbonyle et d'aralkoxycarbonyle, le groupe, le cas échéant R₃, étant substitués par un halogène au moins, pour former un produit de la formule générale (V) 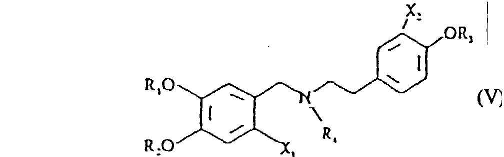
que l'on réduit le produit de condensation obtenu (base de Schiff), en introduisant, le cas échéant, si R₄ est de l'hydrogène, un groupe de protection N dans le composé de la formule générale V.
(C) que le composé ainsi obtenu de la formule générale (V) est cyclisé oxydativement en transformant avec une base et un agent d'oxydation,
(D) que l'on réduit le composé, ainsi obtenu, de la formule générale (I), dans laquelle Y₁ et Y₂ signifient tous deux O (cétone), avec L-Selektride, K-Selektride, KS-Selektride ou LS-Selektride en un composé de la formule générale (I), dans laquelle Y₁ signfie hydroxy, et en ce que l'on décompose un composé racémique de la formule générale (I), dans laquelle R₄ est choisi parmi le groupe constitué de d'hydrogène, d'alkyle, d'alkényle, d'alkinyle, d'aryle et d'aralkyle, par cristallisation avec un acide chiral dans les énantiomères correspondants.

2. Procédé de préparation de dérivés de 4a,5,9,10,-11,12-hexahydro-6H-benzofuro[3a,3,2-ef][2]benzazépine avec la formule générale choisi parmi le groupe constitué de 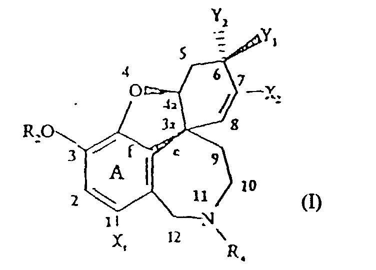 et 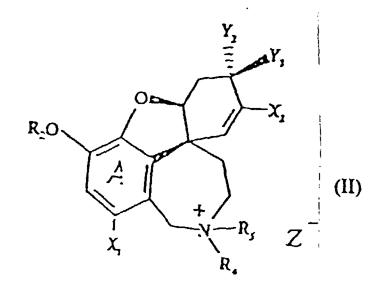 ou de sels de celui-ci, où
R₂, R₄, X₁ et X₂ sont soit identiques, soit différents et choisis parmi le groupe constitué d'hydrogène, de fluore, de chlore, de brome, d'iode, d'hydroxy, d'alkoxy, d'alkyle de faible indice, le cas échéant, substitué par un halogène au moins, d'alkényle de faible indice, d'alkinyle de faible indice, d'aryle, d'aralkyle, d'aryloxyalkyle, de formyle, d'alkylecarbonyle, d'arylcarbonyle, d'aralkylcarbonyle, d'alkyloxycarbonyle, d'aryloxycarbonyle, d'aralkyloxycarbonyle, d'alkylsulfonyle, d'aralkylsulfonyle et d'arylsulfonyle ;
où Y₁ et Y₂ sont soit identiques, soit différents et choisis parmi le groupe constitué d'hydrogène, de fluore, de chlore, de brome, d'iode, d'hydroxy, d'alkoxy, d'alkyle de faible indice, le cas échéant, substitué par un halogène au moins, de l'alkényle de faible indice, de l'alkinyle de faible indice, d'aryle, d'aralkyle, d'arylloxyalkyle, de formyle, d'alkylecarbonyle, d'arylcarbonyle, d'aralkylcarbonyle, d'alkyloxycarbonyle, d'aryloxycarbonyle, d'aralkyloxycarbonyle, d'alkylsulfonyle, d'aralkylsulfonyle et d'arylsulfonyle ou qu'ils sont ensemble = O (cétone) ;
où A est un noyau benzénique, qui est substitué au moins une fois par de l'alkyle de faible indice, de l'alcène, de l'alkine de faible indice, de l'alkoxy, du fluore, du chlore, du brome, de l'iode, de l'alkyle qui est substitué par un halogène au moins, de l'aralkyle, de l'hydroxy, de l'amino primaire, de l'amino secondaire, de l'amino tertiaire, du nitro, du nitrile, de l'alkylamino, de l'arylamino, de l'aldéhyde, de l'acide de carbone et des derivés de l'acide de carbone.
où Z est un anion d'un acide organique pharmaceutiquement acceptable ou un anion anorganique ; et où
R₅ est choisi parmi le groupe constitué de d'hydrogène, de formyle, d'alkyle, d'alkényle, d'aryle, d'aralkyle, d'alkylecarbonyle, d'arylcarbonyle, d'aralkylecarbonyle, d'alkyloxycarbonyle, d'aryloxycarbonyle, d'aralkyloxycarbonyle, d'alkylsulfonyle, d'arylsulfonyle et d'aralkylsulfonyle ;
caractérisé en ce que l'on condense
(A) un produit de la formule générale (III) 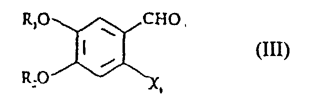
où R₁ et R₂ sont choisis parmi le groupe constitué d'hydrogène, d'alkyle, le cas échéant, substitué par un halogène au moins, d'alkenyle, d'aryle, d'arylcarbonyle, d'aralkyle, d'alkylcarbonyle et d'aralkylcarbonyle ; et
où X₁ est choisi parmi le groupe constitué d'hydrogène, de fluore, de chlore, de brome, d'iode et de butyle tertiaire ;
(B) avec un produit de la formule générale (IV) 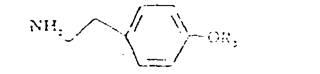
dans laquelle R₃ est choisi parmi le groupe constitué d'hydrogène, d'alkyle, d'alkényle, d'aryle, d'aralkyle, d'alkylecarbonyle, d'arylcarbonyle et d'aralkoxycarbonyle, le groupe, le cas échéant R₃ étant substitué par un halogène au moins, pour former le composé de la formule générale (V), 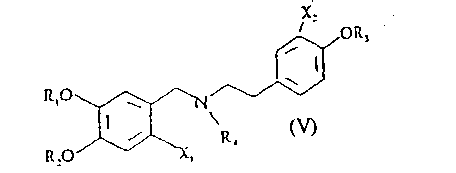
que l'on réduit le produit de la condensation (base de Schiff), en introduisant, le cas échéant, si R₄ est de l'hydrogène, un groupe de protection N dans le composé de la formule générale (V),
(C) que le produit de la formule générale (V), que l'on a obtenu de cette façon, est cyclisé oxydativement en transformant avec une base et un agent d'oxydation,
(D) que l'on réduit le produit, obtenu de la formule générale (I), dans laquelle Y₁ et Y₂ signifient ensemble O (cétone), à l'aide de DiBAI, de REDAI ou des Superhydrures pour obtenir un composé de la formule générale (I), que l'on dissocie les diastéreo-isomères du produit de la formule générale (I), dans laquelle soit Y₁, soit Y₂ est de l'hydroxy, au moyen d'un procédé chromatographique, et que l'on décompose un composé racémique de la formule générale (I), dans laquelle R₄ est choisi parmi le groupe constitué d'hydrogène, d'alkyle, d'alkényle, d'alkinyle, d'aryle et d'aralkyle, par cristallisation avec un acide chiral en enantiomères correspondants.

3. Procédé de préparation de dérivés de la 4a,5,9,10,-11,12-hexahydro-6H-benzofuro[3a,3,2-][2]benzazépine avec la formule générale, choisi parmi le groupe constitué de 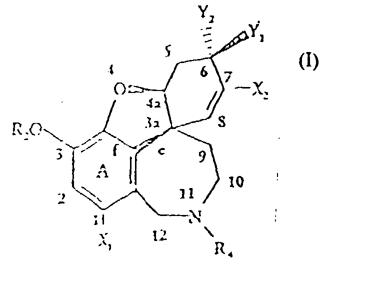 et 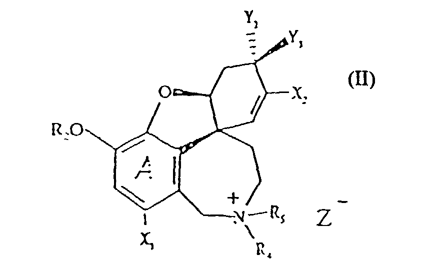 ou de sels de celui-ci, où
R₂, R₄, X₁ et X₂ sont soit identiques, soit différents et choisis parmi le groupe constitué d'hydrogène, de fluore, de chlore, de brome, d'iode, d'hydroxy, d'alkoxy, d'alkyle de faible indice, le cas échéant substitué par un halogène au moins, d'alkenyle, d'alkinyle de faible indice, d'aryle, d'aralkyle, d'aryloxyalkyle, de formyle, d'alkylecabronyle, d'arylcarbonyle, d'aralkylecarbonyle, d'alkyloxycarbonyle, d'aryloxycarbonyle, d'aralkyloxycarbonyle, d'alkylsulfonyle, d'aralkylsulfonyle et d'arylsulfonyle ;
où Y₁ et Y₂ sont soit identiques, soit différents et choisis parmi le groupe constitué d'hydrogène, de fluore, de chlore, de brome, d'iode, d'hydroxy, d'alkoxy, d'alkyle de faible indice, qui est substitué le cas échéant par un halogène au moins, d'alkényle de faible indice, d'alkinyle de faible indice, d'aryle, d'aralkyle, d'aryloxyalkyle, de formyle, d'alkylecarbonyle, d'arylcarbonyle, d'aralkylcarbonyle, d'alkyloxycarbonyle, d'aryloxycarbonyle, d'aralkyloxycarbonyle, d'alkylsulfonyle, d'aralkylsulfonyle, d'arylsulfonyle ou sensemble = O (cétone) ;
où A est un noyau benzénique, qui est, le cas échéant, substitué une fois au moins par de l'alkyle de faible indice, par de l'alcène de faible indice, par de l'alkine de faible indice, par de l'alkoxy, du fluore, du chlore, du brome, de l'iode, de l'alkyle qui est substitué par un halogène au moins, de l'aralkyle, de l'hydroxy, de l'amino primaire, de l'amino secondaire, de l'amino tertiaire, du nitro, du nitrile, de l'alkylamino, de l'arylmino, de l'aldéhyde, de l'acide de carbone, et de dérivés d'acide de carbone ;
où Z est un anion d'un acide organique pharmaceutiquement acceptable ou un anion anorganique et
où R₅ est choisi parmi le groupe constitué d'hydrogène, de formyle, d'alkyle, d'alkényle, d'aryle, d'aralkyle, d'alkylecarbonyle, d'arlylcarbonyle, d'aralkylcarbonyle, d'alkyloxycarbonyle, d'aryloxycarbonyle, d'aralkyloxycarbonyle, d'alkylesulfonyle, d'arylesulfonyle et d'aralkylesulfonyle;
caractérisé en ce que l'on condense
(A) un produit de la formule générale (III) 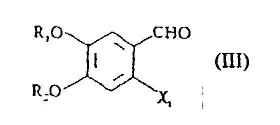
dans laquelle R₁ et R₂ sont choisis parmi le groupe constitué d'hydrogène, d'alkyle substitué, le cas échéant, par un halogène au moins, d'alkényle, d'aryle, d'arylcarbonyle, d'aralkyle, d'alkylcarbonyle et d'aralkylcarbonyle; et
où X₁ est choisi parmi le groupe constitué d'hydrogène, de fluore, de chlore, de brome, d'iode et de butyle tertiaire ;
(B) avec un produit de la formule générale (IV) 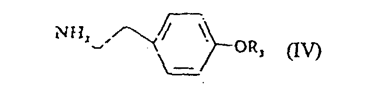
où R₃ est choisi parmi le groupe constitué d'hydrogène, d'alkyle, d'alkényle, d'aryle, d'aralkyle, d'alkylecarbonyle, d'arylcarbonyle et d'aralkoxycarbonyle, le groupe, le cas échéant R₃, étant substitué par un halogène au moins, pour obtenir un composé de la formule générale (V) 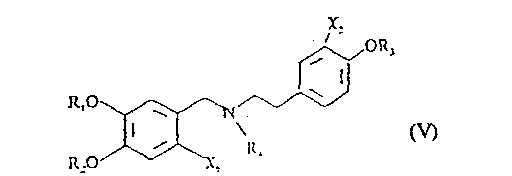
que l'on réduit le produit de la condensation obtenu (base de Schiff),
en introduisant, le cas échéant, si R₄ est de l'hydrogène, un groupe de protection N dans le composé de la formule générale (V) ;
(C) que le composé ainsi obtenu de la formule générale (V) est cyclisé oxydativement en transformant avec une base et un agent d'oxydation,
(D) que l'on transforme un composé ainsi obtenu de la formule générale (I), dans laquelle Y₁ et Y₂ signifient, ensemble, O (cétone), en un cétal ou un thiocétal, que l'on réduit le cétal ou le thiocétal ainsi obtenu que l'on décompose le groupe cétal ou thiocétal dans le composé de type narwédinecétal formé, que l'on transforme un composé racémique de la formule générale (I), dans laquelle R₄ est choisi parmi un groupe constitué d'hydrogène, d'alkyle, d'alkényle, d'aryle et d'aralkyle, par cristallisation induite par chiralité en l'énantiomère correspondant et que l'on réduit l'énantiomère ainsi obtenu avec L-Selektride, K-Selektride, KS-Selektride ou LS-Selektride au produit de la formule générale (I).

4. Procédé de préparation de dérivés de la 4,5,9,10,-11,12-hexahydro-6H-benzofuro[3a,3,2-ef][2]benzazépine avec la formule générale, choisi parmi le groupe constitué de 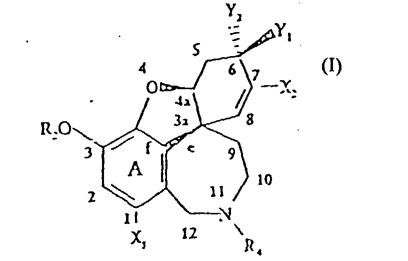 et 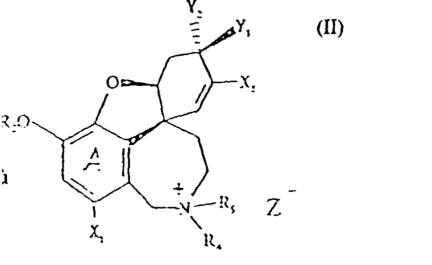 ou de sels de celui-ci, où
R₂, R₄, X₁ et X₂ sont soit identiques, soit différents et choisis parmi le groupe constitué d'hydrogène, de fluore, de chlore, de brome, d'iode, d'hydroxy, d'alkoxy, d'alkyle de faible indice, le cas échéant, substitué par un halogène au moins, de l'alkényle de faible indice, de l'alkinyle de faible indice, d'aryle, d'aralkyle, d'aryloxyalkyle, de formyle, d'alkylcarbonyle, d'arylcarbonyle, d'aralkylcarbonyle, d'alkyloxycarbonyle, d'aryloxycarbonyle, d'aralkyloxycarbonyle, d'alkylsulfonyle, d'aralkylsulfonyle et d'arylsulfonyle ;
où Y₁ et Y₂ sont soit identiqucs soit différents et choisis parmi le groupe constitué d'hydrogène, de fluore, de chlore, de brome, d'iode, d'hydroxy, d'alkoxy, d'alkyle de faible indice qui est substitué, le cas échéant, par un halogène au moins, de l'alkényle de faible indice, de l'alkinyle de faible indice, d'aryle, d'aralkyle, d'aryloxyalkyle, de formyle, d'alkylcarbonyle, d'arylcarbonyle, d'aralkylcarbonyle, d'alkyloxycarbonyle, d'aryloxycarbonyle, d'aralkyloxycarbonyle, d'alkylsulfonyle, d'aralkylsulfonyle, d'arylsulfonyle ou, ensemble, O (cétone) ;
où A est un noyau benzénique, qui est, le cas échéant, substitué une fois au moins par de l'alkyle de faible indice, par de l'alcène de faible indice, par de l'alkine de faible indice, par de l'alkoxy, du fluore, du chlore, du brome, de l'iode, de l'alkyle qui est substitué par un halogène au moins, de l'aralkyle, de hydroxy, de l'amino primaire, de l'amino secondaire, de l'amino tertiaire, du nitro, du nitrile, de l'alkylamino, de l'arylamino, de l'aldéhyde, de l'acide de carbone, et de dérivés d'acide de carbone ;
où Z est un anion d'un acide organique pharmaceutiquement acceptable ou un anion anorganique et
où R₅ est choisi parmi le groupe constitué d'hydrogène, de formyle d'alkyle, d'alkényle, d'aryle, d'aralkyle, d'alkylecarbonyle, d'arylcarbonyle, d'aralkylcarbonyle, d'alkyloxycarbonyle, d'aryloxycarbonyle, d'aralkyloxycarbonyle, d'alkylesulfonyle, d'arylesulfonyle et d'aralkylesulfonyle ;
caractérisé en ce que l'on cyclise oxydativement un produit de la formule générale (Va) 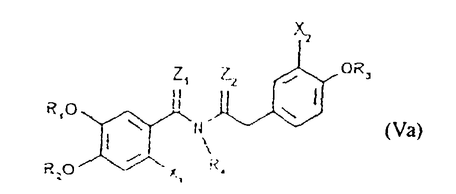
dans laquelle R₁, R₂, R₃, R₄, X₁, X₂ ont les significations mentionnées dans la revendication 1 et dans laquelle Z₁ et Z₂ signifient O, S, N, en introduisant, le cas échéant, si R₄ est de l'hydrogène, un groupe de protection N dans le composé de la formule générale (Va), de manière à obtenir un produit de la formule générale (Ia) 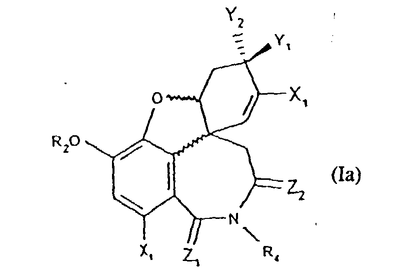
en transformant avec une base et un agent d'oxydation,
(D) que l'on transforme un produit, obtenu de cette manière, de la formule générale (I), dans laquelle Y₁ et Y₂ signifient, ensemble, O (cétone), en un cétal ou un thiocétal, que l'on réduit le cétal ou le thiocétal ainsi obtenu, que l'on décompose, dans le composé formé de type narwedincétal, le groupe cétal ou thiocétal, que l'on transforme un composé racémique de la formule générale (I), dans laquelle R₄ est choisi parmi le groupe constitué d'hydrogène, d'alkyle, d'alkényle, d'alkinyle, d'aryle et d'aralkyle, par cristallisation induite par chiralité dans l'énantiomère correspondant et que l'on réduit l'énantiomère ainsi obtenu avec L-Selektride, K-Selektride, KS-Selektride ou LS-Selektride afin d'obtenir un produit de la formule générale (I).

5. Procédé selon une des revendications 1 à 4, caractérisé en ce que l'on exécute la réaction de condensation (étape B) dans un solvant à la température de reflux et que l'on sépare tout au plus l'eau qui se forme.

6. Procédé selon une des revendications 1 à 5, caractérisé en ce que l'on exécute la réduction du produit de condensation (base de Schiff) obtenu à l'étape B à l'aide d'un agent de réduction choisi parmi le groupe constitué de borohydrure de sodium, borohydrure de potassium, cyanoborhydrure de sodium, LiAIH₄ et de mélanges de ceux-ci.

7. Procédé selon une des revendications 1 à 6, caractérisé en ce que, à l'étape C, on transforme le composé de la formule généralc (V) ou (Va) à l'aide d'une base, qui est choisie parmi un groupe constitué de sodium hydrogénocarbonate, carbonate de potassium. de NaOH, de KOH et de pyridine.

8. Procédé selon une des revendications 1 à 7, caractérisé en ce que, à l'étape CI on transforme le composé de la formule générale (V) ou (Va) à l'aide d'un oxydant choisi parmi le groupe constitué de Pb(Oac)₄, KMnO₄, chlorure ferreux, ferricyanure de potassium et H₂O₂.

9. Procédé selon une des revendications 1 à 8, caractérisé en ce que, à l'étape C, on exécute la transformation dans un solvant choisi parmi le groupe constitué de toluène et de xylène.

10. Procédé selon une des revendication 3 à 9, caractérisé en ce qu'à l'étape D, le produit de la formule générale (I) ou (Ia), dans laquelle Y₁ et Y₂ signifient, ensemble, O (cétone) est transformé en un cétal ou un thiocétal, en ce que le produit de la formule générale (I) ou (Ia) est transformé avec un composé choisi parmi le groupe constitué d'un alcool R₆-OH et d'un thiol R₆-SH, R₆ étant choisi parmi le groupe constitué d'alkyle, d'alkényle, d'aryle, d'aralkyle, d'alkylecarbonyle, d'arylcarbonyle et d'aralkylcarbonyle, R₅ étant, le cas échéant, substitué par un halogène au moins.

11. Procédé selon une des revendications 3 à 9, caractérisé en ce qu'à l'étape D, le produit de la formule générale (I) ou (Ia), dans laquelle Y₁ et Y₂ signifient, ensemble, O (cétone), est réduit en un cétal ou un thiocétal en transformant le produit de la formule générale (I) avec un diol R₆ (OH)₂ ou un dithiol R₆ (SH)₂, R₆ ayant les significations mentionnées dans la revendication 10.

12. Procédé selon revendication 11, caractérisé en ce qu'à l'étape D, on transforme avec du propylène glycol pour former le cétal.

13. Procédé selon une des revendications 3 à 12, caractérisé en ce que l'on réduit le cétal ou le thiocétal avec un agent de réduction choisi dans le groupe constitué de borohydrure de sodium, de borohydrure de potassium, de cyanoborhydrure de sodium, de LiAIH₄, L-Selektride, DiBAI, REAI, K-Selektride, KS-Selektride, LS-Selektride, Superhydrures, 9-BBN Zn/CacL₂ et de mélanges de ceux-ci.

14. Procédé selon la revendication 13, caractérisé en ce que l'agent de réduction est du LiAIH₄.

15. Procédé selon une des revendications 1 à 14, caractérisé en ce que le produit obtenu est le sel d'ammonium quaternaire de la formule générale (II).

16. Procédé selon la revendication 15. caractérisé en ce que Z est choisi parmi le groupe constitué de tartrate. de lactate. de citrate, d'acétate, de maléinate, de fluorure, de chlorure, de bromure, d'iodure, de sulfate. de phosphate et de chlorate.

17. Procédé selon revendication 16, caractérisé en ce que R₁, R₂ et R₃ sont choisis parmi le groupe constitué d'hydrogène, d'alkyle, d'alkényle, d'aryle, d'aralkyle, d'alkylecarbonyle, d'arylcarbonyle et d'aralkylcarbonyle, R₁, R₂ et R₃ étant, le cas échéant, substitués par un halogène au moins.

18. Procédé selon une des revendications 1 à 16, caractérisé en ce que R₄ et R₅ sont choisis parmi le groupe constitué d'hydrogène, de formyle, d'alkyle, d'alkényle, d'aryle, d'aralkyle, d'alkylcarbonyle, d'arylcarbonyle, d'aralkylecarbonyle, d'alkylesulfonyle, d'arylsulfonyle et d'aralkylsulfonyle, R₄ et R₅ étant, le cas échéant, substitués par un halogène au moins.

19. Procédé selon une des revendications 1 à 18, caractérisé en ce que X₁ et X₂ sont choisis parmi le groupe constitué d'hydrogène, de fluorure, de chlorure, de bromure, d'iodure et de t-butyle.

20. Procédé selon une des revendications 1 à 19, caractérisée en ce que l'on protège l'azote du composé de la formule générale (V) ou Va) contre l'oxydation en introduisant un composé choisi parmi le groupe constitué de formyle, d'aralkyle, d'alkylcarbonyl, d'arylcarbonyle, d'aralkylcarbonyle, d'aralkyloxycarbonyle, d'alkylsulfonyle, d'aralkylsulfonate et d'arylsulfonyle.

21. Procédé selon revendication 20, caractérisé en ce que l'on introduit un groupe de formyle en transformant le produit de la formule générale (V) ou (Va) avec 1 à 50 fois la quantité de formate d'éthyle en présence de quantités catalytiques d'acide formique.

22. Procédé selon une des revendications 1 à 21, caractérisé en ce que l'on prépare un produit de la formule générale (V), dans laquelle
R₁, R₂ et R₃ sont choisis parmi un groupe constitué d'hydrogène, d'alkyle, d'aryle, d'aralkyle, d'alkylecarbonyle, d'arylcarbonyle et d'aralkylcarbonyle ;
X₁ est du brome ;
X₂ est de l'hydrogène ; et
R₄ est choisi parmi un groupe constitué d'hydrogène, de formyle, d'aralkyle, d'alkylcarbonyle, d'arylcarbonyle, d'aralkylcarbonyle, d'alkyloxycarbonyle, d'aryloxycarbonyle, d'aralkyloxycarbonyle, d'alkylesulfonyle, d'aralkylsulfonyle et d'arylsulfonyle,
en transformant un produit de la formule générale (V) 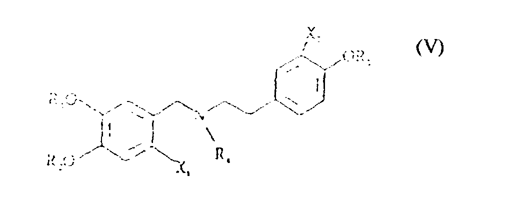 dans laquelle R₄ est CHO et X₁ cst de l'hydrogène, avec un réactif de bromation.

23. Procédé selon une des revendications 1 à 22, caractérisé en ce que l'on cyclise un produit de la formule générale (V) 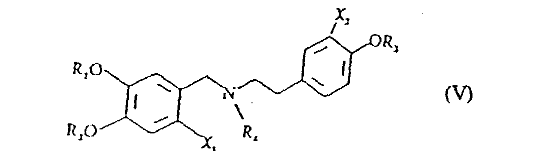 dans laquelle
R₂ est choisi parmi un groupe constitué d'hydrogène, d'alkyle, d'aryle, d'arylcarbonyle, d'aralkyle, d'alkylcarbonyle et d'arylcarbonyl ;
où X₁ est choisi parmi un groupe constitué d'hydrogène, de fluore, de chlore, de brome, d'iode et detobutyle ;
où R₄ est choisi parmi un groupe constitué d'hydrogène, de formyle, d'aralkyle, d'alkylcarbonyle, d'arylcarbonyle, d'aralkylcarbonyle, d'alkoxycarbonyle, d'aryloxycarbonyle, d'aralkyloxycarbonyle, d'alkylsulfonyle, d'aralkylsulfonyle et d'arylsulfonyle ; et
dans laquelle R₃ est de l'hydrogène ;
en un produit de la formule générale (I), dans laquelle
R₂, R₄ et X₁ ont les significations ci-dessus,
X₂ est de l'hydrogène ou du brome ; et
Y₁ et Y₂ sont ensemble O (cétone) en transformant avec une base et un oxydant.

24. Procédé selon revendication 23, caractérisé en ce que la cyclisation oxydative est effectuée en présence de tetraalkylammoniumchlorure, tel que de l'aliquote, de l'éther-couronne, de l'acide ascorbique, du chlorure de cuivre ou du trifluorure d'acide acétique ou de mélanges de ceux-ci.

25. Procédé de débromation d'un produit de la formule générale (I) caractérisé en ce que, pour débromer, on transforme un produit de la formule générale (I) 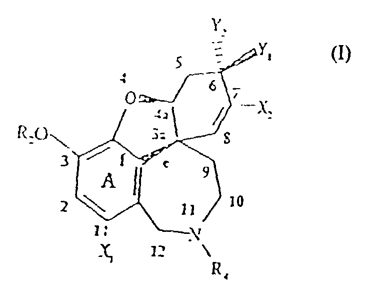
dans laquelle X₁ est du brome ;
dans laquelle R₂, R₄ et X₂ sont soit identiques, soit différents et choisis parmi un groupe constitué d'hydrogène, de fluore, de chlore, de brome, d'iode, d'hydroxy, d'alkoxy, d'alkyle de faible indice, substitué, le cas échéant, par un halogène au moins, d'alkényle de faible indice, d'alkinyle de faible indice, d'aryle, d'aralkyle, d'aryloxyalkyle, de formyle, d'alkylcarbonyle, d'arylcarbonyle, d'aralkylcarbonyle, d'alkyloxycarbonyle, d'aryloxycarbonyle, d'aralkyloxycarbonyle, d'alkylsulfonyle, d'aralkylsulfonyle et d'arylsulfonyle ;
dans laquelle Y₁ et Y₂ sont soit identiques, soit différents et choisis parmi le groupe constitué d'hydrogène, de fluore, de chlore, de brome, d'iode, d'hydroxy, d'alkoxy, d'alkyle de faible indice, substitué, le cas échéant, par un halogène au moins, d'alkényle de faible indice, d'alkinyle de faible indice, d'aryle, d'aralkyle, d'aryloxyalkyle, de formyle, d'alkylcarbonyle, d'arylcarbonyle, d'aralkylcarbonyle, d'alkyloxycarbonyle, d'aryloxycarbonyle, d'aralkyloxycarbonyle, d'alkylsulfonyle, d'aralkylsulfonyle et d'arylsulfonyle ou, ensemble, O (cétone) ;
dans laquelle A est un noyau benzénique, qui est, le cas échéant, substitué une fois au moins par de l'alkyle de faible indice, par de l'alcène de faible indice, par de l'alkine de faible indice, par de l'alkoxy, du fluore, du chlore, du brome, de l'iode. de l'alkyle qui est substitué par un halogène au moins, de l'aralkyle, de l'hydroxy, de l'amino primaire, de l'amino secondaire, de l'amino tertiaire, du nitro, du nitrile, de l'alkylamino, de l'arylamino, de l'aldéhyde, de l'acide de carbone, et de dérivés d'acide de carbone ;
avec un mélange constitué de:
a) acide formique, triéthylamine et palladium(charbon actif ou
b) poudre de zinc métallique et CaCl₂ dans l'alcool.

26. Procédé de préparation de produits du type N-déméthylebromegalanthamine et N-déméthyleépibromegalanthamine, caractérisé en ce que l'on réduit un composé de la formule générale (I) 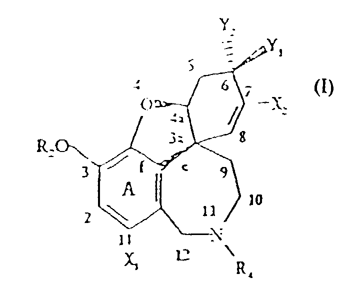 où
R₂ est de l'alkyle,
X₁ du brome;
R₄ du CHO ;
X₂ de l'hydrogène;
Y₁ et Y₂ sont O (cétone) et où
A est un noyau benzénique, qui est, le cas échéant, substitué une fois au moins par de l'alkyle de faible indice, par de l'alcène de faible indice, par de l'alkine de faible indice, par de l'alkoxy, du fluore, du chlore, du brome, de l'iode, de l'alkyle qui est substitué par un halogène au moins, de l'aralkyle, de l'hydroxy, de l'amino primaire, de l'amino secondaire, de l'amino tertiaire, du nitro, du nitrile, de l'alkylamino, de l'arylamino, de l'aldéhyde, de l'acide de carbone, et de dérivés d'acide de carbone.

27. Procédé selon revendication 26, caractérisé en ce que l'on réduit avec DIBAL-H, REDAI ou Superhydrure, L-Selektride, K-Selektride, KS-Selektride ou LS-Selektride.

28. Procédé selon une des revendications 1 à 24, caractérisé en ce que l'acide chiral est choisi parmi le groupe constitué de dibenzoyle d'acide tartrique, de di-p-toluolyle d'acide tartrique, d'acide tartrique, d'acidc citrique, d'acide camphorique, d'acide de camphane, d'acide sulfonique de camphre ou d'acide phénylglycolique.

29. Procédé selon revendication 28, caractérisé en ce que l'acide chiral est choisi parmi un groupe constitué de (+)di-p-toluoyle-D-acide tartrique et (-)di-p-toluoyle-L-acide tartrique.

30. Procédé selon une des revendications 3 à 29, où Y₁ et Y₂ sont choisis parmi le groupe constitué de OR₆ et OR₆O, où X₁ est du brome et R₄ du CHO.

31. Nouveau produit choisi parmi le groupe constitué de
a) Bromegalanthamine de la formule 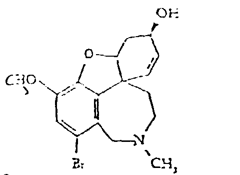
b) Epibromegélanthamine de la formule 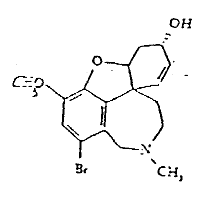
c) N-déméthylebromegalanthamine de la formule 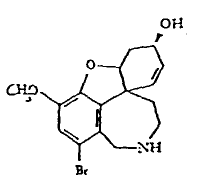
d) N-deméthyle-épibrometalanthamine de la formule 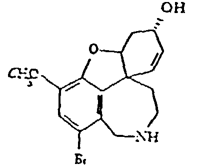
e) Brome-N-formule-narwédine-propylèneglycolcétal de la formule 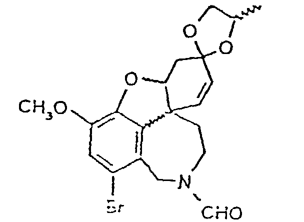
f) Narwédine-propylèneglycolcétal de la formule 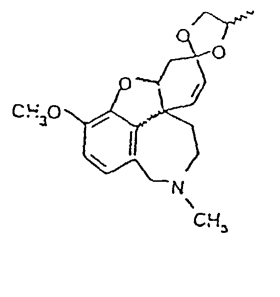
g) Brome-N-formule-narwédine-éthylèneglycolcétal de la formule 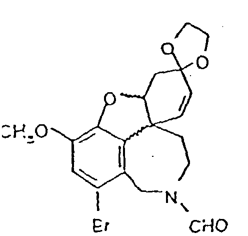
h) Narwédine-éthylèneglycolcétal de la formule 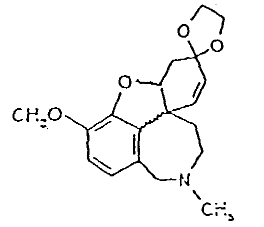
i) O-(2-hydroxyethyle)-galanthamine de la formule 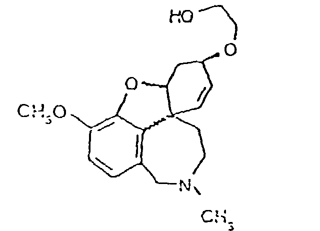
j) Brome-N-démethyle-narwédine-éthylèneglycolcétal de la formule 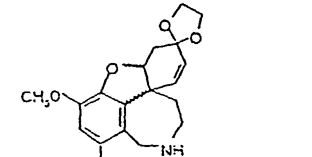
k) Brome-N-benzyle-narwédine-éthylèneglyolcétal de la formule 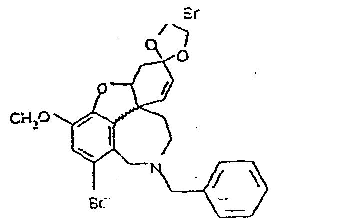
l) Brome-N-déméthylenarwédine de la formule 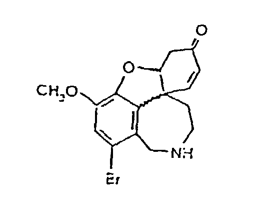
n) 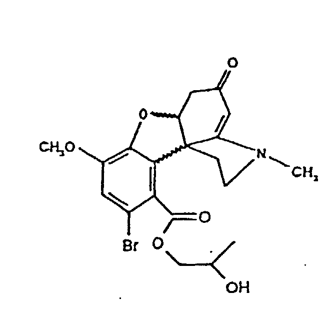
o)
